# FASCICULE DE BREVET EUROPEEN

(11) **EP 1 673 338 B1**
(45) Date de publication et mention de la délivrance du brevet: **19.09.2007**
(21) Numéro de dépôt: 02779622.6
(22) Date de dépôt: 08.08.2002
(51) Int. Cl.: C07C 323/52, A61K 31/22, A61K 31/23

(54) **COMPOSES DERIVES D'ACIDES GRAS PREPARATION ET UTILISATIONS**
FETTSÄUREVERBINDUNGEN UND DEREN HERSTELLUNG UND VERWENDUNGEN
FATTY ACID COMPOUNDS, PREPARATION AND USES THEREOF

(30) Priorité: 09.08.2001 FR 0110645
(43) Date de publication de la demande: 28.06.2006
(73) Titulaire: Genfit, 59120 Loos (FR)
(72) Inventeur: NAJIB-FRUCHART, Jamila, 40000 Marrakech (MA); CAUMONT-BERTRAND, Karine, F-59930 La Chapelle d'Armentieres (FR)
(74) Mandataire: Tezier Herman, Béatrice
(86) Numéro de dépôt international: PCT/FR2002/002831
(87) Numéro de publication internationale: WO 2003/014073

(56) Documents cités:
- EP-A- 0 018 342
- EP-A- 0 447 553
- DE-A- 2 028 240
- US-A- 2 010 154
- US-A- 4 134 770
- DATABASE CAPLUS [en ligne] CHEMICAL ABSTRACTS SERVICE, COLUMBUS, OHIO, US; retrieved from STN Database accession no. 1983:18071 XP002203065 & JP 57 082573 A (MATSUMO YUSHI-SEIYAKU CO LTD) 24 mai 1982 (1982-05-24)
- L.P. MOLLEYRES ET AL: J. BIOL. CHEM., vol. 263, no. 29, 1988, pages 14832-14838, XP001042275
- K. LARSSON: ACTA CRYSTALLOGR., vol. 16, 1963, pages 741-748, XP000926795
- R. LI ET AL: J. ORG. CHEM., vol. 58, no. 7, 1993, pages 1952-1954, XP002203063
- CHEMICAL ABSTRACTS, vol. 51, no. 13, 10 juillet 1957 (1957-07-10) Columbus, Ohio, US; abstract no. 9489f, XP002203064 & L.C.F. BLACKWELL ET AL: J. CHEM. SOC., 1957, pages 165-169,
- DATABASE CAPLUS [en ligne] CHEMICAL ABSTRACTS SERVICE, COLUMBUS, OHIO, US; retrieved from STN Database accession no. 1976:151548 XP002203066 & JP 50 160358 A (ADEKA ARGUS CHEMICAL CO LTD) 25 décembre 1975 (1975-12-25)
- T. NAALSUND ET AL: CHEMISTRY AND PHYSICS OF LIPIDS, vol. 112, no. 1, 2001, pages 59-65, XP000926796
- U. HEIMANN ET AL: LIEBIGS ANN. CHEM., vol. 6, 1980, pages 858-862, XP001042276

## Description

La présente invention se rapporte à de nouvelles molécules, leur préparation et leurs utilisations, notamment dans les domaines de la santé humaine et animale. Les composés de l'invention sont, en partie, des dérivés d'acides gras et possèdent des propriétés pharmacologiques, anti-oxydantes et anti-inflammatoires avantageuses. L'invention décrit également les différentes utilisations de ces composés, les compositions pharmaceutiques et cosmétiques les contenant ainsi que les procédés permettant leur préparation. Les composés de l'invention sont utilisables notamment pour prévenir ou traiter les maladies cardiovasculaires, le syndrome X, la resténose, le diabète, l'obésité, l'hypertension, certains cancers, des maladies dermatologiques ainsi que dans le domaine cosmétique, pour prévenir ou traiter les effets du vieillissement cutané, notamment l'apparition de rides, etc..

L'athérosclérose et ses complications cardiovasculaires sont la principale cause de morbidité et de mortalité dans les pays fortement industrialisés. L'athérosclérose et ses complications sont également une conséquence importante du diabète de type II. Un lien strict de cause à effet a été démontré entre les dislipidémies et les maladies cardiovasculaires. Un taux circulant élevé de LDL-cholestérol est défavorable. Le risque occasionné par un taux élevé de LDL-cholestérol est amplifié par une élévation du taux de triglycérides. L'importance de la stabilité des lésions athéroscléreuses dans la survenue d'accidents cardiovasculaires a en outre été mise en évidence. Le rôle de l'oxydation des LDL dans le développement de la plaque d'athérosclérose et de sa fragilisation est mieux compris.

Les traitements médicamenteux de l'athérosclérose visent à réduire les taux circulants de cholestérol ou de triglycérides, à augmenter la stabilité des plaques d'athérosclérose, à réduire les contraintes mécaniques auxquelles sont soumis les vaisseaux (réduction de la tension artérielle) et à réduire les facteurs de risques annexes tels que le diabète.

Parmi les médicaments actuellement utilisés dans le traitement des dislipidémies, on trouve les fibrates et les statines. Les thiazolidinediones sont utilisées dans le traitement du diabète de type II.

Les fibrates sont largement utilisés pour le traitement des hypertriglycéridémies. Ils ont également des effets favorables au niveau de l'hypercholestérolémie. Ils sont généralement bien tolérés mais présentent un certain nombre d'effets secondaires : réactions cutanées, effets neurologiques, effets musculaires et gastro-intestinaux. Les effets toxiques sont rares (reins, muscles, articulation, peau, hépatite, etc.). Quant à l'incidence sur le risque cancéreux, elle est importante chez le rongeur même si elle n'a pas été démontrée chez l'homme.

Les statines sont largement utilisées pour le traitement de l'hypercholestérolémie. Il a été démontré que le traitement de patients ayant subi un premier accident vasculaire réduit considérablement le risque de récidive. Des signes occasionnels ou des symptômes d'hépatites ou de myopathie ont par ailleurs été décrits.

Les thiazolidinediones (troglitazone) ont récemment été introduites pour le traitement de la résistance à l'insuline. De ce fait, le recul nécessaire à une estimation objective de l'ensemble des effets adverses de ces molécules n'est pas suffisant. Dans ce contexte, l'observation d'une augmentation de la fréquence de tumeurs coliques dans un modèle animal prédisposé au cancer du colon (souris Min portant une mutation du gène APC) est défavorable. Une thiazolidinedione (troglitazone) a d'ailleurs été tout récemment retirée du marché pour des questions de toxicité hépatique.

Les principales molécules utilisées dans le traitement médicamenteux de l'athérosclérose (fibrates, statines) ont un spectre d'action pléiotropique. Les fibrates activent une classe de récepteurs nucléaires (PPARα, PPARγ, etc.) impliqués dans la coordination de l'expression de protéines responsables du transport ou du métabolisme des lipides. Le caractère pléiotropique du spectre d'action des fibrates réside dans la diversité des gènes cibles des PPARs. Les statines diminuent la synthèse de novo du cholestérol en inhibant l'activité de l'HMG-CoA réductase.

DE 2028240 A décrit des esters de glycérols comprenant des groupes alkylthioalcoyles à longue chaîne alkyle. Ces composés sont utilisés comme stabilisateurs pour des compositions contenant des protéines. JP 57082573 A (abrégé Chemical Abstracts 1983:18071; XP2203065) décrit le triester de glycérol et de l'acide 3-(9-octadécénylthio)propanoïque et son utilisation comme lubrifiant pour fibre synthétiques. Des esters de polyols, en particulier de glycérol, sont décrits dans EP 0018342 A ainsi que par L. P. Molleyres et al. dans J. Biol. Chem., 263 (29), pp. 14832-38, (1988) (XP001042275). Leurs application en thérapeutiques y sont discutées.

La présente invention propose une nouvelle famille de composés possédant des propriétés pharmacologiques avantageuses et utilisables pour le traitement curatif ou préventif de diverses pathologies.

Les composés de l'invention répondent à la formule générale (I): 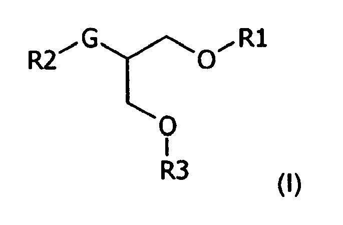 dans laquelle :
- G représente un atome d'oxygène, un atome de soufre ou un groupe N-R4 dans lequel R4 est un atome d'hydrogène ou un groupe alkyle linéaire ou ramifié, saturé ou non, éventuellement substitué, comportant de 1 à 5 atomes de carbone,
- R1, R2 et R3, identiques ou différents, représentent (i) un atome d'hydrogène, (ii) un groupe CO-R dans lequel R est un groupe alkyle linéaire ou ramifié, saturé ou non, éventuellement substitué, dont la chaîne principale comporte de 1 à 25 atomes de carbone, ou (iii) un groupe de formule CO-(CH₂)₂ₙ₊₁-X-R' dans lequel X est un atome de soufre, un atome de sélénium, un groupe SO ou un groupe SO₂, n est un nombre entier compris entre 0 et 11, préférentiellement égal à 0 ou 1 et encore plus préférentiellement à 0, et R' est un groupe alkyle linéaire ou ramifié, saturé ou non, éventuellement substitué, dont la chaîne principale comporte de 13 à 23 atomes de carbone et éventuellement un ou plusieurs hétérogroupes, de préférence 0, 1 ou 2, plus préférentiellement 0 ou 1, choisis parmi un atome d'oxygène, un atome de soufre, un atome de sélénium, un groupe SO et un groupe SO₂, l'un au moins des groupes R1, R2 et R3 étant un groupe de formule CO-(CH₂)₂ₙ₊₁-X-R' tel que défini ci-dessus.

L'invention concerne également une composition pharmaceutique comprenant un composé de formule générale (I).

L'invention concerne également une composition cosmétique comprenant un composé de formule générale (I).

L'invention a aussi pour objet l'utilisation des composés ci-dessus à titre de médicament, pour le traitement de diverses pathologies, notamment de pathologies impliquant une dérégulation du métabolisme des lipides.

L'invention concerne aussi des méthodes de préparation de composés tels que décrits ci-dessus.

Dans les composés de formule générale (I) selon l'invention, le groupe G représente avantageusement un atome d'oxygène ou un groupe N-R4. D'autre part, lorsque G est N-R4, R4 représente préférentiellement un atome d'hydrogène ou un groupe méthyle.

Dans les composés de formule générale (I) selon l'invention, le ou les groupes R, identiques ou différents, représentent préférentiellement un groupe alkyle linéaire ou ramifié, saturé ou insaturé, substitué ou non, dont la chaîne principale comporte de 1 à 20 atomes de carbone, encore plus préférentiellement de 7 à 17 atomes de carbone.

Dans les composés de formule générale (I) selon l'inyention, le ou les groupes R', identiques ou différents, représentent préférentiellement un groupe alkyle linéaire ou ramifié, saturé ou insaturé, substitué ou non, dont la chaîne principale comporte de 13 à 20 atomes de carbone, encore plus préférentiellement de 14 à 17 atomes de carbone.

Des exemples particuliers de groupes alkyle à chaîne longue saturée pour R ou R' (e.g., supérieure ou égale à 7 carbone) sont notamment les groupes C₇H₁₅, C₁₀H₂₁, C₁₁H₂₃, C₁₃H₂₇, C₁₄H₂₉, C₁₆H₃₃, C₁₇H₃₅, C₁₅H₃₁, C_{20:5}(5, 8, 11, 14, 17) et C_{22:6}(4, 7, 10, 13, 16, 19).

C_{20:5}(5, 8, 11, 14, 17) est l'acide eicosapentaenoïque (EPA) et C_{22:6}(4, 7, 10, 13, 16, 19) est l'acide docosahexaenoïque (DHA).

Des exemples de groupes alkyle à chaîne longue insaturée sont notamment les groupes C₁₄H₂₇, C₁₄H₂₅, C₁₅H₂₉, C₁₇H₂₉, C₁₇H₃₁, C₁₇H₃₃, C₁₉H₂₉, C₁₉H₃₁, C₂₁H₃₁, C₂₁H₃₅, C₂₁H₃₇, C₂₁H₃₉, C₂₃H₄₅.

Des exemples de groupes alkyle à chaîne longue ramifiée sont notamment les groupes (CH₂)_{n'}-CH(CH₃)C₂H₅, (CH=C(CH₃)-(CH₂)₂)_{n"}-CH=C(CH₃)₂ ou (CH₂)₂ₓ₊₁-C(CH₃)₂-(CH₂)_{n"'}-CH₃ (x étant un nombre entier égal à ou compris entre 1 et 11, n' étant un nombre entier égal à ou compris entre 1 et 22, n" étant un nombre entier égal à ou compris entre 1 et 5, n"' étant un nombre entier égal à ou compris entre 0 et 22, et (2x+n"') étant inférieur ou égal à 22).

Dans un mode particulier de mise en oeuvre, le ou les groupes R, identiques ou différents, peuvent également représenter avantageusement un groupe alkyle inférieur comprenant de 1 à 6 atomes de carbone. A titre d'exemple spécifique on peut citer notamment les groupes méthyle, éthyle, propyle et butyle, de préférence méthyle et éthyle.

D'autre part, le groupe alkyle, du substituant R ou R' peut également être cyclique, notamment dans le groupe R. Des exemples de groupes alkyle cycliques sont notamment le cyclopropyle, le cyclobutyle, le cyclopentyle et le cyclohexyle.

Comme indiqué ci-avant, les groupes alkyle peuvent être éventuellement substitués par un ou plusieurs substituants, identiques ou différents. Les substituants sont choisis de préférence parmi un atome d'halogène (iode, chlore, fluor, brome) et un groupe OH, =O, NO₂, NH₂, CN, CH₂-O, CH₂OCH₃, CF₃ et COOZ (Z étant un atome d'hydrogène ou un groupe alkyle, de préférence comportant de 1 à 6 atomes de carbone).

Dans un composé de formule générale (1), les groupes alkyle R et R' peuvent être identiques ou différents. Toutefois, on préfère les composés dans lesquels les groupes alkyle R' sont identiques entre eux ou présentent une longueur de chaîne similaire, c'est-à-dire ne présentant pas un écart supérieur à 3 atomes de carbone environ.

Un exemple tout particulièrement préféré de groupe R' est un groupe alkyle saturé et linéaire, comprenant avantageusement de 13 à 17 atomes de carbone, de préférence de 14 à 16, encore plus préférentiellement 14 atomes.

Par ailleurs, dans le groupe CO-(CH₂)₂ₙ₊₁-X-R', X représente tout préférentiellement un atome de soufre ou de sélénium et avantageusement un atome de soufre.

Par ailleurs, dans le groupe CO-(CH₂)₂ₙ₊₁-X-R', n est de préférence différent de 1 et est en particulier égal à 0. Un exemple spécifique de groupe CO-(CH₂)₂ₙ₊₁-X-R' selon l'invention est le groupe CO-CH₂-S-C₁₄H₂₉.

Des composés préférés au sens de l'invention sont donc des composés de formule générale (I) ci-dessus dans laquelle au moins un des groupes R1, R2 et R3 représente un groupe CO-(CH₂)₂ₙ₊₁-X-R' dans lequel X représente un atome de sélénium ou de préférence de soufre et/ou R' est un groupe alkyle saturé et linéaire comprenant de 13 à 17 atomes de carbone, de préférence de 14 à 16, encore plus préférentiellement 14 atomes de carbone.

A cet égard, des composés particuliers selon l'invention sont ceux dans lesquels R2 est un groupe de formule CO-(CH₂)₂ₙ₊₁-X-R', de préférence dans laquelle X représente un atome de sélénium ou de préférence de soufre et/ou R' est un groupe alkyle saturé et linéaire comprenant de 13 à 17 atomes de carbone, plus préférentiellement dans lesquels n est égal à 0, en particulier un groupe de formule CO-CH₂-S-C₁₄H₂₉.

Dans ces composés, R1 et R3, identiques ou différents, représentent avantageusement un atome d'hydrogène ou un groupe CO-R, de préférence un groupe CO-R.

D'autres composés particuliers selon l'invention sont ceux dans lesquels deux des groupes R1, R2 et R3 sont des groupes CO-(CH₂)₂ₙ₊₁-X-R', identiques ou différents, de préférence dans lesquels X représente un atome de sélénium ou de préférence de soufre et/ou R' est un groupe alkyle saturé et linéaire comprenant de 13 à 17 atomes de carbone, plus préférentiellement dans lesquels n est égal à 0, en particulier des groupes CO-CH₂-S-C₁₄H₂₉ .

Des composés particulièrement préférés sont les composés de formule générale (I) ci-dessus dans laquelle :
- G est un groupe N-R4 dans lequel R4 est un atome d'hydrogène ou un groupe méthyle, et
- au moins deux des groupes R1, R2 et R3 représentent un groupe CO-(CH₂)₂ₙ₊₁-X-R' tel que défini ci-avant, identique ou différent, de préférence identique.

D'autres composés préférés sont les composés de formule générale (I) ci-dessus dans laquelle R1, R2 et R3, identiques ou différents, de préférence identiques, représentent un groupe CO-(CH₂)₂ₙ₊₁-X-R' tel que défini ci-avant, de préférence dans lesquels X représente un atome de sélénium ou de préférence de soufre et/ou R' est un groupe alkyle saturé et linéaire comprenant de 13 à 17 atomes de carbone, plus préférentiellement dans lesquels n est égal à 0, et en particulier des groupes CO-CH₂-S-C₁₄H₂₉.

Une autre famille de composés préférés comprend les composés de formule générale (I) ci-dessus dans laquelle l'un des groupes R1, R2 et R3 est un groupe CO-(CH₂)₂ₙ₊₁-X-R' tel que défini ci-avant, un autre des groupes R1, R2 et R3 est un groupe CO-R tel que défini ci-avant et le troisième des groupes R1, R2 et R3 est un atome d'hydrogène.

Une famille particulière est celle dans laquelle R1 est un groupe de formule CO-(CH₂)₂ₙ₊₁-X-R', de préférence dans laquelle X représente un atome de sélénium ou de préférence de soufre et/ou R' est un groupe alkyle saturé et linéaire comprenant de 13 à 17 atomes de carbone, plus préférentiellement dans laquelle n est égal à 0 et en particulier un groupe CO-CH₂-S-C₁₄H₂₉. Dans cette famille, l'un et/ou les deux groupes R2 et R3 représentent avantageusement un atome d'hydrogène ou de préférence un groupe CO-R, identique ou non.

Une autre famille de composés selon l'invention est celle dans laquelle l'un des substituants R1, R2 ou R3 est un groupe COCH₃.

Des composés préférés au sens de l'invention sont ceux, de formule générale (I) décrite ci-dessus, dans laquelle :
- R2 est un groupe de formule CO-(CH₂)₂ₙ₊₁-X-R', en particulier CO-CH₂-S-C₁₄H₂₉ et, de préférence, R1 et R3, identiques ou différents, représentent un atome d'hydrogène ou un groupe CO-R. De tels composés dans lesquels R1 et R3, identiques ou différents, représentent tous deux un groupe CO-R sont préférés ; ou
- R1 est un groupe de formule CO-(CH₂)₂ₙ₊₁-X-R', en particulier un groupe CO-CH₂-S-C₁₄H₂₉ et, de préférence, l'un et/ou les deux groupes R2 et R3 représentent un atome d'hydrogène ou un groupe CO-R, identique ou non ; ou
- R1, R2, et R3, identiques, représentent un groupe de formule CO-(CH₂)₂ₙ₊₁-X-R', en particulier CO-CH₂-S-C₁₄H₂₉.

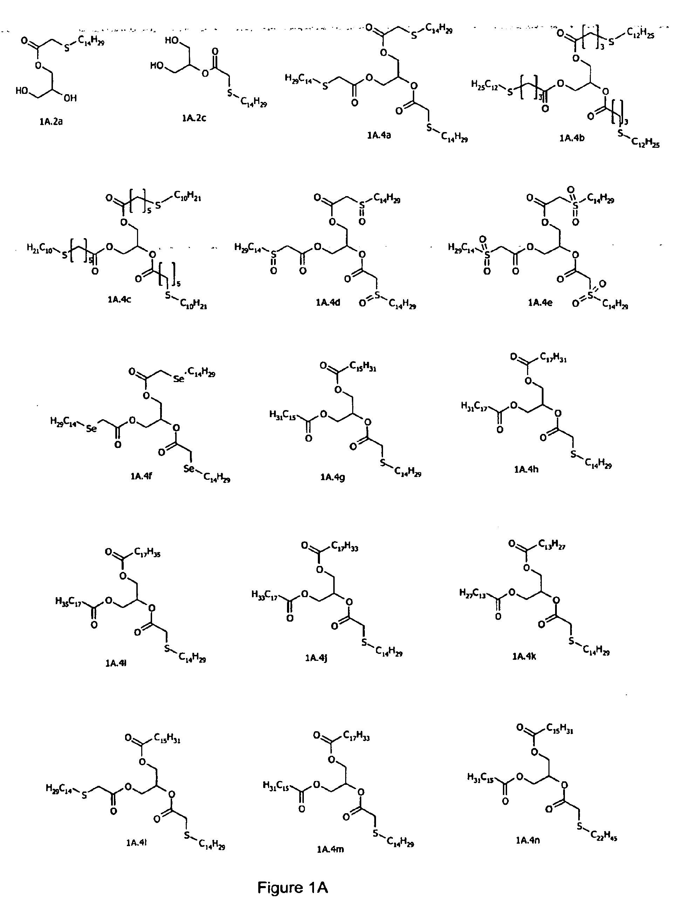

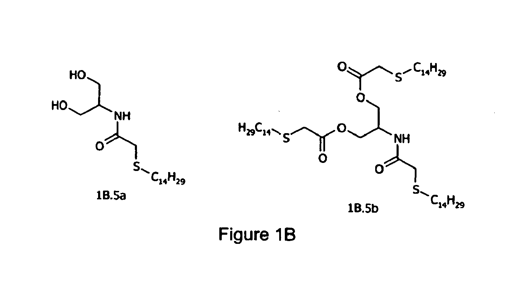

Des exemples de composés préférés selon l'invention sont représentés sur les Figures 1A et 1 B.

Les composés de l'invention peuvent être sous forme de sels, notamment de sels d'addition basiques ou acides, préférentiellement compatibles avec un usage pharmaceutique ou cosmétique. Parmi les acides pharmaceutiquement ou cosmétiquement acceptables, on peut citer, à titre non limitatif, les acides chlorhydrique, bromhydrique, sulfurique, phosphorique, acétique, trifluoroacétique, lactique, pyruvique, malonique, succinique, glutarique, fumarique, tartrique, maléique, citrique, ascorbique, camphorique, etc.. Parmi les bases pharmaceutiquement ou cosmétiquement acceptables, on peut citer à titre non limitatif, l'hydroxyde de sodium, l'hydroxyde de potassium, la triéthylamine, la *tert*-butylamine, etc..

L'invention concerne également l'emploi des composés de formule générale (I) et en particulier ceux décrits ci-avant, dans le domaine pharmaceutique ou cosmétique.
Elle concerne ainsi l'utilisation d'un composé de formule générale (I) et en particulier tel que décrit ci-avant pour la préparation d'une composition pharmaceutique pour la prévention et/ou le traitement de diverses pathologies, comme notamment les maladies cardiovasculaires, le syndrome X, la resténose, le diabète, l'obésité, l'hypertension, le cancer ou des maladies dermatologiques. Elle concerne également l'utilisation d'un composé de formule générale (I) et en particulier tel que décrit ci-avant pour la préparation d'une composition cosmétique pour la protection de la peau, pour lutter contre le vieillissement cutané et ses effets, pour lutter contre l'apparition ou le développement de rides, etc..
Ainsi, les composés utilisés dans le domaine pharmaceutique ou cosmétique sont de formule générale (1), dans laquelle :
- G représente un atome d'oxygène, un atome de soufre ou un groupe N-R4 dans lequel R4 est un atome d'hydrogène ou un groupe alkyle linéaire ou ramifié, saturé ou non, éventuellement substitué, comportant de 1 à 5 atomes de carbone,
- R1, R2 et R3, identiques ou différents, représentent (i) un atome d'hydrogène, (ii) un groupe CO-R dans lequel R est un groupe alkyle linéaire ou ramifié, saturé ou non, éventuellement substitué, dont la chaîne principale comporte de 1 à 25 atomes de carbone, ou (iii) un groupe de formule CO-(CH₂)₂ₙ₊₁-X-R' dans lequel X est un atome de soufre, un atome de sélénium, un groupe SO ou un groupe SO₂, n est un nombre entier compris entre 0 et 11, préférentiellement égal à 0 ou 1 et encore plus préférentiellement à 0, et R' est un groupe alkyle linéaire ou ramifié, saturé ou non, éventuellement substitué, dont la chaîne principale comporte de 2 à 23 atomes de carbone et éventuellement un ou plusieurs hétérogroupes, de préférence 0, 1 ou 2, plus préférentiellement 0 ou 1, choisis parmi un atome d'oxygène, un atome de soufre, un atome de sélénium, un groupe SO ou un groupe SO₂, l'un au moins des groupes R1, R2 et R3 étant un groupe de formule CO-(CH₂)₂ₙ₊₁-X-R' tel que défini ci-dessus.

Les composés utilisés de préférence dans ce cadre correspondent à des composés de formule (I) dans laquelle R' est un groupe alkyle linéaire ou ramifié, saturé ou non, éventuellement substitué, dont la chaîne principale comporte de 9 à 23 atomes de carbone et éventuellement un ou plusieurs hétérogroupes.
Avantageusement, les composés de formule (I) utilisés sont tels que définis ci-dessus.

L'utilisation d'un composé selon l'invention peut en effet permettre de diminuer les taux de triglycérides et de cholestérol circulants, d'inhiber la modification oxydative des LDL, d'induire l'expression d'enzymes impliquées dans la β-oxydation mitochondriale et peroxysomale, d'augmenter les capacités d'oxydation des acides gras hépatiques, d'induire la croissance des mitochondries dans les fibres musculaires de type I et II, d'activer PPARα et PPARγ, ou encore de diminuer la croissance des cellules tumorales.

A cet égard, les composés de l'invention possèdent, avantageusement un tropisme amélioré pour le foie et peuvent donc être administrés par voie orale ou systémique. D'autre part, en raison de leur structure, les composés de l'invention ont un effet durable.

Cette invention a pour but de développer une composition pharmaceutique comprenant au moins un composé selon l'invention éventuellement accompagné d'un véhicule acceptable sur le plan pharmaceutique.

L'invention concerne également une composition cosmétique comprenant au moins un composé selon l'invention éventuellement accompagné d'un véhicule cosmétiquement acceptable.

L'invention concerne également une composition nutritionnelle contenant l'un au moins des composés tels que définis ci-avant, utilisable seule ou en combinaison avec d'autres agents.

La présente invention concerne aussi une méthode de traitement comprenant l'administration à un mammifère d'une quantité efficace d'un composé selon l'invention. Les mammifères concernés peuvent être des animaux domestiques ou non ou des êtres humains.

Les composés ou compositions selon l'invention peuvent être administrés de différentes manières et sous différentes formes. Ainsi, ils peuvent être par exemple administrés de manière systémique, par voie orale, parentérale, par inhalation ou par injection, comme par exemple par voie intraveineuse, intramusculaire, sous-cutanée, trans-dermique, intra-artérielle, etc.. Pour les injections, les composés sont généralement conditionnés sous forme de suspensions liquides, qui peuvent être injectées au moyen de seringues ou de perfusions, par exemple. A cet égard, les composés sont généralement dissous dans des solutions salines, physiologiques, isotoniques, tamponnées, etc., compatibles avec un usage pharmaceutique et connues de l'homme du métier. Ainsi, les compositions peuvent contenir un ou plusieurs agents ou véhicules choisis parmi les dispersants, solubilisants, émulsifiants, stabilisants, conservateurs, tampons, etc.. Des agents ou véhicules utilisables dans des formulations liquides et/ou injectables sont notamment la méthylcellulose, l'hydroxyméthylcellulose, la carboxyméthylcellulose, le polysorbate 80, le mannitol, la gélatine, le lactose, des huiles végétales, l'acacia, les liposomes, etc..

Les composés peuvent ainsi être administrés sous forme de gels, huiles, comprimés, suppositoires, poudres, gélules, capsules, aérosols, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

Les composés peuvent être administrés oralement auquel cas les agents ou véhicules utilisés sont choisis préférentiellement parmi l'eau, la gélatine, les gommes, le lactose, l'amidon, le stéarate de magnésium, le talc, une huile, le polyalkylène glycol, etc..

Pour une administration parentérale, les composés sont préférentiellement administrés sous la forme de solutions, suspensions ou émulsions avec notamment de l'eau, de l'huile ou des polyalkylène glycols auxquels il est possible d'ajouter, outre des agents conservateurs, stabilisants, émulsifiant, etc., des sels permettant d'ajuster la pression osmotique, des tampons, etc..

Pour un usage cosmétique, les composés de l'invention peuvent être administrés sous forme de crème, comme par exemple des crèmes de soin, des crèmes solaires, des huiles, des gels, etc..

Il est entendu que le débit et/ou la dose injectée peuvent être adaptés par l'homme du métier en fonction du patient, de la pathologie concernée, du mode d'administration, etc.. Typiquement, les composés sont administrés à des doses pouvant varier entre 1 µg et 2 g par administration, préférentiellement de 0,1 mg à 1 g par administration. Les administrations peuvent être quotidiennes ou répétées plusieurs fois par jour, le cas échéant. D'autre part, les compositions selon l'invention peuvent comprendre, en outre, d'autres agents ou principes actifs.

Les composés de l'invention peuvent être préparés à partir de produits du commerce, en mettant en oeuvre une combinaison de réactions chimiques connues de l'homme du métier. L'invention concerne également des procédés de préparation des composés tels que définis ci-avant.

Selon un premier procédé de l'invention, les composés de formule (I) dans lesquels G est un atome d'oxygène ou de soufre, R1, R2 et R3 identiques ou différents, représentent un groupe CO-R ou un groupe CO-(CH₂)₂ₙ₊₁-X-R', sont obtenus à partir d'un composé de formule (I) dans laquelle G est respectivement un atome d'oxygène ou de soufre, R2 est un atome d'hydrogène et R1 et R3, identiques ou différents, représentent un groupe CO-R ou CO-(CH₂)₂ₙ₊₁-X-R', et d'un composé de formule A°-CO-A dans laquelle A est un groupe réactif choisi par exemple parmi OH, Cl, O-CO-A° et OR", R" étant un groupe alkyle, et A° est le groupe R ou le groupe (CH₂)₂ₙ₊₁-X-R', en présence éventuellement d'agents de couplages ou d'activateurs connus de l'homme de métier.

Les composés de formule (1) selon l'invention dans laquelle G est un atome d'oxygène, R2 est un atome d'hydrogène et R1 et R3, identiques ou différents, représentent un groupe CO-R ou CO-(CH2)₂ₙ₊₁-X-R', peuvent être obtenus de différentes façons.

Selon un premier mode, on fait réagir une molécule de glycérol avec un composé de formule A°-CO-A1 dans laquelle A1 est un groupe réactif choisi par exemple parmi OH, CI et OR", R" étant un groupe alkyle, et A° est le groupe R ou le groupe (CH₂)₂ₙ₊₁-X-R', en présence éventuellement d'agents de couplages ou d'activateurs connus de l'homme de métier. Cette réaction permet la synthèse de composés dits symétriques, dans lesquels R1 et R3 ont la même signification. Cette réaction peut être mise en oeuvre en adaptant les protocoles décrits par exemple dans Feuge et al., J. Am. Oil Chem. Soc., 1953, 30, 320-325; Gangadhar et al., Synth. Commun., 1989, 19, 2505-2514; Han et al., Biorg. Med. Chem. Lett., 1999, 9, 59-64 ; ou Robinson, J. Pharm. Pharmacol., 1960, 12, 685-689.

Les composés de formule (I) selon l'invention dans laquelle G est un atome d'oxygène, R2 est un atome d'hydrogène et R1 et R3, identiques ou différents, représentent un groupe CO-R ou CO-(CH₂)₂ₙ₊₁-X-R', peuvent également être obtenus à partir d'un composé de formule (I) selon l'invention dans laquelle G est un atome d'oxygène, R2 et R3 représentent un atome d'hydrogène et R1 est un groupe CO-R ou CO-(CH₂)₂ₙ₊₁-X-R' (cette forme particulière de composés de formule (I) étant nommée composés IV), et d'un composé de formule A°-CO-A2 dans laquelle A2 est un groupe réactif choisi par exemple entre OH et Cl, et A° est le groupe R ou le groupe (CH₂)₂ₙ₊₁-X-R', en présence éventuellement d'agents de couplages ou d'activateurs connus de l'homme de métier. Cette réaction est avantageusement réalisée selon le protocole décrit par exemple dans Daubert et al., J. Am. Chem. Soc., 1943, 65, 2144-2145 ; Feuge et Lovegren, J. Am. Oil Chem. Soc., 1956, 33, 367-372 ; Katoch et al., Bioorg. Med. Chem., 1999, 7, 2753-2758 ou Strawn et al., J. Med. Chem., 1989, 32, 643-648.

Les composés IV décrits ci-dessus peuvent être préparés par un procédé comprenant :
a) réaction d'un composé de formule générale (II) 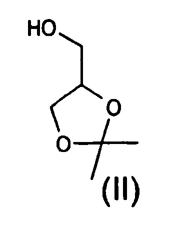 avec un composé de formule A°-CO-A2 dans laquelle A2 est un groupe réactif choisi par exemple entre OH et Cl, et A° est le groupe R ou le groupe (CH₂)₂ₙ₊₁-X-R', en présence éventuellement d'agents de couplages ou d'activateurs connus de l'homme de métier pour donner un composé de formule générale (III) 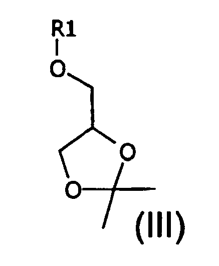 dans laquelle R1 représente un groupe CO-R ou CO-(CH₂)₂ₙ₊₁-X-R' ; et
b) déprotection du composé (III) par un acide (acide acétique, acide trifluoroacétique, acide borique, acide sulfurique, etc.) pour donner un composé de formule générale (IV) tel que défini ci-avant.

Selon un autre procédé particulier de l'invention les composés de formule (I) dans laquelle G est un atome d'oxygène, R3 est un atome d'hydrogène et R1 et R2, identiques ou différents, représentent un groupe CO-R ou CO-(CH₂)₂ₙ₊₁-X-R', peuvent être obtenus à partir d'un composé de formule (I) selon l'invention dans laquelle G est un atome d'oxygène, R2 et R3 représentent un atome d'hydrogène et R1 est un groupe CO-R ou CO-(CH₂)₂ₙ₊₁-X-R' (composés IV), selon les étapes suivantes :
a) réaction du composé (IV) avec un composé PxE dans lequel Px est un groupement protecteur ; et E est un groupe réactif choisi par exemple parmi OH ou un halogène, pour donner un composé de formule générale (V) dans laquelle R1 est un groupe CO-R ou CO-(CH₂)₂ₙ₊₁-X-R'. La réaction peut avantageusement être mise en oeuvre en adaptant le protocole décrit par Gaffney et Reese, Tet. Lett., 1997, 38, 2539-2542 dans lequel PxE peut représenter le composé 9-phénylxanthène-9-ol ou le 9-chloro-9-phénylxanthène 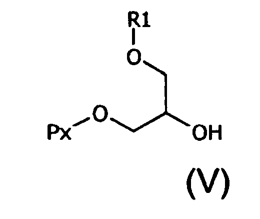
b) réaction du composé de formule (V) avec un composé de formule A°-CO-A2 dans laquelle A2 est un groupe réactif choisi par exemple entre OH et Cl, et A° est le groupe R ou le groupe (CH₂)₂ₙ₊₁-X-R', en présence éventuellement d'agents de couplages ou d'activateurs connus de l'homme de métier pour donner un composé de formule générale (VI), dans laquelle R1 et R2, identiques ou différents, représentent un groupe CO-R ou CO-(CH₂)₂ₙ₊₁-X-R' et Px est un groupement protecteur 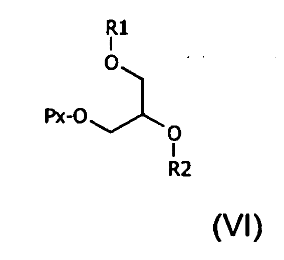
c) déprotection du composé (VI), en milieu acide pour donner un composé de formule générale (I) dans laquelle G est un atome d'oxygène, R3 est un atome d'hydrogène et R1 et R2, identiques ou différents, représentent un groupe CO-R ou CO-(CH₂)₂ₙ₊₁-X-R'.

Selon un autre procédé particulier de l'invention, les composés de formule générale (I) dans laquelle G est un atome d'oxygène, R1 et R3 représentent un atome d'hydrogène et R2 représente un groupe CO-R ou CO-(CH₂)₂ₙ₊₁-X-R', sont obtenus par un procédé comprenant :
a) la réaction d'un composé de formule (VII) 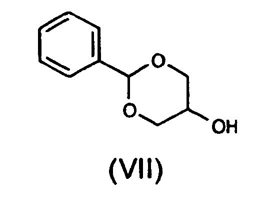 avec un composé de formule A°-CO-A2 dans laquelle A2 est un groupe réactif choisi par exemple entre OH et Cl, et A° est le groupe R ou le groupe (CH₂)₂ₙ₊₁-X-R', en présence éventuellement d'agents de couplages ou d'activateurs connus de l'homme de métier pour donner un composé de formule générale (VIII) 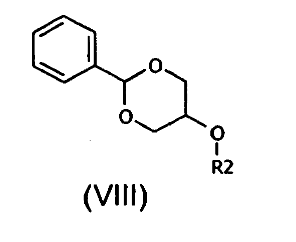 dans laquelle R2 représente un groupe CO-R ou CO-(CH₂)₂ₙ₊₁-X-R' ; et
b) déprotection du composé de formule (VIII) en milieu acide ou par hydrogénation catalytique pour donner un composé de formule générale (I) dans laquelle G est un atome d'oxygène, R1 et R3 représentent un atome d'hydrogène et R2 représente un groupe CO-R ou CO-(CH₂)₂ₙ₊₁-X-R'.

Les étapes ci-dessus peuvent être réalisées avantageusement selon les protocoles décrits par Bodai et al., Syn. Lett., 1999, 6,759-761 ; Paris et al., J. Med. Chem., 1980, 23, 9-12 ; Scriba et al., Arch. Pharm. (Weinheim), 1993, 326, 477-481 ou Seltzman et al., Tet. Lett., 2000, 41, 3589-3592.

Les composés de formule (I) selon l'invention dans laquelle G est un atome de soufre, **R2** est un atome d'hydrogène et, R1 et R3, identiques ou différents, représentent un groupe CO-R ou CO-(CH₂)₂ₙ₊₁-X-R', peuvent être obtenus à partir du composé de formule (IX) par le procédé suivant : 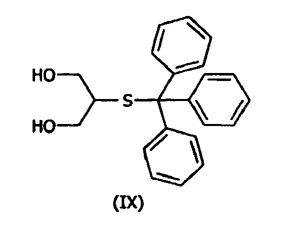
a) Réaction du composé IX et d'un premier composé de formule A°-CO-A3 dans laquelle A3 est un groupe réactif choisi par exemple entre OH, O-CO-A° et CI, et A° est le groupe R ou le groupe (CH₂)₂ₙ₊₁-X-R', puis d'un second composé de formule A°-CO-A3 dans laquelle, indépendamment du premier composé, A3 est un groupe réactif choisi par exemple entre OH, O-CO-A° et CI, et A° est le groupe R ou le groupe (CH₂)₂ₙ₊₁-X-R', en présence éventuellement d'agents de couplages ou d'activateurs connus de l'homme de métier,
b) Déprotection du groupement thiol par l'acétate mercurique.

Ce procédé est avantageusement réalisé selon le protocole décrit dans Aveta et al., Gazz. Chim. Ital., 1986, 116 (11), 649-652.

Les composés de formule (I) selon l'invention dans laquelle G est un atome de soufre, R2 et R3 sont des atomes d'hydrogène et, R1 représente un groupe COR ou CO-(CH₂)₂ₙ₊₁-X-R', peuvent être obtenus à partir du composé de formule (IX) par le procédé suivant :
a) Réaction du composé IX et d'un premier composé de formule A°-CO-A3 dans laquelle A3 est un groupe réactif choisi par exemple entre OH, O-CO-A° et Cl, et A° est le groupe R ou le groupe (CH₂)₂₊₁-X-R' en quantité stoechiométrique, en présence éventuellement d'agents de couplages ou d'activateurs connus de l'homme de métier,
b) Déprotection du groupement thiol par l'acétate mercurique.

Le composé de formule (IX) peut être préparé par un procédé comprenant :
a) réaction d'un 2-halogénomalonate de diméthyle avec le tritylthiol pour donner le composé de formule X 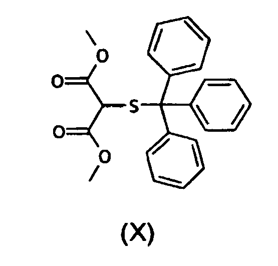
b) Réduction des fonctions acétate par LiAlH₄.

Les composés de formule (I) dans lesquels G est un groupe N-R4 et dans laquelle R1, R2 et R3 identiques ou différents, représentent un groupe CO-R ou un groupe CO-(CH₂)₂ₙ₊₁-X-R', sont obtenus à partir d'un composé de formule (I) dans laquelle G est un groupe N-R4, R1 et R3 sont des atomes d'hydrogène, R₂ un groupe CO-R ou un groupe CO-(CH₂)₂ₙ₊₁-X-R' (composé XI) selon le procédé suivant :

Réaction d'un composé XI et d'un premier composé de formule A°-CO-A2 dans laquelle A2 est un groupe réactif choisi par exemple entre OH et Cl, et A° est le groupe R ou le groupe (CH₂)₂ₙ₊₁-X-R', puis d'un second composé de formule A°-CO-A2 dans laquelle, indépendamment du premier composé, A2 est un groupe réactif choisi par exemple entre OH et Cl, et A° est le groupe R ou le groupe (CH₂)₂ₙ₊₁-X-R', en présence éventuellement d'agents de couplages ou d'activateurs connus de l'homme de métier.

Ce procédé est avantageusement réalisé selon le protocole décrit dans, Terradas et al., J. Amer. Chem. Soc., 1993, 115, 390-396.

Les composés de formule (I) selon l'invention dans laquelle G est un groupe N-R4 et dans laquelle R1 et R2 représentent un groupe CO-R ou CO-(CH₂)₂ₙ₋₁-X-R', et R3 est un atome d'hydrogène peuvent être obtenus par réaction d'un composé XI et d'un composé de formule A°-CO-A₂ dans laquelle A2 est un groupe réactif choisi par exemple entre OH et Cl, et A° est le groupe R ou le groupe (CH₂)₂ₙ₊₁-X-R' en quantité stoechiométrique, en présence éventuellement d'agents de couplages ou d'activateurs connus de l'homme de métier.

Les composés de formule (I) selon l'invention dans lesquels G est un groupe NH, R1 et R3 sont des atomes d'hydrogène, R2 un groupe CO-R ou un groupe CO-(CH₂)₂ₙ₊₁-X-R' (composé XIa) peuvent être obtenus de différentes façons.

Selon un premier procédé, on fait réagir une molécule de 2-aminopropane-1,3-diol avec un composé de formule A°-CO-A dans laquelle A est un groupe réactif choisi par exemple entre OH, O-CO-A°, OR" et CI, et A° est le groupe R ou le groupe (CH₂)₂₊₁-X-R' en présence éventuellement d'agents de couplages ou d'activateurs connus de l'homme de métier.

Cette réaction peut être mise en oeuvre en adaptant les protocoles décrits par exemple dans Daniher et Bashkin, Chem. Commun., 1998, 10, 1077-1078, Khanolkar et al., J. Med. Chem., 1996, 39, 4515-4519, Harada et al., Chem. Pharm. Bull., 1996, 44 (12), 2205-2212., Kurfuerst et al., Tetrahedron, 1993, 49 (32), 6975-6990., Shaban et al., Carbohydr. Res., 1977, 59, 213-233, Putnam et Bashkin, Chem. Commun., 2000, 767-768.

Les composés de formule (I) selon l'invention dans lesquels G est un groupe NH, R1 et R3 sont des atomes d'hydrogène, R2 un groupe CO-R ou un groupe CO-(CH₂)₂ₙ₊₁-X-R' (composé XIa) peuvent également être obtenus selon le procédé suivant :
a) Réaction du composé de formule XII avec un composé de formule A°-CO-A dans laquelle A est un groupe réactif choisi par exemple entre OH, O-CO-A°, OR" et Cl, et A° est le groupe R ou le groupe (CH₂)₂ₙ₊₁-X-R' en présence éventuellement d'agents de couplages ou d'activateurs connus de l'homme de métier 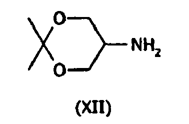 pour donner un composé de formule générale XIII 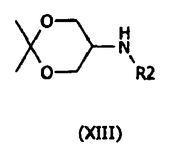
b) déprotection du composé XIII.

Ce procédé peut avantageusement être mis en oeuvre selon le protocole décrit dans Harada et al., Chem. Pharm. Bull., 1996, 44 (12), 2205-2212.

Les composés de formule (I) selon l'invention dans lesquels G est un groupe N-R4 dans lequel R4 n'est pas un atome d'hydrogène, R1 et R3 sont des atomes d'hydrogène, R2 un groupe CO-R ou un groupe CO-(CH₂)₂ₙ₊₁-X-R' (composé Xlb) peuvent être obtenus selon le procédé suivant :
a) Réaction du composé de formule XII avec un composé de formule A°-CO-A dans laquelle A est un groupe réactif choisi par exemple entre OH, O-CO-A°, OR" et Cl, et A° est le groupe R ou le groupe (CH₂)₂ₙ₊₁-X-R' en présence éventuellement d'agents de couplages ou d'activateurs connus de l'homme de métier. 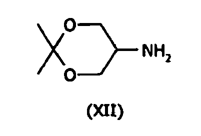 pour donner un composé de formule générale XIII 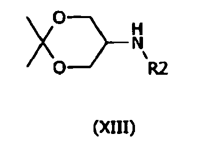
b) Réaction du composé XIII avec un composé de type R4-A4 dans lequel A4 est un groupe réactif choisi par exemple entre Cl ou Br, en milieu basique,
c) déprotection du composé XIII.
   La faisabilité, la réalisation et d'autres avantages de l'invention sont illustrés plus en détails dans les exemples qui suivent, qui doivent être considérés comme illustratifs et non limitatifs.

### Légende des figures :

Figure 1A: Structure d'acylglycérols selon l'invention (exemples 2a, 2c, 4a-n).
Figure 1 B: Structure de composés particuliers selon l'invention (exemples 5a-b).
Figure 2: Evaluation des effets de l'exemple 4a sur le métabolisme du cholestérol plasmatique chez le rat.

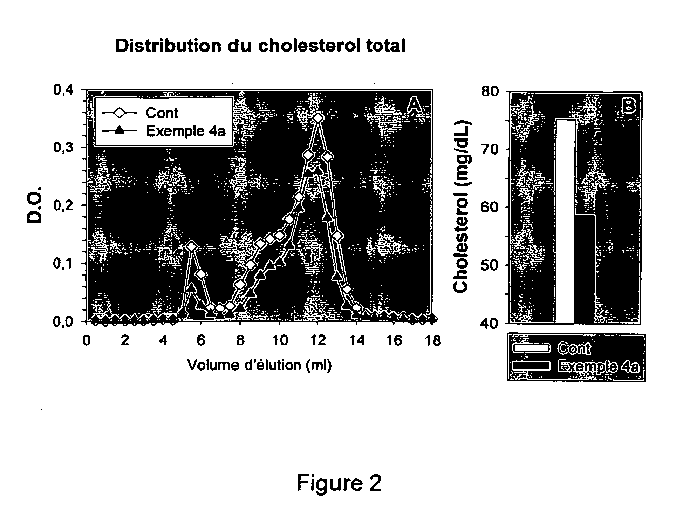

   Sur la figure 2A sont représentés les effets du traitement de rats Sprague-Dawley avec l'exemple 4a (300 mg/kg/j) sur la distribution du cholestérol dans les lipoparticules évaluée par chromatographie d'exclusion. On observe une distribution typique du cholestérol dans plusieurs populations de lipoparticules de tailles différentes. En outre, on observe une diminution du cholestérol dans les différentes classes de lipoparticules suite au traitement des animaux avec l'exemple 4a, particulièrement les particules de grande taille (VLDL, et LDL) et les particules de petite taille (HDL). Cette diminution est caractéristique des effets des activateurs de PPARα.
   Cette diminution se traduit par une diminution du cholestérol plasmatique total comme indiqué dans la figure 2B.
Figure 3 : Evaluation des effets de l'exemple 4a sur le métabolisme des triglycérides plasmatiques chez le rat.
   Sur la figure 3A sont représentés les effets du traitement de rats Sprague-Dawley avec l'exemple 4a (300 mg/kg/j) sur la distribution des triglycérides dans les lipoparticules évaluée par chromatographie d'exclusion. On observe une distribution typique des triglycérides principalement localisée dans une population de lipoparticules de grande taille. En outre, on observe une diminution des triglycérides dans cette classe de lipoparticules suite au traitement des animaux avec l'exemple 4a. Cette diminution est caractéristique des effets des activateurs de PPARα.
   Cette diminution se traduit par une diminution des triglycérides plasmatiques comme indiqué dans la figure 3B.
Figure 4 : Evaluation des effets de l'exemple 4a sur le métabolisme des phospholipides plasmatiques chez le rat.
   Sur la figure 4A sont représentés les effets du traitement de rats Sprague-Dawley avec l'exemple 4a (300 mg/kg/j) sur la distribution des phospholipides dans les lipoparticules évaluée par chromatographie d'exclusion. On observe une distribution typique des phospholipides dans plusieurs populations de lipoparticules de tailles différentes. En outre, on observe une diminution des phospholipides dans les différentes classes de lipoparticules suite au traitement des animaux avec l'exemple 4a, particulièrement les particules de grande taille (VLDL).
   Cette diminution se traduit par une diminution des phospholipides plasmatiques totaux comme indiqué dans la figure 4B.
Figure 5 : Evaluation des effets de l'exemple 4a sur l'expression de gènes hépatiques chez le rat.

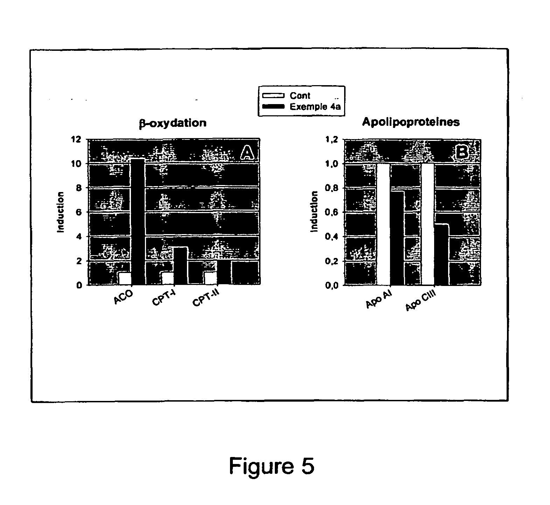

   Sur la figure 5A sont représentés les effets du traitement de rats Sprague-Dawley avec l'exemple 4a (300 mg/kg/j) sur l'expression hépatique des gènes impliqués dans la β-oxydation peroxysomale (ACO) ou mitochondriale (CPT-I et CPT-II) des acides gras. On observe une forte activation de l'expression hépatique de l'ACO, un des principaux gènes cibles de PPARα, qui suggère une forte activation de la capacité de catabolisme des acides gras par les peroxisomes hépatiques. En parallèle, nous observons une augmentation plus faible de l'expression des gènes CPT-I et CPT-II qui suggère une activation de la capacité de catabolisme des acides gras par les mitochondries hépatiques. Sur la figure 5B sont représentés les effets du traitement de rats Sprague-Dawley avec l'exemple 4a (300 mg/kg/j) sur l'expression hépatique des gènes impliqués dans le transport du cholestérol (Apo Al) ou des triglycérides (Apo CIII). On observe une légère diminution de l'expression hépatique de l'Apo Al qui pourrait expliquer en partie la diminution du taux plasmatique de cholestérol. On observe également une diminution plus nette de l'expression de l'Apo CIII qui pourrait expliquer en partie la diminution du taux plasmatique de triglycérides.
Figure 6 : Evaluation des effets de l'exemple 4a sur le métabolisme du cholestérol et des triglycérides plasmatiques chez le rat Zucker.

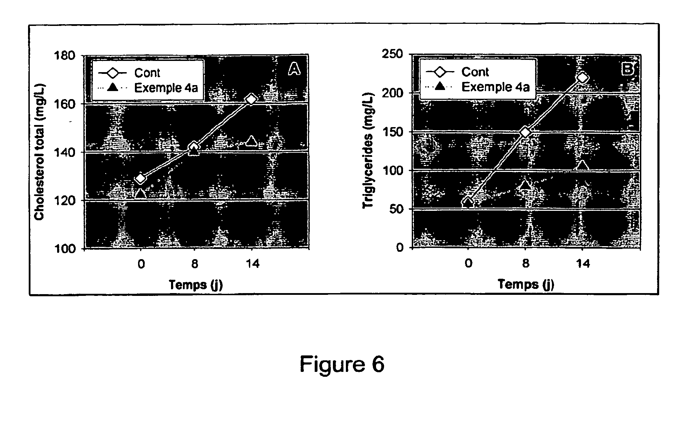

   Sur la figure 6A sont représentés les effets du traitement de rats Zucker avec l'exemple 4a (300 mg/kg/j) sur le taux plasmatique de cholestérol total. Les rats Zucker sont des rats résistants à l'action de l'insuline caractérisés par une augmentation graduelle du taux plasmatique de cholestérol. La figue 1a montre cette évolution dans le groupe contrôle. Elle montre également que cette évolution est freinée par le traitement des animaux avec l'exemple 4a. Sur la figure 6B sont représentés les effets du traitement de rats Zucker avec l'exemple 4a (300 mg/kg/j) sur le taux plasmatique de triglycérides. Les rats Zucker sont également caractérisés par une augmentation graduelle du taux plasmatique de triglycérides. La figure 6B montre cette évolution dans le groupe contrôle. Elle montre également que cette évolution est fortement ralentie par le traitement des animaux avec l'exemple 4a.
Figure 7: Evaluation des effets de l'exemple 4a sur le taux plasmatique d'insuline et l'équilibre glucidique chez le rat Zucker.

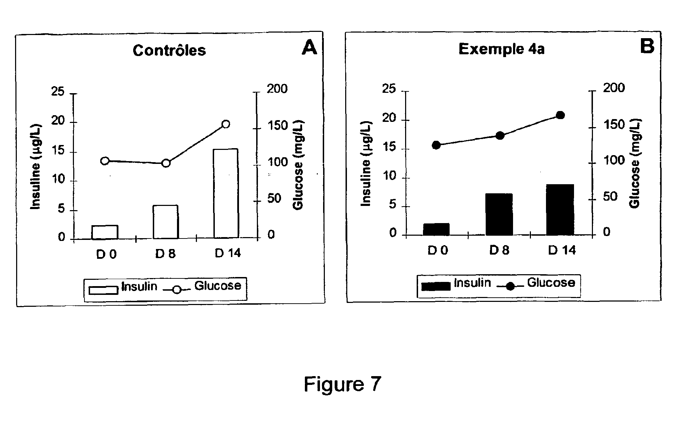

   Sur la figure 7 sont représentés les effets du traitement de rats Zucker avec l'exemple 4a (300 mg/kg/j) sur les taux plasmatiques d'insuline et de glucose. Les rats Zucker sont des rats résistant à l'action de l'insuline caractérisés par une augmentation compensatrice graduelle du taux plasmatique d'insuline. La figure 7A montre cette évolution dans le groupe contrôle. La figure 7B montre que cette évolution est freinée par le traitement des animaux avec l'exemple 4a, notamment après 14 jours. Les taux de glucose étant équivalents dans les deux groupes (figures 7A et 7B), le traitement avec l'exemple 4a augmente donc la sensibilité à l'insuline.

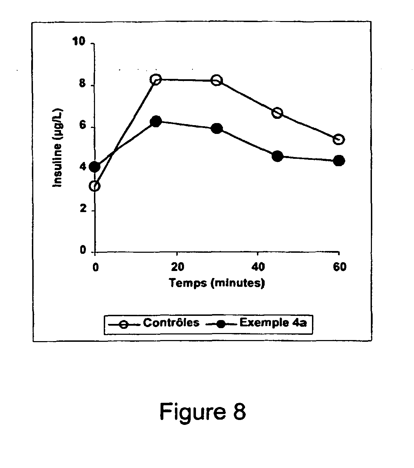

Figure 8 : Evaluation des effets de l'exemple 4a sur le taux d'insuline lors d'un test de tolérance au glucose chez le rat Zucker.
   Sur la figure 8 sont représentés les effets du traitement de rats Zucker avec l'exemple 4a (300 mg/kg/j) sur le taux plasmatique d'insuline lors d'un test de tolérance au glucose. Les résultats montrent que les rats traités avec l'exemple 4a utilisent moins d'insuline pour répondre à l'injection de glucose réalisée en début de test. Le traitement avec l'exemple 4a a donc pour effet d'augmenter la sensibilité à l'insuline.

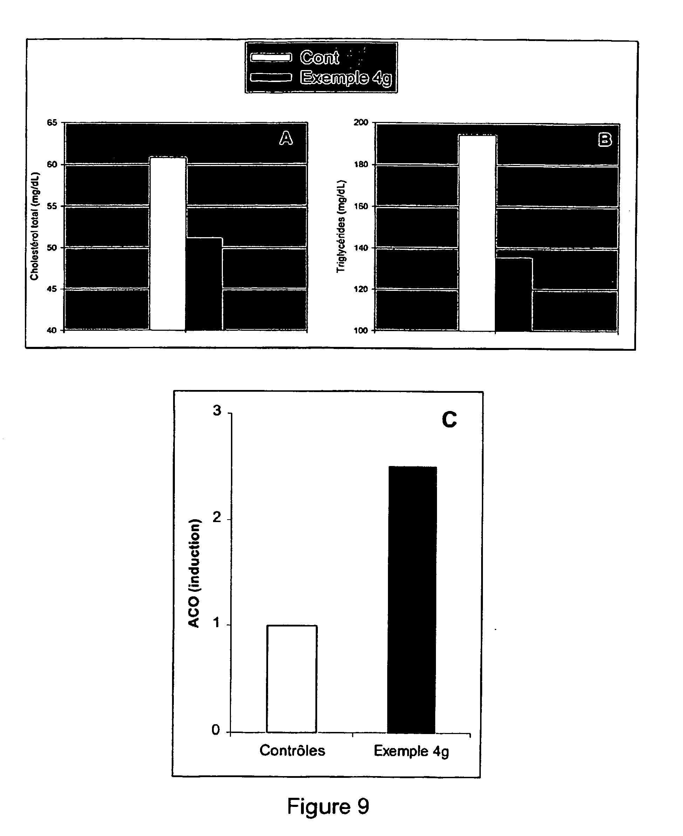

Figure 9 : Evaluation des effets de l'exemple 4g sur les taux de lipides plasmatiques et sur l'expression d'un gène hépatique chez le rat.
   Sur les figures 9A et 9B sont représentés les effets du traitement de rats Wistar avec l'exemple 4g (300 mg/kg/j) sur le taux plasmatique de cholestérol et sur la concentration plasmatique des triglycérides respectivement, après 14 jours de traitement. Les résultats montrent que le traitement a pour effet une diminution nette des taux circulants de cholestérol et de triglycérides. Sur la figure 9C est représenté l'effet du traitement de rats Wistar avec l'exemple 4g (300 mg/kg/j) sur l'expression hépatique d'un gène impliqué dans la β-oxydation peroxysomale des acides gras (ACO). On observe une forte activation de l'expression hépatique de l'ACO, un des principaux gènes cibles de PPARα.

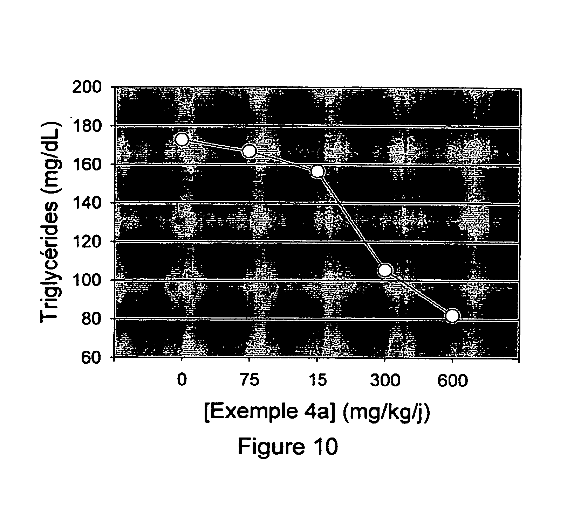

Figure 10: Evaluation de la dépendance vis-à-vis de la dose de l'effet de l'exemple 4a sur le métabolisme des triglycérides plasmatiques chez le rat Zucker.
   Sur la figure 10 sont représentés les effets du traitement de rats Zucker avec l'exemple 4a testé à des doses de 0 à 600 mg/kg/j sur le taux plasmatique de triglycérides. Elle montre une diminution graduelle de cette concentration en fonction de la dose de composé.

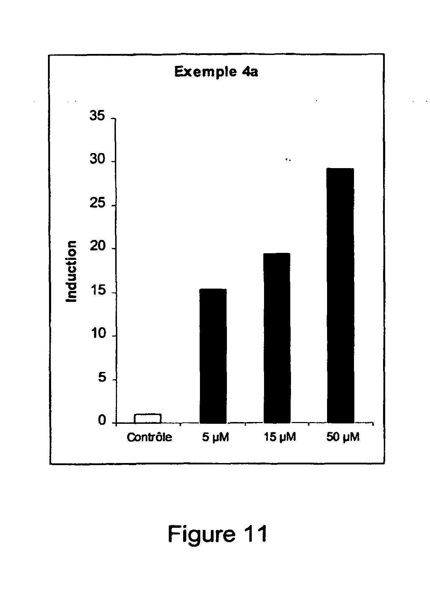

Figure 11 : Evaluation des effets de l'exemple 4a sur l'activation de PPARα *in vitro.*
   Sur la figure 11 sont représentés les effets de l'exemple 4a sur l'activation de PPARα évaluée *in vitro* dans les cellules HepG2, en utilisant une chimère constituée du domaine de liaison à l'ADN du facteur de transcription Gal4 et du domaine de liaison du ligand de PPARα. Les résultats montrent que l'exemple 4a est capable d'activer très fortement le récepteur nucléaire PPARα, et ceci d'une manière dose dépendante.

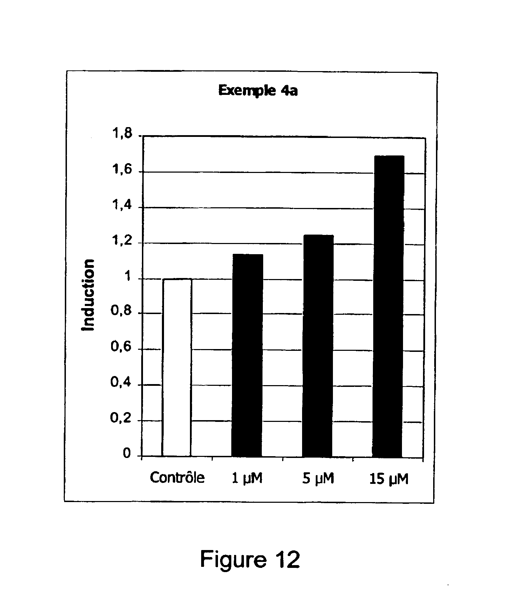

Figure 12: Evaluation des effets de l'exemple 4a sur l'expression du gène ABCA1 *in vitro* dans les macrophages humains.
   Sur la figure 12 sont représentés les effets de l'exemple 4a solubilisé selon l'exemple 6, sur l'expression du gène ABCA-1 *in vitro* dans les macrophages humains préparés selon l'exemple 12. Les résultats montrent que l'exemple 4a est capable d'activer très fortement l'expression du gène ABCA1 dans les macrophages humains, et ceci d'une manière dose dépendante.

### EXEMPLES :

### EXEMPLE 1 : Préparation de dérivés d'acides gras

### EXEMPLE 1a : Préparation de l'acide tétradécylthioacétique

L'hydroxyde de potassium (34.30 g, 0.611 mol), l'acide mercaptoacétique (20.9 ml, 0.294 mol) et le 1-bromotétradécane (50 ml, 0.184 mol) sont ajoutés dans l'ordre au méthanol (400 ml). Ce mélange est placé sous agitation pendant une nuit à température ambiante. Au mélange réactionnel précédent est alors ajoutée une solution d'acide chlorhydrique concentré (60 ml) dissous dans l'eau (800 ml). L'acide tétradécylthioacétique précipite. Le mélange est laissé sous agitation une nuit à température ambiante. Le précipité est ensuite filtré, lavé cinq fois à l'eau puis séché au dessiccateur. Le produit est recristallisé dans le méthanol (rendement 94%).
Rf (CH₂Cl₂-MeOH 9-1) : 0.60
F°: 67-68°C
IR: νCO acide 1726 et 1684 cm⁻¹
RMN(¹H, CDCl₃): 0.84-0.95 (t, 3H, -CH₃, J = 6.5 Hz) ; 1.20-1.45 (massif, 22H, - CH₂-) ; 1.55-1.69 (quint, 2H, **-CH₂**-CH₂-S-, J=6.5Hz) ; 2.63-2.72 (t, 2H, CH₂-CH₂-S-, J = 7.3 Hz) ; 3.27 (s, 2H, S-**CH₂**-COOH)
SM : M-1 = 287

### EXEMPLE 1b: Préparation de l'acide 4-(dodécylthio)butanoïque

Le dodécanethiol (2.01 g ; 10 mmol) et le bromobutyrate d'éthyle (1.971 g ; 10 mmol) sont placés sous agitation à température ambiante sous atmosphère inerte. L'hydroxyde de potassium (1.36 g ; 21 mmol) dissous dans 50 ml d'éthanol est ajouté lentement. Le mélange réactionnel est porté à reflux pendant 3 heures. L'éthanol est évaporé sous vide. Le résidu est repris par l'eau et acidifié. Le précipité formé est filtré, lavé à l'eau et séché (rendement 90%).
Rf (CH₂Cl₂-MeOH 9-1) : 0.46
IR: νCO acide 1689 cm⁻¹
RMN (¹H, CDCl₃) : 0.86-0.91 (t, 3H, -CH₃, J = 6.2 Hz) ; 1.25-1.45 (massif, 18H, - CH₂-) ; 1.53-1.63 (quint, 2H, -**CH₂**-CH₂-S-, J=6.7Hz) ; 1.87-2.00 (quint, 2H, - CH₂-S-**CH₂**-CH₂-CH₂-COOH, J=7.2Hz) ; 2.47-2.55 (m, 4H, -**CH₂**-S-**CH₂**-CH₂-CH₂-COOH) ; 2.55-2.62 (t, 2H, -CH₂-S-CH₂-CH₂-**CH₂**-COOH)
SM : M-1 = 287

### EXEMPLE 1c: Préparation de l'acide 6-(décylthio)hexanoïque

Le décanethiol (4.57 g ; 25 mmol) et l'acide 4-bromobutyrique (5 g ; 25 mmol) sont placés sous agitation à température ambiante sous atmosphère inerte. L'hydroxyde de potassium dissous dans 50 ml d'éthanol est ajouté lentement. Le mélange réactionnel est porté à reflux pendant 3 heures. L'éthanol est évaporé sous vide. Le résidu est repris par l'eau et acidifié. Le précipité formé est filtré, lavé à l'eau et séché (rendement 95%).
Rf (CH₂Cl₂-MeOH 9-1) : 0.37
IR: vCO acide 1690 cm⁻¹
RMN (¹H, CDCl₃) : 0.86-0.91 (t, 3H, -CH₃, J = 6.5 Hz) ; 1.22-1.41 (massif, 14H, - CH₂-) ; 1.42-1.50 (m, 2H, CH₂-S-CH₂-CH₂-**CH₂**-CH₂-CH₂-COOH) ; 1.53-1.75 (massif, 6H, -**CH₂-**CH₂-S-CH₂-**CH₂**-CH₂-**CH₂**-CH₂-COOH); 2.35-2.42- (t, 2H, -CH₂-S-CH₂-CH₂-CH₂-CH₂-**CH₂-**COOH); 2.48-2.55 (massif, 4H, **-CH₂-S-CH₂-).**
SM : M-1 = 287

### EXEMPLE 1 d : Préparation de l'acide tétradécylsélénoacétique

### Préparation du tétradécyldisélénure

Sous atmosphère inerte, du sélénium (1.19 g ; 15 mmol) est introduit dans un mélange THF/eau (1/1) (50 ml). Le mélange réactionnel est refroidi dans un bain de glace avant d'ajouter lentement le tétraborohydrure de sodium (1.325 g ; 35 mmol). Une seconde fraction de sélénium (1.19 ; 15 mmol) est ajoutée. Le mélange réactionnel est maintenu sous agitation à température ambiante pendant 15 min puis chauffé à reflux pour dissoudre tous les réactifs. Le bromotétradécane (9 ml ; 30 mmol) dissous dans 25 ml de THF est ajouté. Le mélange réactionnel est laissé sous agitation à température ambiante pendant 3 heures. Le milieu réactionnel est alors extrait au dichlorométhane. Les phases organiques sont groupées, séchées sur du sulfate de magnésium, filtrées et portées à sec. Le produit est utilisé sans autre purification.
Rf (éther de pétrole) : 0.77
F° : 43°C (cristaux jaunes)
IR: vCH 2960-2850 cm⁻¹
RMN (¹H, CDCl₃) : 0.87-0.93 (t, 6H, -CH₃, J = 6.5 Hz) ; 1.20-1.48 (massif, 40H, - CH₂-) ; 1.62-1.80 (m, 4H, -CH₂-CH₂-Se-) ; 2.88-2.96 (t, 4H, -CH₂-CH₂-Se-).

### Préparation de l'acide tétradécylsélénoacétique

Sous atmosphère inerte, le ditétradécyldisélénure (8.5 g ; 17 mmol) est dissous dans un mélange THF/eau (150 ml/50 ml) et refroidi dans un bain de glace. Le tétraborohydrure de sodium (2.9 g ; 61 mmol) est ajouté lentement (la solution se décolore) puis est ajouté l'acide bromoacétique (8.5 g ; 61 mmol) dissous dans un mélange THF/eau (25 ml /25 ml). Le mélange réactionnel est maintenu sous agitation à température ambiante pendant 6 heures. Le mélange réactionnel est extrait à l'éther puis la phase aqueuse est acidifiée. Le précipité obtenu est filtré, lavé plusieurs fois à l'eau et séché (rendement 29%).
Rf (CH₂Cl₂-MeOH 9-1) :0.60
F°: 68°C
IR: νCO acide 1719 et 1680 cm⁻¹
RMN (¹H, CDCl₃) : 0.85-0.95 (t, 3H, -CH₃, J = 6.5 Hz) ; 1.25-1.48 (massif, 22H, - CH₂-) ; 1.65-1.78 (quint, 2H, -CH₂-CH₂-Se-, J=6.5Hz); 2.78-2.84 (t, 2H, CH₂-CH₂-Se-, J = 7 Hz) ; 3.18 (s, 2H, Se-CH₂-COOH)
SM : M-1 = 335

### EXEMPLE 1e : Préparation de l'acide tétradécylsulfoxyacétique

L'acide tétradécylthioacétique (5 g ; 17.4 mmol) (exemple 1a) est dissous dans un mélange méthanol/dichlorométhane (160 ml / 80 ml). Le mélange réactionnel est mis sous agitation et refroidi dans un bain de glace avant d'ajouter lentement l'oxone^{®} (12.8 g ; 21 mmol) dissous dans l'eau (160 ml). Le mélange réactionnel est maintenu sous agitation à température ambiante pendant 3 heures. Les solvants sont évaporés sous vide. Le précipité formé dans la phase aqueuse résiduelle est essoré, lavé plusieurs fois à l'eau et séché (rendement 90%).
Rf (CH₂Cl₂-MeOH 9-1) : 0.27
IR: νCO acide 1723 et 1690 cm⁻¹
RMN (¹H, DMSO): 0.80-0.92 (t, 3H, -CH₃, J = 6.4 Hz) ; 1.19-1.50 (massif, 22H, - CH₂-); 1.55-1.71 (quint, 2H, **-CH₂**-CH₂-SO-); 2.70-2.89 (t, 2H, -CH₂-**CH₂**-SO-CH₂-COOH, J=6.7Hz); 3.52-3.70 (d, 1H, -CH₂-SO-**CH₂**-COOH, J=14.5Hz); 3.80-3.95 (d, 1H, -CH₂-SO-**CH₂**-COOH, J=14.1Hz).
SM : M+1 = 305 ; M+23 = 327 (M+Na⁺); M+39 = 343 (M+K⁺)

### EXEMPLE 1f: Préparation de l'acide 6-(décylsulfoxy)hexanoïque

Le produit est préparé selon la procédure précédemment décrite (exemple 1e) à partir de l'acide 6-(décylthio)hexanoïque(exemple 1c) rendement 94%.
Rf (CH₂Cl₂-MeOH 9-1) : 0.18
RMN (¹H, CDCl₃) : 0.86-0.91 (t, 3H, -CH₃, J = 6.8 Hz) ; 1.20-1.40 (massif, 14H, - CH₂-) ; 1.40-1.60 (m, 2H, CH₂-SO-CH₂-CH₂-**CH₂**-CH₂-CH₂-COOH) ; 1.63-1.95 (massif, 6H, **-CH₂**-CH₂-S-CH₂-**CH₂**-CH₂-**CH₂**-CH₂-COOH) ; 2.35-2.42 (m, 3H, - CH₂-S-CH₂-CH₂-CH₂-CH₂-**CH₂**-COOH et -CH₂-SO-**CH₂**-CH₂-CH₂-CH₂-CH₂-COOH) ; 2.60-2.71 (m, 1H, -CH₂-SO-**CH₂**-CH₂-CH₂-CH₂-CH₂-COOH); 2.75-2.85 (m, 1H, - **CH₂**-SO-(CH₂)₅-COOH) ; 2.80-3.01 (m, 1H, -**CH₂**-SO-(CH₂)₅-COOH).

### EXEMPLE 1g: Préparation de l'acide tétradécylsulfonylacétique

L'acide tétradécylthioacétique (5 g ; 17.4 mmol) (exemple 1a) est dissous dans un mélange méthanol/dichlorométhane (160 ml / 80 ml). Le mélange réactionnel est placé sous agitation et refroidi dans un bain de glace avant d'ajouter lentement l'oxone^{®} (21.8 g ; 35 mmol) dissous dans l'eau (160 ml). Le mélange réactionnel est maintenu sous agitation à température ambiante pendant 3 heures. Les solvants sont évaporés sous vide. Le précipité formé dans la phase aqueuse résiduelle est essoré, lavé plusieurs fois à l'eau et séché (rendement 89%).
Rf (CH₂Cl₂-MeOH 9-1) : 0.21
IR: νCO acide 1701 cm⁻¹
RMN (¹H, DMSO) : 0.85-0.96 (t, 3H, -CH₃, J = 6 Hz) ; 1.20-1.40 (massif, 22H, - CH₂-) ; 1.40-1.55 (m, 2H, **-CH₂**-CH₂-CH₂-SO₂-) ; 1.80-1.96 (m, 2H, **-CH₂**-CH₂-SO₂-) ; 3.22-3.34 (t, 2H, -CH₂**-CH₂**-SO₂-CH₂-COOH, J=8Hz) ; 4.01 (s, 2H, -CH₂-SO₂**-CH₂**-COOH).
SM : M-1 = 319

### EXEMPLE 1h : Préparation de l'acide 6-(décylsulfonyl)hexanoïque

Le produit est obtenu selon la procédure précédemment décrite (exemple 1g) à partir de l'acide 6-(décylthio)hexanoïque (exemple 1c). rendement 87%.
Rf (CH₂Cl₂-MeOH 9-1) : 0.15
IR: νCO acide 1689 cm⁻¹
RMN (¹H, CDCl₃) : 0.85-0.96 (t, 3H, -CH₃, J = 6.5 Hz) ; 1.22-1.40 (massif, 12H, - CH₂-) ; 1.40-1.61 (massif, **4H**, -SO₂CH₂-CH₂-**CH₂**-) ; 1.65-1.95 (massif, 6H, - **CH₂**-CH₂-SO₂-**CH₂**-CH₂-CH₂-**CH₂**-CH₂-COOH) : 2.35-2.46 (m, 2H, -**CH₂**-COOH) ; 2.60-2.84 (m, 2H, -**CH₂**-SO₂-CH₂-CH₂-CH₂-CH₂-CH₂-COOH) ; 2.90-3.02 (m, 2H, - CH₂-SO₂-**CH₂**-CH₂-CH₂-CH₂-CH₂-COOH).

### EXEMPLE 1i: Préparation de l'acide docosylthioacétique

Le produit est obtenu selon la procédure précédemment décrite (exemple 1a) à partir de l'acide mercaptoacétique et du bromodocosane.
Rendement 90%
Rf (CH₂C)₂-MeOH 9-1) : 0.62
IR: vCO acide 1728 et 1685 cm⁻¹
RMN (¹H, CDCl₃) : 0.83-0.94 (t, 3H, -CH₃, J = 6.6 Hz) ; 1.18-1.48 (massif, 38H, - CH₂-) ; 1.55-1:69 (quint, 2H, **-CH₂**-CH₂-S-, J = 6.7 Hz) ; 2.63-2.72 (t, 2H, CH₂-**CH₂**-S-, J = 7.3 Hz) ; 3.26 (s, 2H, S-**CH₂**-COOH)

### EXEMPLE 2: Préparation de monoacylglycérols

### EXEMPLE 2a : Préparation du 1-tétradécylthioacétylglycérol

### Préparation du tétradécylthioacétate de (2,3-O-isopropylidène)propyle

Dans un ballon immergé dans un bain de glace, l'acide tétradécylthioacétique (4 g, 13.86 mmol) est dissous dans le tétrahydrofuranne (100 ml) avant d'ajouter l'EDCI (2.658 g, 13.86 mmol), la diméthylaminopyridine (1.694 g, 13.86 mmol) puis le solketal (1.72 ml, 13.86 mmol) dans cet ordre. Le mélange réactionnel est laissé sous agitation à température ambiante pendant 4 jours. Le solvant est évaporé sous vide. Le résidu est repris par le dichlorométhane, lavé par une solution aqueuse d'HCl 1 N puis par une solution aqueuse de NaHCO₃ 10% et enfin par une solution saturée en NaCl. La phase organique est séchée sur sulfate de magnésium, filtrée et évaporée sous vide. L'huile résiduelle obtenue est purifiée par chromatographie sur gel de silice (acétate d'éthyle-cyclohexane 1-9). Le produit est obtenu sous forme d'huile jaune (rendement 80%).
Rf (cyclohexane-acétate d'éthyle 8-2) : 0.65
IR: vCOester 1736 cm⁻¹
RMN (¹H, CDCl₃) : 0.86 (t, 3H, -CH₃, J = 7.8 Hz) ; 1.25 (massif, 20H, -CH₂-); 1.33 (s, 3H, CH₃ isopropylidène) ; 1.37 (s, 3H, CH₃ isopropylidène) ; 1.59 (m, 4H, OCO-CH₂-**CH₂**-CH₂-); 2.62 (t, 2H, -O-CO-CH₂-S-**CH₂**-, J = 7.40 Hz) ; 3.25 (s, 2H, -O-CO**-CH₂-**S-CH₂-) ; 3.75 (m, 1H, -CO-O-CH₂**-CH**(isopropylidène)) ; 4.08 (m, 2H, -CO-O-CH₂CH**CH₂**(isopropylidène)) ; 4.18 (m, 1H, -CO-O**-CH₂-**isopropylidène) ; 4.35 (m, 1 H, -CO-O**-CH₂-**isopropylidène).

### Préparation du 1-tétradécylthioacétylglycérol

Le tétradécylthioacétate de (2,3-O-isopropylidène)propyle (4.163 g, 10.356 mmol) est dissous dans l'acide acétique (60 ml) et laissé sous agitation à température ambiante. Après 11 jours de réaction, le mélange réactionnel est dilué dans l'eau puis extrait par de l'acétate d'éthyle. La phase organique est lavée par une solution aqueuse saturée en NaCl puis séchée sur sulfate de magnésium, filtrée et le solvant est évaporé. La poudre blanche obtenue est recristallisée dans l'heptane (rendement 90%).
Rf (acétate d'éthyle-cyclohexane 5-5) : 0.30
F : 63-65°C
IR: vCOester 1720 cm⁻¹
RMN (¹H, CDCl₃) : 0.89 (t, 3H, -CH₃, J = 6.6 Hz) ; 1.28 (massif, 20H, -CH₂-) ; 1.59 (massif, 4H, **-CH₂-CH₂**-CH₂-S-) ; 2.64 (t, 2H, CH₂-CH₂-S-, J = 7.23 Hz) ; 3.26 (s, 2H, S-CH₂-COOH) ; 3.64 (m, 2H, -COO-**CH₂**-CHOH-CH₂OH) ; 3.97 (m, 1 H, -COO-CH₂-**CHOH**-CH₂OH) ; 4.27 (m, 2H, -COO-CH₂-CHOH-**CH₂**OH).
SM : M+23 = 385 (M+Na⁺) (M+H non détectée)

### EXEMPLE 2b : Préparation du 1-palmitoylglycérol

Ce composé est synthétisé selon la procédure précédemment décrite (exemple 2a) à partir du solketal et de l'acide palmitique.
*Palmitate de (2,3-O-isopropylidène)propyle*
   rendement 55%
   Rf (CH₂Cl₂): 0.35
   F : 32-33°C
   IR: vCOester 1733 cm⁻¹
   RMN (¹H, CDCl₃): 0.89 (t, 3H, -CH₃, J = 6.6 Hz) ; 1.27 (massif, 24H, -CH₂-) ; 1.39 (s, 3H, CH₃ isopropylidène) ; 1.45 (s, 3H, CH₃ isopropylidène) ; 1.62 (m, 4H, OCO-CH₂-CH₂-CH₂-) ; 2.32.(t, 2H, -O-CO-**CH₂**-CH₂-CH₂-, J =7.40.Hz) ;.3.75 (dd, 1H, -CO-O-CH₂-**CH**(isopropylidène), J=8.3Hz et J=2.1Hz) ; 4.10 (m, 2H, -CO-O-CH₂-CHCH₂(isopropylidène)); 4.18 (dd, 1H, -CO-O-**CH₂**-isopropylidène, J=11.6Hz et J=4.6Hz) ; 4.33 (m, 1 H, -CO-O-**CH₂**-isopropylidène).
*1 palmitoylglycérol*
   rendement 84%
   Rf (acétate d'éthyle-cyclohexane 5-5) : 0.30
   F : 72-74°C
   IR: vCOester 1730 cm⁻¹
   RMN (¹H, CDCl₃) : 0.89 (t, 3H, -CH₃, J = 6.5 Hz) ; 1.26 (massif, 24H, -CH₂-) ; 1.64 (m, 2H, OCO-CH₂-**CH₂**-CH₂-) ; 2.36 (t, 2H, -O-CO-**CH₂**-CH₂-CH₂-, J = 7.40 Hz) ; 3.60 (dd, 1H, -CO-O-CH₂-CHOH-**CH₂**OH, J=11.8Hz et J=6.1Hz) : 3.71 (dd, 1H, -CO-O-CH₂-CHOH-**CH₂**OH, J=11.8Hz et J=3.9Hz); 3.94 (m, 1H**,** -CO-O-CH₂-CHOH-CH₂OH) ; 4.19 (m, 2H, -CO-O-**CH₂**-CHOH-CH₂OH) ;

### EXEMPLE 2c : Préparation du 2-tétradécylthioacétylglycérol

### Préparation du 1,3-benzylidèneglycérol

Le glycérol (30 g ; 0.326 mol), le benzaldéhyde (34.5 g ; 0.326 mol) et l'acide p-toluène sulfonique (50 mg) sont dissous dans 350 ml de toluène et placés à reflux dans un appareil de Dean-Stark pendant 18 heures. Le mélange réactionnel est porté à sec. Le produit résiduel est purifié par chromatographie sur gel de silice (éluant : cyclohexane/acétate d'éthyle 8/2 puis 7/3) puis recristallisé (rendement : 20%)
Rf (acétate d'éthyle-cyclohexane 5-5) : 0.34
IR: vOH 3286 cm⁻¹
RMN (¹H, CDCl₃) : 3.19 (sl, 1H échangeable, -OH) ; 3.64 (sl, 1H, -O-CH₂-CHOH-CH₂O-) ; 3.99-4.16 (dd, 2H, -O-C**Ha**Hb-CHOH-CHaHbO-, J=1.1 Hz et J=10.4Hz) ; 4.17-4.23 (dd, 2H, -O-CHa**Hb**-CHOH-CHa**Hb**O-, J=1.6Hz et J=11.5Hz) ; 5.57 (s, 1H, Φ-CH-) 7.34-7.45 (m, 3H, H aromatiques) ; 7.49-7.55 (m, 2H, H aromatiques).

### Préparation du tétradécylthioacétate de (1,3-O-benzylidène)propyle

Dans un ballon immergé dans un bain de glace, l'acide tétradécylthioacétique (0.800 g, 2.774 mmol) est dissous dans le THF (75 ml) avant d'ajouter l'EDCI (0.532 g, 2.774 mmol), la diméthylaminopyridine (0.339 g, 2.774 mmol) puis le 1,3-benzylidèneglycérol (0.5 g, 2.774 mmol) dans cet ordre. Le mélange est laissé sous agitation à température ambiante pendant 16 heures. Le solvant est évaporé. Le résidu obtenu est repris par le dichlorométhane, lavé par une solution d'acide chlorhydrique 1 N puis par une solution de carbonate de potassium à 10% et enfin par une solution aqueuse saturée en sel. La phase organique est séchée sur MgSO₄, filtrée et portée à sec. Le résidu est repris dans l'éther de pétrole. Le précipité formé est filtré puis purifié par chromatographie sur gel de silice (éluant : acétate d'éthyle-cyclohexane 2-8) et permet d'obtenir le produit souhaité sous forme de poudre blanche (rendement 50%).
Rf (acétate d'éthyle-cyclohexane 2-8) : 0.53
F : 51-53°C
IR: νCO ester 1723 cm⁻¹
RMN (¹H, CDCl₃) : 0.85-0.96 (t, 3H, CH₃, J=6.8Hz) ; 1.19-1.44 (massif, 20H, - CH₂) ; 1.52-1.69 (massif, 4H, -**CH₂-CH₂**-CH₂-S-) ; 2.62-2.80 (t, 2H, -CH₂-CH₂-**CH₂**-S-, J=7.2Hz) ; 3.34 (s, 2H, -CH₂-S-**CH₂**-COO-) ; 4.12-4.29 (dd, 2H, -O-C**Ha**Hb-CHOH-CHaHbO-, J=1.7Hz et J=13.1Hz) ; 4.30-4.41 (dd, 2H, -O-CHa**Hb-**CHOH-CHaHbO-, J=1.3Hz et J=13.1Hz) ; 4.75-4.79 (t, 1H, -O-CH₂-CHOH-CH₂O-, J=1.7Hz) ; 5.59 (s, 1H, Φ-CH-) ; 7.35-7.45 (m, 3H, H aromatiques) ; 7.48-7.57 (m, 2H, H aromatiques).

### Préparation du 2-tétradécylthioacétyiglycérol

Le tétradécylthioacétate de (1,3-O-benzylidène)propyle (0.576 g, 1.278 mmol) est dissous dans un mélange de dioxanne et de triéthylborate (50-50 v/v) avant d'ajouter l'acide borique (0.317 g, 5.112 mmol). Le mélange réactionnel est chauffé à 100°C pendant 4 heures. Sont encore ajoutés 2 équivalents d'acide borique (0.158 g, 2.556 mmol) puis 2 équivalents après 5.5 heures et 7 heures de réaction. Après 24 heures de réaction, le triéthylborate est évaporé. Le résidu est repris par de l'acétate d'éthyle et lavé par l'eau. La phase aqueuse est neutralisée avec NaHCO₃ puis extraite avec le dichlorométhane. La phase organique est lavée avec de l'eau saturée en sel, séchée sur MgSO₄, filtrée et portée à sec. Le résidu est purifié par chromatographie sur gel de silice (éluant acétate d'éthyle-cyclohexane 5-5) (rendement 62%).
Rf (acétate d'éthyle-cyclohexane 7-3) : 0.51
IR: νCO ester 1739 cm⁻¹
RMN (¹H, CDCl₃) : 0.82-0.95 (t, 3H, -CH₃, J = 6.9 Hz) ; 1.15-1.35 (massif, 22H,-CH₂-) ; 1.55-1.68 (m, 2H, **-CH₂**-CH₂-S-) ; 2.23 (sl, 2H, OH) ; 2.65 (m, 2H, CH₂-CH₂-S-) ; 3.26 (s, 2H, S-**CH₂**-COOH) ; 3.64-3.73 (m, 4H, -COO-**CH₂-**CHOH-CH₂OH) ; 3.97 (m, 1 H, -COO-CH₂-**CH**OH-CH₂OH).

### EXEMPLE 3 : Préparation de 1,3-diacylglycérols

### EXEMPLE 3a : Préparation du 1,3-dipalmitoylglycérol

Le glycérol (10 g ; 0.109 mol ; 1eq), l'acide palmitique (55.69 g ; 0.217 mol ; 2 eq), la dicyclohexylcarbodiimide (44.77 g ; 0.217 mol ; 2eq) et la diméthylaminopyridine (26.51 g ; 0.217 mol ; 2eq) sont dissous dans le dichlorométhane. Le mélange réactionnel est placé sous agitation à température ambiante pendant 48 heures. La dicyclohexylurée formée est filtrée et lavée plusieurs fois au dichlorométhane. Le filtrat est porté à sec. Le produit résiduel est purifié par chromatographie sur gel de silice (éluant : dichlorométhane) (rendement : 45%).
Rf (CH₂Cl₂): 0.30
F°: 70-73°C
IR: vCO ester 1735 et 1716 cm⁻¹
RMN (¹H, CDCl₃) : 0.86-91 (t, 6H, -CH₃, J = 6.5 Hz) ; 1.27 (massif, 48H, -CH₂-) ; 1.60-1.65 (quint, 4H, OCOCH₂-**CH₂**-, J=7.4Hz) ; 2.32-2.38 (t, 4H, OCO**CH₂**-CH₂-, J=7.6.Hz); 2.51-2.52 (d, 1H, OH (échangeable)) ; 4.06-4.21 (massif, 5H, -**CH₂-CH-CH₂**-)
SM : M+23 = 591 (M+Na⁺) ; M+39 = 607 (M+K⁺) ; (M+H non détectée)

### EXEMPLE 3b : Préparation du 1,3-dilinoléylglycérol

Ce composé est obtenu selon la procédure précédemment décrite (exemple 3a) à partir du glycérol et de l'acide linoléique. Le produit est obtenu sous forme d'huile incolore (rendement 26%).
Rf (CH₂Cl₂) : 0.30
IR: νCO ester 1743 et 1719 cm⁻¹
RMN (¹H, CDCl₃) : 0.83-0.93 (t, 6H, -CH₃, J = 6.5 Hz) ; 1.15-1.44 (massif, 28H, - CH₂-) ; 1.55-1.70 (quint, 4H, OCOCH₂-**CH₂-**, J=7.4Hz) ; 1.90-2.15 (massif, 8H, - **CH₂**-CH=CH-CH₂-CH=CH-**CH₂-**); 2.30-2.41 (t, 4H, OCO**CH₂**-CH₂- J=7.6 Hz) ; 2.48-2.52 (d, 1H, OH (échangeable)) ; 2.70-2.83 (t, 4H, -CH₂-CH=CH-**CH₂-**CH=CH-CH₂-) ; 4.05-4.25 (massif, 5H, -C**HaHb**-CH-CH**aHb**-) ; 5.25-5.46 (m, 8H, -CH₂-**CH**=**CH**-CH₂-**CH**=**CH**-CH₂-).
SM : M+23 = 639 (M+Na⁺) ; M+39 = 655 (M+K⁺) ; (M+H non détectée)

### EXEMPLE 3c : Préparation du 1,3-distéarylglycérol

Ce composé est obtenu selon la procédure précédemment décrite (exemple 3a) à partir du glycérol et de l'acide stéarique. Le produit est obtenu sous forme d'une poudre blanche (rendement 21%).
Rf (CH₂Cl₂): 0.30
IR: νCO ester 1735 et 1716 cm⁻¹
RMN (¹H, CDCl₃): 0.83-0.91 (t, 6H, -CH₃, J = 6.5 Hz) ; 1.27 (massif, 56H, -CH₂-) ; 1.59-1.66 (quint, 4H, OCOCH₂-**CH₂**-, J=7.4Hz) ; 2.33-2.38 (t, 4H, OCO**CH₂**-CH₂-, J=7.5 Hz) ; 2.45-2.47 (d, 1H, OH (échangeable), J=4.3Hz); 4.08-4.23 (massif, 5H, **-CHaHb-CH-CHaHb-).**
SM : M+23 = 647 (M+Na⁺); (M+H non détectée)

### EXEMPLE 3d: Préparation du 1,3-dioléylglycérol

Ce composé est obtenu selon la procédure précédemment décrite (exemple 3a) à partir du glycérol et de l'acide oléique.Le produit est obtenu sous forme d'huile incolore (rendement 15%).
Rf (CH₂Cl₂) : 0.23
IR: vCO ester 1743 et 1720 cm⁻¹
RMN (¹H, CDCl₃) : 0.89 (t, 6H, -CH₃, J = 7.2 Hz) ; 1.30 (massif, 40H, -CH₂-) ; 1.64 (quint, 4H, OCOCH₂-**CH₂**-, J=7.4Hz) ; 2.02 (massif, 8H, -CH₂-CH=CH-CH₂-) ; 2.36 (t, 4H, OCO**CH₂**-CH₂-, J=7.2 Hz) ; 2.45 (d, 1 H, OH (échangeable), J=4.2Hz) ; 4.18 (massif, 5H, -C**HaHb**-C**H**-C**HaHb**-) ; 5.35 (m, 4H, -CH₂-**CH=CH**-CH₂-).
SM : M+23 = 643 (M+Na⁺) ; (M+H non détectée)

### EXEMPLE 3e : Préparation du 1,3-ditétradécanoylglycérol

Ce composé est obtenu selon la procédure précédemment décrite (exemple 3a) à partir du glycérol et de l'acide tétradécanoïque.Le produit est obtenu sous forme d'une poudre blanche (rendement 30%).
Rf (CH₂Cl₂) : 0.30
IR: νCO ester 1733 et 1707 cm⁻¹
RMN (¹H, CDCl₃) : 089 (t, 6H, -CH₃, J = 6.5 Hz) ; 1.26 (massif, 40H, -CH₂-) ; 1.62 (quint, 4H, OCOCH₂-**CH₂**-, J=7.4Hz) ; 2.36 (t, 4H, OCO**CH₂**-CH₂-, J=7.5 Hz) ; 2.45 (d, 1 H, OH (échangeable), J=4.3Hz) ; 4.15 (massif, 5H, -CH**aHb**-C**H**-C**HaHb**-).

### EXEMPLE 3f : Préparation de 1-oléyl-3-palmitoylglycérol

Le palmitate de glycérol (exemple 2b) (5.516 g ; 0.017 mol) est dissous dans le dichlorométhane (500 ml) avant d'ajouter la dicyclohexylcarbodiimide (5.165 g ; 0.025 mol), la diméthylaminopyridine (3.058 g ; 0.025 mol) et l'acide oléique (4.714 g ; 0.017 mol). Le mélange réactionnel est maintenu sous agitation à température ambiante pendant 24 heures. Le précipité de dicyclohexylurée est filtré, rincé au dichlorométhane et le filtrat est évaporé sous vide. Le résidu obtenu est purifié par chromatographie sur gel de silice (éluant CH₂Cl₂) et permet d'obtenir le composé souhaité sous forme de solide blanc (rendement: 23%).
Rf (CH₂Cl₂) : 0.24
F : 30°C
IR: νCO ester 1731 et 1710 cm⁻¹
RMN (¹H, CDCl₃) : 087 (t, 6H, -CH₃, J = 6.5 Hz) ; 1.26 (massif, 44H, -CH₂-) ; 1.62 (quint, 4H, OCOCH₂-**CH₂**-, J=7.4Hz) ; 2.01 (massif, 4H, -**CH₂**-CH=CH-**CH₂**-) ; 2.36 (t, 4H, OCO**CH₂**-CH₂-, J=7.3 Hz) ; 2.465 (d, 1H, OH (échangeable), J=4.3Hz) ; 4.17 (massif, 5H, -C**HaHb**-C**H**-CH**aHb**-) ; 5.34 (m, **4H**, -CH₂-**CH=CH**-CH₂-).
SM : M+23 = 617 (M+Na⁺) ; (M+H non détectée)

### EXEMPLE 4 : Préparation de 1,2,3-triacylglycérols

### EXEMPLE 4a : Préparation du 1,2,3-tritétradécylthioacétylglycérol

Le glycérol (1 g, 10.86 mmol) est dissous dans le dichlorométhane (200 ml) avant d'ajouter la dicyclohexylcarbodiimide (7.84 g, 38.01 mmol), la diméthylaminopyridine (4.64 g, 38.01 mmol) et l'acide tétradécylthioacétique (9.40 g, 32.58 mmol). Le mélange est laissé sous agitation à température ambiante. Après 48 heures de réaction, le précipité de dicyclohexylurée est filtré, lavé plusieurs fois au dichlorométhane et le filtrat est évaporé. Le résidu obtenu est purifié par chromatographie sur gel de silice (CH₂Cl₂-cyclohexane 4-6). Le 1,2,3-tritétradécylthioacétylglycérol est obtenu sous forme de poudre blanche (rendement 65%).
Rf (CH₂Cl₂-Cyclohexane 7-3) : 0.47
F°: 57°C
IR: νCO ester 1738 et 1722 cm⁻¹
RMN (¹H, CDCl₃) : 0.89 (t, 9H, -CH₃, J = 6.5 Hz) ; 1.26 (massif, 66H, -CH₂-) ; 1.62 (m, 6H**,** -CH₂-**CH₂**-CH₂-S-) ; 2.63 (t, 6H, CH₂-**CH₂**-S-, J = 7.3 Hz) ; 3.23 (s, 6H, S-**CH₂**-COOH) ; 4.27 (dd, 2H, -CHaHb-CH-CHaHb-, J=12Hz et J=6Hz) ; 4.39 (dd, 2H, -CHa**Hb**-CH-CHa**Hb**-, J=12Hz et J=4.3Hz) ; 5.34 (m, 1H, -CHaHb-CH-CHaHb-)
SM : M+23 = 925 (M+Na⁺) ; M+39 = 941 (M+K⁺); 903 (M+H non détectée)

### EXEMPLE 4b : Préparation du 1,2,3-tri-(4-dodécylthio)butanoylglycérol

Ce composé est obtenu selon la procédure précédemment décrite (exemple 4a) à partir de l'acide 4-(dodécylthio)butanoïque (exemple 1 b) et du glycérol.
Rf (CH₂Cl₂-Cyclohexane 7-3) : 0.43
IR: νCO ester 1738 et 1727 cm⁻¹
RMN (¹H, CDCl₃) : 0.84-0.92 (t, 9H, -CH₃, J = 6.3 Hz) ; 1.22-1.44 (massif, 54H, - CH₂-) ; 1.50-1.64 (massif, 6H, -**CH₂**-CH₂-S-CH₂-CH₂-CH₂-COOH); 1.83-1.97 (massif, 6H, -CH₂-S-CH₂-**CH₂**-CH₂-COOH) ; 2.42-2.59 (massif, 18H, **-CH₂**-CH₂-CH₂-S-**CH₂**-CH₂-**CH₂**-COOH ; 4.11-4.20 (dd, 2H, -CHaHb-CH-CHaHb-, J=12Hz et J=5.9Hz) ; 4.29-4.36 (dd, 2H, -CHa**Hb**-CH-CHa**Hb**-, J=12Hz et J=4.5Hz) ; 5.22-5.32 (m, 1 H, -CHaHb-**CH**-CHaHb-)
SM : M+23 = 925 (M+Na⁺) ; M+39 = 941 (M+K⁺) ; 903 (M+H non détectée)

### EXEMPLE 4c : Préparation du 1,2,3-tri-(6-décylthio)hexanoylglycérol

Ce composé est obtenu selon la procédure précédemment décrite (exemple 4a) à partir de l'acide 6-(décylthio)hexanoïque (exemple 1 c) et du glycérol.
Rf (CH₂Cl₂-Cyclohexane 7-3) : 0.43
IR: νCO ester 1730 cm⁻¹
RMN (¹H, CDCl₃) : 0.85-0.92 (t, 9H, -CH₃, J = 6.5 Hz) ; 1.21-1.50 (massif, 48H, -
CH₂-) ; 1.51-1.72 (massif, 18H, -**CH₂**-CH₂-S-CH₂-**CH₂**-CH₂-**CH₂**-CH₂-COOH) ; 2.28-2.40 (massif, 6H, -CH₂-S-CH₂-CH₂-CH₂-CH₂-**CH₂**-COOH) ; 2.45-2.57 (massif, 12H, **-CH₂-**S-**CH₂**-) ; 4.10-4.20 (dd, 2H, -C**Ha**Hb-CH-C**Ha**Hb-, J=12Hz et J=6Hz); 4.25-4.38 (dd, 2H, -CHa**Hb-**CH-CHa**Hb-**, J=12Hz et J=4.3Hz) ; 5.22-5.32 (m, 1H, -CHaHb-**CH**-CHaHb-)
SM : M+23 = 925 (M+Na⁺); M+39 = 941 (M+K⁺) ; 903 (M+H non détectée)

### EXEMPLE 4d : Préparation du 1,2,3-tritétradécylsulfoxyacétylglycérol

Ce composé est obtenu selon la procédure précédemment décrite (exemple 4a) à partir de l'acide tétradécylsulfoxyacétique (exemple 1e) et du glycérol.
Rf (CH₂Cl₂-Cyclohexane 7-3) : 0.33
IR: νCO ester 1730 cm⁻¹
RMN (¹H, CDCl₃) : 0.84-0.92 (t. 9H; -CH3. J = 6.7 Hz) ; 1.22-1.39 (massif, 66H, CH₂-) ; 1.40-1.54 (massif, 6H, **-CH₂**-CH₂-SO-) ; 2.82-2.89 (massif, 6H, -CH₂**-CH₂-**SO-CH₂-COO-) ; 3.68 (s, 6H, -CH₂-SO**-CH₂**-COOH) ; 4.20-4.30 (massif, 5H, -CH₂-C**H**-C**H**₂-).
SM : M+1 = 951 ; M+23 = 974 (M+Na⁺) ; M+39 = 990 (M+K⁺)

### EXEMPLE 4e : Préparation du 1,2,3-tri-(tétradécylsulfonyl)acétylgivcérol

Ce composé est obtenu selon la procédure précédemment décrite (exemple 4a) à partir de l'acide tétradécylsulfonylacétique (exemple 1g) et du glycérol.
Rf (CH₂Cl₂-Cyclohexane 7-3) : 0.50
IR: νCO ester 1741 cm⁻¹
RMN (¹H, CDCl₃) : 0.84-0.92 (t, 9H, -CH₃, J = 6.7 Hz) ; 1.22-1.38 (massif, 60H, - CH₂-) ; 1.39-1.48 (massif, 6H, -**CH₂**-CH₂-CH₂-SO₂-) ; 1.81-1.94 (massif, 6H, - **CH₂**-CH₂-SO₂-) : 3.21-3.30 (t, 6H, -CH₂-CH₂-SO₂-CH₂-COOH, J=8Hz) ; 3.95 (s, 6H, -CH₂-SO₂-CH₂-COOH) ; 4.23-4.33 (massif, 5H, -CH₂-CH-CH₂-).

### EXEMPLE 4f: Préparation du 1,2,3 tri-tétradécyrlsélénoacétylglycérol

Ce composé est obtenu selon la procédure précédemment décrite (exemple 4a) à partir de l'acide tétradécylsélénoacétique (exemple 1d) et du glycérol.
Rf (CH₂Cl₂-Cyclohexane 7-3) : 0.74
IR: νCO ester 1737 et 1721 cm⁻¹
RMN (¹H, CDCl₃) : 0.85-0.92 (t, 9H, -CH₃, J = 6.2 Hz) ; 1.23-1.46 (massif, 66H, - CH₂-) ; 1.62-1.76 (massif, 6H, -CH₂**-CH₂**-CH₂-Se-) ; 2.72-2.79 (t, 6H, CH₂**-CH₂-**Se-, J=7.4Hz) ; 3.15 (s, 6H, Se**-CH₂-**COOH) ; 4.13-4.23 (massif, 5H, **-CH₂-CH-**CH₂-).

### EXEMPLE 4g : Préparation du 1,3-dipalmitoyl-2-tétradécylthioacétylglycérol

Le 1,3-dipalmitoylglycérol (5.64 g ; 9.9 mmol ; 1eq), l'acide tétradécylthioacétique (5.74 g ; 19.8 mmol ; 2eq), la dicyclohexylcarbodiimide (4.1 g ; 19.8 mmol ; 2eq) et la diméthylaminopyridine (2.42 g ; 19.8 mmol ; 2eq) sont mis en solution dans le dichlorométhane. Le mélange réactionnel est laissé sous agitation à température ambiante pendant 3 jours. La dicyclohexylurée formée est filtrée et lavée plusieurs fois au dichlorométhane. Le filtrat est porté à sec. Le produit résiduel est purifié par chromatographie sur gel de silice (éluant: dichlorométhane/cyclohexane 4/6) (rendement : 80%).
Rf (CH₂Cl₂-Cyclohexane 7-3) : 0.32
F°: 60-62°C
IR: νCO ester 1744 et 1730 cm⁻¹
RMN (¹H, CDCl₃) : 0.86-0.91 (t, 9H, -CH₃, J = 6.6 Hz) ; 1.10-1.45 (massif, 70H, - CH₂-) ; 1.57-1.64 (massif, 6H, -CH₂-CH₂-CH₂-S- et OCOCH₂-**CH₂**); 2.30-2.35 (t, 4H. OCOC**H₂**-CH₂-, J=7.4 Hz) ; 2.60-2.66 (t, 2H, CH₂-CH₂-S-, J = 7.4 Hz) ; 3.23 (s, 2H, S-**CH₂**-COOH) ; 4.14-4.21 (dd, 2H, -CHaHb-CH-CHaHb-, J=12Hz et J=5.8Hz); 4.30-4.36 (dd, 2H, -CHaHb-CH-CHaHb-, J=12Hz et J=4Hz); 5.26-5.33 (m, 1 H, -CHaHb-CH-CHaHb-)
SM : M+23 = 861 (M+Na⁺) ; M+39 = 877 (M+K⁺) ; (M+H non détectée)

### EXEMPLE 4h : Préparation du 1,3-dilinoléyl-2-tétradécylthioacétylglycérol

Ce composé est obtenu selon la procédure précédemment décrite (exemple 4g) à partir du 1,3-dilinoléylglycérol (exemple 3b) et de l'acide tétradécylthioacétique (exemple 1a). Le produit est obtenu sous forme d'huile visqueuse incolore (rendement : 56%).
Rf (CH₂Cl₂-Cyclohexane 7-3) : 0.32
IR: vCO ester 1745cm⁻¹
RMN (¹H, CDCl₃) : 0.82-0.93 (t, 9H, -CH₃, J = 6.6 Hz) ; 1.15-1.45 (massif, 50H, - CH₂-) ; 1.52-1.70 (massif, 6H, -CH₂-CH₂-CH₂-S- et OCOCH₂-**CH₂**); 1.93-2.14 (massif, 8H, **-CH₂**-CH=CH-**CH₂**-) ; 2.28-2.37 (t, 4H, OCO**CH₂**-CH₂-, J=7.5 Hz) ; 2.59-2.67 (t, 2H, CH₂-**CH₂**-S-, J = 7.4 Hz) ; 2.70-2.83 (t, 4H, -CH₂-CH=CH-**CH₂-**CH=CH-CH₂-) ; 3.22 (s, 2H, S-**CH₂**-COOH) ; 4.12-4.23 (dd, 2H, -CHaHb-CH-C**Ha**Hb-, J=12Hz et J=6.2Hz) ; 4.28-4.37 (dd, 2H, -CH**aHb**-CH-CHa**Hb**-, J=12Hz et J=4Hz) ; 5.24-5.45 (m, 1 H, -CHaHb-C**H**-CHaHb-)
SM : M+23 = 909 (M+Na⁺); M+39 = 925 (M+K⁺); (M+H non détectée)

### EXEMPLE 4i:Préparation du 1,3-distérayl-2-tétradécylthioacétylglycérol

Ce composé est obtenu selon la procédure précédemment décrite (exemple 4g) à partir du 1,3-distéraylglycérol (composé 3c) et de l'acide tétradécylthioacétique (composé 1a).
rendement 41 %
Rf (CH₂Cl₂) : 0.32
IR: vCO ester 1744 et 1731 cm⁻¹
RMN (¹H, CDCl₃) : 0.86-0.91 (t, 9H, -CH₃, J = 6.6 Hz) ; 1.10-1.45 (massif, 78H, -
CH₂-) ; 1.57-1.64 (massif, 6H, -CH₂-**CH₂**-CH₂-S- et OCOCH₂-**CH₂**) 2.29-2.35 (t, 4H, OCO**CH₂**-CH₂-, J=7.4 Hz) ; 2.60-2.66 (t, 2H, CH₂-CH₂-S-, J = 7.4 Hz) ; 3.23 (s, 2H, S-**CH₂**-COOH) ; 4.14-4.21 (dd, 2H, -C**Ha**Hb-CH-C**Ha**Hb-, J=12Hz et J=5.8Hz) ; 4.30-4.36 (dd, 2H, -CHa**Hb**-CH-CHa**Hb**-, J=12Hz et J=4Hz) ; 5.26-5.32 (m, 1H, -CHaHb-CH-CHaHb-)

### EXEMPLE 4j : Préparation du 1,3-oléyl-2-tétradécylthioacétylglycérol

Ce composé est obtenu selon la procédure précédemment décrite (exemple 4g) à partir du 1,3-dioléylglycérol (composé 3d) et de l'acide tétradécylthioacétique (composé 1a).

Le produit est obtenu sous forme d'huile visqueuse incolore (rendement : 32%) Rf (CH₂Cl₂-Cyclohexane 7-3) : 0.50
IR: νCO ester 1746cm⁻¹
RMN (¹H, CDCl₃) : 0.89 (t, 9H, -CH₃, J = 6.4 Hz) ; 1.31 (massif, 62H, -CH₂-) ; 1.60 (massif, 6H, -CH₂-**CH₂**CH₂-S- et OCOCH₂**-CH₂)** ; 2.02 (massif, 8H, **-CH₂-**CH=CH-**CH₂-**) ; 2.33 (t, 4H, OCO**CH₂**-CH₂-, J=7.3 Hz) ; 2.63 (t, 2H, CH₂-**CH₂**-S-, J=7.7 Hz) ; 3.23 (s, 2H, S-**CH₂**-COOH) ; 4.18 (dd, 2H, -C**Ha**Hb-CH-C**Ha**Hb-, J=12.4Hz et J=6.4Hz); 4.33 (dd, 2H, -CHa**Hb**-CH-CHa**Hb**-, J=12.4Hz et J=4.5Hz) ; 5.33 (massif, 1H, -CHaHb-CH-CHaHb- et -CH₂-**CH=CH-**CH₂-)
SM : M+23 = 913 (M+Na⁺) ; M+39 = 929 (M+K⁺) ; (M+H non détectée)

### EXEMPLE 4k : Préparation du 1,3-ditétradécanoyl-2-tétradécylthioacétylglycérol

Ce composé est obtenu selon la procédure précédemment décrite (exemple 4g) à partir du 1,3-ditétradécanoylglycérol (composé 3e) et de l'acide tétradécylthioacétique (composé 1a). (rendement : 28%).
Rf (CH₂Cl₂-Cyclohexane 7-3) : 0.30
F°: 60-62°C
IR: νCO ester 1744 et 1730 cm⁻¹
RMN (¹H, CDCl₃) : 0.87 (t, 9H, -CH₃, J = 7.2 Hz) ; 1.27 (massif, 62H, -CH₂-); 1.60 (massif, 6H, -CH₂**-CH₂**-CH₂-S- et OCOCH₂-**CH₂**) ; 2.33 (t, 4H, OCO**CH₂**-CH₂-, J=7.7 Hz) ; 2.63 (t, 2H, CH₂**-C**H₂-S-, J = 7.2 Hz) ; 3.23 (s, 2H, S-**CH₂**-COOH) ; 4.18 (dd, 2H, -C**Ha**Hb-CH-C**Ha**Hb-, J=12Hz et J=5.8Hz) ; 4.33 (dd, 2H, -CHaHb-CH-CHa**Hb**-, J=11.5Hz et J=5.8Hz) ; 5.30 (m, 1 H, -CHaHb-CH-CHaHb-).
SM : M+23 = 805 (M+Na⁺) ; (M+H non détectée)

### EXEMPLE 41 : Préparation du 1-palmitoyl-2,3-ditétradécyrlthioacétylglyrcérol

Le 1-palmitate de glycérol (4.804 g ; 0.014 mol) est dissous dans le dichlorométhane (300 ml) avant d'ajouter la dicyclohexylcarbodiimide (7.498 g ; 0.036 mol), la diméthylaminopyridine (4.439 g ; 0.036 mol) et l'acide tétradécylthioacétique (8.386 g ; 0.029 mol). Le mélange réactionnel est placé sous agitation à température ambiante pendant 48 heures. Le précipité de dicyclohexylurée est filtré, lavé au dichlorométhane. Le filtrat est porté à sec. Le résidu obtenu est purifié par chromatographie sur gel de silice (éluant dichlorométhane-cyclohexane 4-6) et donne le composé souhaité sous forme de poudre blanche (rendement 42%).
Rf (CH₂Cl₂-Cyclohexane 7-3) : 0.31
F : 57-59°C
IR: νCO ester 1736 et 1722 cm⁻¹
RMN (¹H, CDCl₃) : 0.89 (t, 9H, -CH₃, J = 6.6 Hz) ; 1.27 (massif, 68H, -CH₂-); 1.60 (massif, 6H, -CH₂-**CH₂-**CH₂-S- et OCOCH₂-**CH₂**); 2.33 (t, 2H, OCO**CH₂**-CH₂-, J=7 Hz) ; 2.63 (t, 4H, CH₂-**CH₂**-S-, J = 8.9 Hz) ; 3.23 (s, 4H, S-**CH₂**-COOH); 4.23 (m, 2H, -CHaHb-CH-CHaHb-) ; 4.37 (m, 2H, -CHaHb-CH-CHaHb) ; 5. 31 (m, 1 H, -CHaHb-CH-CHaHb-)
SM : M+23 = 893 (M+Na⁺) ; M+39 = 909 (M+K⁺) ; (M+H non détectée)

### EXEMPLE 4m : Préparation du 1-oléyl-3-palmitoyl-2-tétradécylthioacétylglycérol

Le 3-oléyl-1-palmitoylglycérol (2 g; 0.003 mol) est dissous dans le dichlorométhane (150 ml) avant d'ajouter la dicyclohexylcarbodiimide (1.040 g ; 0.005 mol), la diméthylaminopyridine (0.616 g ; 0.005 mol) et l'acide tétradécylthioacétique (1.455 g; 0.005 mol). Le mélange est laissé sous agitation à température ambiante pendant 24 heures, Le précipité de dicyclohexylurée est filtré, rincé au dichlorométhane et le filtrat est concentré. Le résidu obtenu est purifié par chromatographie sur gel de silice (éluant CH₂Cl₂-cyclohexane 4-6) et permet d'obtenir le composé souhaité sous forme d'huile (rendement 49%).
Rf (CH₂Cl₂-Cyclohexane 7-3) : 0.45
F < 4°C
IR: νCO ester 1742cm⁻¹
RMN (¹H, CDCl₃) : 0.89 (t, 9H, -CH₃, J = 6.5 Hz) ; 1.26 (massif, 66H, -CH₂-) ; 1.60 (massif, 6H, -CH₂-**CH₂**CH₂-S- et OCOCH₂-**CH₂**); 2.03 (massif, 4H, -**CH₂-**CH=CH-**CH₂**-); 2.33 (t, 4H, OCO**CH₂**-CH₂-, J=7.4 Hz) ; 2.63 (t, 2H, CH₂-**CH₂**-S-, J = 7.4 Hz) ; 3.23 (s, 2H, S-**CH₂**COOH) ; 4.18 (dd, 2H, -C**Ha**Hb-CH-C**Ha**Hb-, J=12.2Hz et J=6.1Hz) ; 4.33 (dd, 2H, -CHa**Hb**-CH-CHa**Hb**-, J=12.2Hz et J=4.4Hz); 5.32 (massif, 3H, -CHaHb-CH-CHaHb- et -CH₂-**CH**=**CH**-CH₂-)
SM : M+23 = 887 (M+Na⁺) ; M+39 = 903 (M+K⁺); (M+H non détectée)

### EXEMPLE 4n : Préparation du 1,3-dipalmitoyl-2-docosylthioacétylalycérol

Le produit est préparé selon la procédure décrite (exemple 4g) à partir du 1,3-dipalmitoylglycérol (exemple 3a) et de l'acide docosylthioacétique (exemple 1i). Rendement : 77%
Rf (CH₂Cl₂-Cyclohexane 7-3) : 0.32
IR: νCO ester 1745 et 1730 cm⁻¹
RMN (¹H, CDCl₃) : 0.86-0.91 (t, 9H, -CH₃, J = 6.6 Hz) ; 1.10-1.45 (massif, 86H, - CH₂-) ; 1.57-1.64 (massif, 6H, -CH₂-**CH₂**-CH₂-S- et OCOCH₂-**CH₂)**; 2.29-2.34 (t, 4H, OCO**CH**₂-CH₂-, J = 7.5 Hz) ; 2.60-2.66 (t, 2H, CH₂-**CH-₂**-S-, J = 7.4 Hz) ; 3.23 (s, 2H, S-CH₂-COOH) ; 4.13-4.22 (dd, 2H, **-CHa**Hb-CH-C**Ha**Hb-, J=12Hz et J=5.8Hz) ; 4.30-4.36 (dd, 2H, -CHaHb-CH-CHa**Hb-**, J=12Hz et J=4Hz) ; 5.27-5.34 (m, 1 H, -CHaHb**-CH**-CHaHb-)

### EXEMPLE 5 : Préparation des dérivés 2-aminoglycérol

### EXEMPLE 5a : Préparation du 2-tétradécylthioacétamidopropane-1,3-diol

L'acide tétradécylthioacétique (2.878 g ; 0.010 mol) et le 2-amino-1,3-propanediol (1 g ; 0.011 mol) sont placés dans un ballon et chauffés 190°C pendant 1 heure. Après refroidissement à température ambiante, le milieu est repris par du chloroforme et lavé à l'eau. La phase organique est séchée sur MgSO₄, filtrée puis évaporée pour fournir un résidu solide ocre. Ce résidu est placé sous agitation dans l'éther diéthylique pendant 12 heures. Le produit est isolé par filtration et fournit une poudre blanche (rendement : 6%).
Rf (CH₂Cl₂-méthanol 9-1): 0.60
F° : 95-97°C
IR: νCO amide 1640 cm⁻¹
RMN (¹H, CDCl₃) : 0.84-0.93 (t, 3H, -CH₃, J = 6.4 Hz) ; 1.21-1.45 (massif, 22H, - CH₂-) ; 1.54-1.72 (m, 2H, -CH₂-CH₂-CH₂-S-); 2.52-2.59 (t, 2H, CH₂-**CH₂**-S-, J = 7.1 Hz) ; 2.63 (sl, 2H, OH) ; 3.27 (s, 2H, S-**CH**₂-COOH) ; 3.77-3.96 (massif, 4H, - **CH₂**-CH-**CH₂**-) ; 3.97-4.04 (m, 1H, - CH₂-CH-CH₂-) ; 7.55 (d, 1H, -CONH-, J=6.7Hz).
SM : M+1=362 ; M+23 = 384 (M+Na⁺) ; M+39 = 400 (M+K⁺)

### EXEMPLE 5b: Préparation du 2-tétradécylthioacétamido-1,3-ditétradécytthioacétyloxypropane

Le 2-tétradécylthioacétamidopropan-1,3-diol (1 g ; 2.77 mmol) (exemple 5a) est dissous dans le dichlorométhane (180 ml) puis la dicyclohexycarbodiimide (1.427 g ; 6.91 mmol), la diméthylaminopyridine (0.845 g ; 6.91 mmol) et l'acide tétradécylthioacétique (1.995 g ; 6.91 mmol) (exemple 1a) sont ajoutés dans cet ordre. Le mélange réactionnel est laissé sous agitation à température ambiante pendant 48 heures. Le précipité de dicyclohexylurée est filtré et lavé par du dichlorométhane et le filtrat est concentré. Le résidu obtenu est purifié par chromatographie sur gel de silice (éluant dichlorométhane-cyclohexane 7-3). Le composé souhaité est obtenu sous forme de poudre blanche (rendement : 66%).
Rf (CH₂Cl₂ 10): 0.18
F° : 82-84°C
IR: νCO ester 1715 et 1730 cm⁻¹ ; νCO amide 1648 cm⁻¹
RMN (¹H, CDCl₃) : 0.84-0.95 (t, 3H, -CH₃, J = 6.6 Hz) ; 1.221-1.45 (massif, 66H, - CH₂-) ; 1.54-1.69 (massif, 6H, -CH₂**CH₂**-CH₂-S-) ; 2.48-2.55 (t, 2H, CH₂-CH₂-S-CH₂-CONH-, J = 7.5 Hz) ; 2.59-2.70 (t, 4H, CH₂-CH₂-S-CH₂-COO-, J = 7.2 Hz) ; 3.24 (s, 6H, S-CH₂-CO-) ; 4.18-4.35 (massif, 4H, -**CH₂**-CH-CH₂-); 4.47-4.60 (m, 1H, - CH₂-CH-CH₂-) ; 7.23 (d, 1 H, -CONH-, J=8.5Hz).
SM : M+23 = 924 (M+Na⁺) ; (M+1 non détectée)

### EXEMPLE 6: Méthode de solubilisation des triacylglycérols selon l'invention.

Les composés selon l'invention décrits dans les exemples 2 à 5 peuvent être solubilisés comme décrit pour l'exemple 4a.
Cette solubilisation est utile pour conduire les expériences *in vitro.*

Une émulsion comprenant de l'exemple 4a et de la phosphatidylcholine (PC) est préparée selon le protocole de Spooner et al (Spooner et al JBC, 1988, vol 263, pp 1444-1453). L'exemple 4a est mélangé à la PC selon un rapport 4:1 (w/w) dans du chloroforme, la mixture est séchée sous azote, puis évaporée toute la nuit sous vide, la poudre qui en résulte est reprise par 0,16 M de KCI contenant 0,01 M d'EDTA puis les particules lipidiques sont dispersées par ultra-sons pendant 30 minutes à 37°C. Les liposomes formés sont ensuite séparés par ultracentrifugation (ultracentrifugeuse XL 80, Beckman Coulter, Villepinte, France) à 25000 tr/m pendant 45 minutes pour récupérer les liposomes dont la taille est supérieure à 100 nm et se rapproche de celle des chylomicrons. Des liposomes constitués uniquement de PC sont préparés en parallèle pour servir de témoin négatif.
La composition des liposomes en exemple 4a est estimée en utilisant le kit de dosage enzymocolorimétrique des triglycérides. Le dosage est effectué contre une gamme standard, préparée grâce au calibrateur des lipides CFAS Réf. N° 759350 (Boehringer Mannheim GmbH, Allemagne). La gamme standard a été construite de 16 à 500 µg/ml. 100 µl de chaque dilution d'échantillon ou de gamme étalon sont déposés par puits d'une plaque de titration (96 puits). Ensuite 200 µl de réactifs triglycérides Réf. 701912 (Boehringer Mannheim. GmbH, Allemagne) sont rajoutés dans chaque puits, et l'ensemble de la plaque est incubée pendant 30 min. à 37°C. La lecture des Densités Optiques (DO) est effectuée à 492 nm sur le spectrophotomètre. Les concentrations en triglycérides de chaque échantillon sont calculées après construction de la courbe étalon selon une fonction linéaire y=ax+b, où y représente les DO et x les concentrations en triglycérides.

Les liposomes de l'exemple 4a, ainsi préparés, peuvent être utilisés dans les expériences *in vitro.*

### EXEMPLE 7 : Evaluation de l'activation des PPARs in vitro

Les composés selon l'invention testés sont les composés dont la préparation est décrite dans les exemples 2 à 5 ci-dessus.

Les récepteurs nucléaires membres de la sous-famille des PPARs qui sont activés par deux classes majeures de drogues, les fibrates et les glitazones, abondamment utilisées en clinique humaine pour le traitement des dislipidémies et du diabète, jouent un rôle important dans l'homéostasie lipidique et glucidique. Particulièrement, le récepteur PPARα module, entre autres, l'expression des gènes codant pour les apolipoprotéines impliquées dans le transport de lipides et l'expression des gènes ACO d'une part et CPT-I et CPT-II d'autre part, impliqués respectivement dans la β-oxydation peroxysomale et mitochondriale. Les exemples suivant montrent que les composés selon l'inyention activent PPARα et PPARγ in vitro.

L'activation des PPARs est évaluée *in vitro* dans des hépatocytes de rat en culture primaire par la mesure de l'expression de gènes cibles des PPARs et par la mesure de l'activité transcriptionnelle de chimères constituées du domaine de liaison à l'ADN du facteur de transcription Gal4 de levure et du domaine de liaison du ligand des différents PPARs. Ces derniers résultats sont ensuite confirmés dans des lignées cellulaires suivant les protocoles suivants.

### 1) Hépatocytes en culture primaire

### a. Protocole de culture

Les hépatocytes de rats sont isolés par perfusion de foies de rat Wistar OFA mâles (Charles River, L'Arbresle, France) dont le poids corporel est compris entre 175 et 225 g à l'aide d'un mélange de collagénase et de thermolysin (Blendzyme 3, Roche, Bâle, Suisse). Le foie de rats anesthésiés au pentobarbital est perfusé via la veine porte, d'abord par 100 ml d'un tampon de lavage (Liver perfusion medium, Gibco, Paisley, UK) et ensuite par 200 ml du milieu de digestion suivant : HBSS dépourvu de CaCl₂ et MgSO₄ (Sigma, S^{t} Louis, MI, USA) supplémenté de 10 mM Hepes, pH 7,6 , de 4 mM CaCl₂ et de 7 mg de Blendzyme 3 suivant une modification du protocole décrit précédemment (Raspé et al. J. Lipid Res. 40, 2099-2110, 1999). Lorsque la viabilité des cellules déterminée par test au Bleu Trypan (Sigma, St Louis, MI, USA) excède 80%, les hépatocytes sont étalés dans des boîtes de culture à 24 puits à raison de 7,5x10⁴ cellules/cm² pour les expériences de transfection ou dans des boîtes de culture à 6 puits à raison de 10⁵ cellules/cm² pour les expériences de quantification des ARN messagers. Les cellules sont ensemencées et incubées pendant 4 heures dans un milieu de culture Williams E supplémenté de 100 U/ml penicillin (Gibco, Paisley, UK), 2 mM L-Glutamine (Gibco, Paisley, UK), 2 % vol/vol UltroSER SF (Biosepra, Cergy St-Christophe, France), 0,2% masse/vol albumine sérique bovine (Sigma, St Louis, MI, USA), 1 µM Dexamethasone (Sigma, St-Louis. MI, USA) et 100 nM T3 (Sigma, St Louis, MI, USA). L'expérience est ensuite poursuivie dans le même milieu de culture dépourvu d'Ultroser. Les composés testés sont ajoutés à la concentration indiquée directement dans le milieu de culture.

### b. Protocole de transfection

Les hépatocytes de rats qui ont été isolés et mis en culture comme décrit ci-dessus sont transfectés dans le milieu de culture dépourvu d'Ultroser pendant une nuit avec le plasmide rapporteur pG5TkpGL3 (10 ng/puit), les vecteurs d'expression pGal4-φ, pGal4-mPPARα, pGal4-hPPARα, pGal4-hPPARγ, pGal4-hPPARδ (10 ng/puit) et le vecteur de contrôle de l'efficacité de transfection pRL-Null (1 ng/puit) (Promega Madison, WI, USA) à l'aide de lipofectine (Gibco, Paisley, UK) ou d'Effecten (QIAGEN, Courtaboeuf, France) suivant le protocole décrit par le fournisseur. Après transfection, les cellules sont traitées et incubées pendant 36 heures comme décrit précédemment (Raspé et al. J. Lipid Res. 40, 2099-2110, 1999). A l'issue de l'expérience, les cellules sont lysées et les activités luciférase sont déterminées à l'aide du kit de dosage Dual-Luciferase™ Reporter Assay System (Promega, Madison, WI, USA) selon la notice du fournisseur comme décrit précédemment (Raspé et al. J. Lipid Res. 40, 2099-2110, 1999). Le contenu en protéine des extraits cellulaires est ensuite évalué à l'aide du kit de dosage Bio-Rad Protein Assay (Bio-Rad, München, Allemagne) selon la notice du fournisseur.

### c. Description des plasmides utilisés

Le plasmides pG5TkpGL3, pGal4-hPPARα, pGal4-hPPARγ et pGal4-φ ont été décrits précédemment (Raspé et al. J. Lipid Res. 40, 2099-2110, 1999). Les constructions pGal4-mPPARα et pGal4-hPPARδ ont été obtenues par clonage dans le vecteur pGal4-φ de fragments d'ADN amplifiés par PCR correspondants au domaines DEF des récepteurs nucléaires PPARα de souris et PPARδ humain.

### d. Mesure des ARN messagers

Les ARN messagers ont été extraits d'hépatocytes de rat en culture primaire à l'aide du réactif Tri-Reagent (Sigma, St Louis, MI, USA) suivant les instructions du fournisseur, dosés par spectrophotométrie et quantifiés par RT-PCR semi-quantitative ou quantitative à l'aide du kit Light Cycler Fast Start DNA Master Sybr Green 1 kit (Hoffman-La Roche, Basel, Suisse) sur un appareil Light Cycler System (Hoffman-La Roche, Basel, Suisse). Des paires d'amorces spécifiques des gènes ACO et Apo All, cibles de PPARα ont été utilisées comme sondes. Des paires d'amorces spécifiques des gènes 36B4, β-actine et GAPDH ont été utilisées comme sondes témoins (Cf. tableau I ci-dessous).

**Tableau I:**

| **nom** | **séquence** | **PCR semi quantitative** | | **PCR quantitative** | | **gène** |
|---|---|---|---|---|---|---|
| | | **Tm** | **nbr cycle** | **Tm** | **nbr cycle sortie** | |
| ApoAI_r_1_s 741 | GCCTGAATCTCCTGG ACAACTG | 58°C | 25 | 58°C | 18 à 20 | Apo AI |
| ApoAI_r_1_as 742 | ATGCCTTTGCATCTC CTTCG | | | | | |
| ApoB_r_ 1_s 743 | ATACAGCCTGAGTGA GCCTCTTCAG | 55°C | 30 | X | X | Apo B |
| ApoB_r_1 _as 744 | CCAGGGAGTTGGAGA CCGTG | | | | | |
| GAPDH_h_1_s 390 | GACATCAAGAAGGTG GTGAA | 55°C | 25 | 55°C | 20 (variable) | GAPDH |
| GAPDH_h_1_as 389 | CCACATACCAGGAAA TGAGC | | | | | |
| beta-actine_h_1_s 189 | TTCAACTCCATCATG AAGTGTGAC | 55°C | 25 | 55°C | variable | β actine |
| beta-actine_h_1_as 188 | TCGTCATACTCCTTG CTTGCTGATCC | | | | | |
| CPTI_r_1_s 517 | GCTGGCTTATCGTGG TGGTG | 60°C | 25 | 60°C | 20 à 25 | CPT-I |
| CPTI_r_1_as 516 | GACCTGAGAGGACCT TGACC | | | | | |
| 36B4_h_1_s 177 | CATGCTCAACATCTC CCCCTTCTCC | X | X | 55°C | 23 | 36B4 |
| 36B4_h_1_as 178 | GGGAAGGTGTAATCC GTCTCCACAG | | | | | |
| ACOX1_r_1_as 457 | CGCATCCATTTCTCC TGCTG | 60°C | 25 | 60°C | 18 à 24 | ACO |
| ACOXl_r_1_s 458 | TTCTGTCGCCACCTC CTCTG | | | | | |
| ApoCIII_r_1_s 797 | ATGCAGCCCCGAATG CTCCTCATCGTGG | 55°C | 30 | 55°C | 28 à 30 | Apo CIII |
| ApoCIII_r_1_as 798 | TCACGGCTCAAGAGT TGGTGTTAC | | | | | |
| CPT2_r_1_s 725 | CAGAAGCCTCTCTTG GATGACAG | 55°C | 25 | X | X | CPT-II |
| CPT2_r_1_as 726 | TTGGTTGCCCTGGTA AGCTG | | | | | |
| ABCA1_h_2_s | CTGAGGTTGCTGCTG TGGAAG | 65°C | 21 | X | X | ABCA1 |
| ABCA1_1_h_2_as | CATCTGAGAACAGGC GAGCC | | | | | |

### 2) Lignées cellulaires

### a. Protocoles de culture

Les cellules HepG2 et RK13 proviennent de l' ECACC (Porton Down, UK) et sont cultivées dans du milieu DMEM supplémenté de 10% vol/vol sérum de veau foetal, 100 U/ml penicillin (Gibco, Paisley, UK) et 2 mM L-Glutamine (Gibco, Paisley, UK). Le milieu de culture est changé tous les deux jours. Les cellules sont conservées à 37°C dans une atmosphère humide contenant 5% CO₂ et 95% d'air.

### b. Transfection

Les cellules HepG2 et RK13 ensemencées dans des boîtes de culture 24 puits à raison de 10⁵ cellules/puits pour les HepG2 et 5x10⁴ cellules/puit pour les cellules RK13 sont transfectées pendant 2 heures avec le plasmide rapporteur pG5TkpGL3 (10 ng/puit), les vecteurs d'expression pGal4-φ, pGal4-mPPARα, pGal4-hPPARα, pGal4-hPPARγ, pGal4-hPPARδ (10 ng/puit) et le vecteur de contrôle de l'efficacité de transfection pRL-null (Promega Madison, WI, USA) (20 ng/puit) suivant le protocole décrit précédemment (Raspé et al. J. Lipid Res. 40, 2099-2110, 1999) et incubées pendant 36 heures avec les composés testés. A l'issue de l'expérience, les cellules sont lysées (Gibco, Paisley, UK) et les activités luciférase sont déterminées à l'aide du kit de dosage Dual-Luciferase™ Reporter Assay System (Promega, Madison, WI, USA) selon la notice du fournisseur comme décrit précédemment (Raspé et al. J. Lipid Res. 40, 2099-2110, 1999). Le contenu en protéine des extraits cellulaires est ensuite évalué à l'aide du kit de dosage Bio-Rad Protein Assay (Bio-Rad, München, Allemagne) selon la notice du fournisseur.

Les résultats obtenus sont présentés sur la Figure 11, et montrent que les composés testés sont capables d'activer très fortement le récepteur nucléaire PPARα.

### EXEMPLE 8: Evaluation des effets sur le métabolisme lipidique in vivo

Les composés selon l'invention testés sont les composés dont la préparation est décrite dans les exemples 2 à 5 ci-dessus.

Les fibrates, abondamment utilisés en clinique humaine pour le traitement des dislipidémies impliquées dans le développement de l'athérosclérose, une des principales cause de mortalité et de morbidité dans les sociétés occidentales, sont de puissants activateurs du récepteur nucléaire PPARα. Celui-ci régule l'expression de gènes impliqués dans le transport (apolipoprotéine telles que Apo AI, ApoAll et ApoC-III, transporteurs membranaires tels que FAT) ou le catabolisme des lipides (ACO, CPT-I ou CPT-II). Un traitement par les activateurs de PPARα se traduit donc chez le rongeur par une diminution des taux circulants de cholestérol et de triglycérides.

Les protocoles suivant permettent de mettre en évidence une baisse du taux de triglycérides et du taux de cholestérol circulant, ainsi que l'intérêt des composés selon l'invention dans le cadre de la prévention et/ou du traitement des maladies cardio-vasculaires.

### 1) Traitement des animaux

Des rats Sprague-Dawley ou Wistar de 200 à 230 g (Charles River, L'Arbresle, France) sont maintenus sous un cycle lumière/obscurité de 12 heures à une température constante de 20 ± 3°C. Après une acclimatation d'une semaine, les rats sont pesés et rassemblés par groupes de 8 animaux sélectionnés de telle sorte que la distribution de taux plasmatiques de cholestérol et de triglycérides soient uniformes. Les composés testés sont suspendus dans la carboxymethylcellulose et administrés par gavage intra-gastrique, à raison d'une, fois par jour pendant 15 jours aux doses indiquées. Les animaux ont un accès libre à l'eau et à la nourriture. A l'issue de l'expérience les animaux sont pesés et sacrifiés sous anesthésie après un jeûne de 5 heures. Le sang est collecté sur EDTA. Le plasma est préparé par centrifugation à 3000 tours/minutes pendant 20 minutes. Des échantillons de foie sont prélevés et conservés congelés dans l'azote liquide pour analyse ultérieure.

### 2) Mesure des lipides et apolipoprotéines sériques

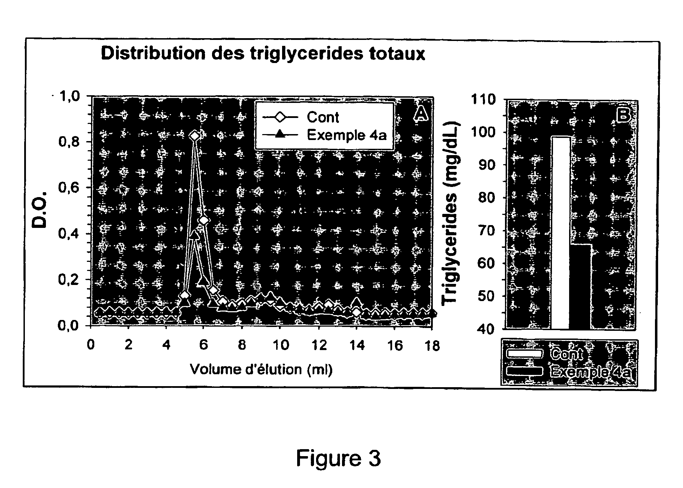

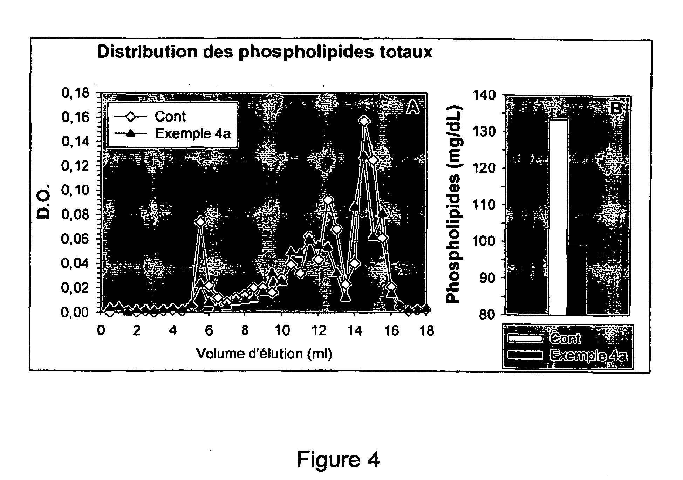

Les concentrations plasmatiques des lipides (cholestérol total et cholestérol libre, triglycérides et phospholipides) sont mesurées par dosage colorimétrique (Bio-Mérieux, Marcy l'Etoile, France) selon les indications du fournisseur. Les concentrations plasmatiques des apolipoprotéines AII, AI et CIII sont mesurées selon les méthodes décrites précédemment (Raspé et al. J. Lipid Res. 40, 2099-2110, 1999, Asset G et al., Lipids, 34, 39-44, 1999).
Pour séparer les lipoproteines selon leur taille, 300 µL de plasma sont injectés sur une colonne Sepharose 6HR 10/30 (Pharmacia, Uppsala, Suède) et élués à flux constant (0,2 ml/minute) avec du PBS (pH 7,2). La densité optique de l'effluent est enregistrée à 280 nm. L'effluent est collecté par fraction de 0,3 ml. Les concentrations de lipides dans les différentes fractions sont mesurées par dosage colorimétrique (Bio-Mérieux, Marcy l'Etoile, France) selon les indications du fournisseur.
Les résultats obtenus sont présentés dans les figures 2, 3, 4, 9A et 9B.

### 3) Analyse des ARNs

L'ARN total a été isolé des fragments de foie par extraction à l'aide du mélange thiocyanate de guanidine/phénol acide/chloroforme suivant le protocole décrit précédemment (Raspé et al. J. Lipid Res. 40, 2099-2110, 1999). Les ARN messagers ont été quantifiés par RT-PCR semi-quantitative ou quantitative à l'aide du kit Light Cycler Fast Start DNA Master Sybr Green I kit (Hoffman-La Roche, Basel, Suisse) sur un appareil Light Cycler System (Hoffman-La Roche, Basel, Suisse). Des paires d'amorces spécifiques des gènes ACO, Apo CIII,Apo AI, CPT-I et CPT-II ont été utilisées comme sondes. Des paires d'amorces spécifiques des gènes 36B4, β-actine et GAPDH ont été utilisées comme sondes témoins (Cf. tableau I).
Les résultats obtenus sont présentés dans les figures 5 et 9C.

### EXEMPLE 9 : Evaluation des propriétés antioxydantes des composés selon l'invention

Les composés selon l'invention testés sont les composés dont la préparation est décrite dans les exemples 2 à 5 ci-dessus.

L'oxydation des LDL est à la base du processus inflammatoire qui conduit à l'athérosclérose et aux maladies cardiovasculaires. Les composés qui retardent ou inhibent cette oxydation présentent donc des effets protecteurs bénéfiques.

### 1. Protection de l'oxydation des LDL par le cuivre ou le dihydrochloride d'azobis (2-amidinopropane) (AAPH) :

L'oxydation des LDL est une modification importante et joue un rôle prépondérant dans la mise en place et le développement de l'athérosclérose (Jurgens, Hoff et al. 1987). Le protocole suivant permet la mise en évidence des propriétés antioxydantes des composés. Sauf mention différente, les réactifs proviennent de chez Sigma (St Quentin, France).
Les LDL sont préparés suivant la méthode décrite par Lebeau et al. (Lebeau, Furman et al. 2000).
Les solutions de composés à tester sont préparées pour avoir des concentrations finales allant de 1 à 100 µM pour une concentration totale d'éthanol de 1% (v/v).

Avant l'oxydation, l'EDTA est retiré de la préparation de LDL par dialyse contre du PBS. La cinétique d'oxydation a ensuite lieu à 30°C en ajoutant 20 µl d'une solution à 16,6 µM de CuSO₄ ou de 2 mM de AAPH à 160 µL de LDL (125 µg de protéines/ml) et 20 µl d'une solution du composé à tester. La formation de diènes, l'espèce à observer, se mesure par densité optique à 234 nm dans les échantillons traités avec les composés mais avec ou sans cuivre (ou AAPH). La mesure de la densité optique à 234 nm est réalisée toutes les 10 minutes pendant 8 heures à l'aide d'un spectrophotomètre thermostaté (Kontron Uvikon 930). Les analyses sont réalisées en triplicata. L'activité des composés est exprimée en pourcentage de décalage de « lag-phase » (phase de latence avant le démarrage de l'oxydation) comparée à celle du contrôle. Nous considérons que les composés ont une activité antioxydante de 100% lorsqu'ils doublent le décalage de la lag-phase de l'échantillon témoin. Les inventeurs mettent en évidence que les composés selon l'invention, décrits dans les exemples 2 à 5, retardent l'oxydation des LDL (induite par le cuivre), ceci indiquant que les composés selon l'invention possèdent un caractère antioxydant intrinsèque.

### 2. Evaluation de la protection conférée par les composés selon l'invention vis-à-vis de la peroxydation lipidique :

La mesure de l'oxydation des LDL est réalisée par la méthode des TBARS. Selon le même principe que celui décrit précédemment, les LDL sont oxydés avec du CuSO₄ et la peroxydation lipidique est déterminée de la manière suivante :
Les TBARS sont mesurés à l'aide d'une méthode spectrophotométrique, l'hydroperoxydation lipidique est mesurée en utilisant l'oxydation péroxyde-lipide dépendante de l'iodure en iode. Les résultats sont exprimés en nmol de malondialdehyde (MDA) ou en nmol d'hydroperoxyde/mg de protéines.
Les résultats obtenus précédemment, en mesurant l'inhibition de la formation de diènés conjugés, sont confirmés par les expériences de mesure de peroxydation lipidique des LDL. Les composés selon l'invention protègent également de manière efficace les LDL contre la peroxydation lipidique induite par le cuivre (agent oxydant).

L'exemple 9 indique que les composés selon l'invention inhibent la modification oxydative des LDL.

### EXEMPLE 10 : Evaluation des effets sur l'expression d'enzymes impliquées dans la β-oxydation mitochondriale et peroxysomale

Les composés selon l'invention testés sont les composés dont la préparation est décrite dans les exemples 2 à 5 ci-dessus.

Les acides gras constituent une réserve essentielle d'énergie. La β-oxydation mitochondriale et peroxysomale des acides gras sont les principales voies de catabolisme des acides gras responsables de la mobilisation de cette énergie. Ces deux processus jouent donc un rôle primordial dans le contrôle des taux sériques d'acide gras libres ainsi que dans la régulation de la synthèse des triglycérides. L'enzyme qui détermine la vitesse de la β-oxydation peroxysomale est l'ACO. La β-oxydation mitochondriale est limitée par le transport des acides gras au sein de la mitochondrie. Celui-ci dépend de l'activité des enzymes CPT-I et CPT-II. La régulation de l'expression des enzymes ACO, CPT-I et CPT-II joue un rôle primordial dans le contrôle de β-oxydation peroxysomale et mitochondriale, respectivement.

Les composés selon l'invention induisent l'expression de l' ACO, de CPT-I et de CPT-II. Cette activité est mise en évidence de la manière suivante :

L'ARN isolé d'hépatocytes en culture primaire décrits dans l'exemple 7 ou de fragment de foie prélevé sur des rats traités avec les composés testés comme décrit dans l'exemple 8 sont quantifiés par RT-PCR semi-quantitative ou quantitative comme décrit dans les exemples 7 et 8 à l'aide de paires d'amorces spécifiques des gènes ACO, CPT-I et CPT-II.

### EXEMPLE 11: Evaluation des capacités d'oxydation des acides gras

Les composés selon l'invention testés sont les composés dont la préparation est décrite dans les exemples 2 à 5 ci-dessus.

Les capacités d'oxydation des acides gras déterminent les taux sériques d'acides gras libres ainsi que la possibilité de synthèse des triglycérides. Une accumulation d'acide gras libres dans le sang ou de triglycérides en dehors du tissu adipeux favorisent la résistance à l'insuline. D'autre part, une élévation des taux plasmatiques de triglycérides est actuellement considérée comme un facteur de risque de maladies cardiovasculaires. Une augmentation des capacités d'oxydation des acides gras présente donc un intérêt thérapeutique.

Les composés selon l'invention activent l'oxydation des acides gras par les mitochondries et les peroxysomes. Cette capacité est mise en évidence de la manière suivante :
On teste les activités CPT-I et CPT-II mitochondriales selon le protocole décrit par Madsen et al, 1999, Biochem Pharmacol. 57, 1011-1019.
On teste l'activité ACO selon le protocole décrit par Asiedu et al, 1995, Eur. J. Biochem, 227, 715-722.
On teste la β-oxydation mitochondriale et peroxysomale des acides gras selon le protocole décrit par Hovik et al, 1990, Biochem J. 270, 167-173.

### EXEMPLE 12: Evaluation des effets sur le transport inverse du cholestérol

Les composés selon l'invention testés sont les composés dont la préparation est décrite dans les exemples 2 à 5 ci-dessus.

La corrélation négative entre le taux d'HDL-cholestérol et les maladies cardiovasculaires est aujourd'hui bien connue. La capacité d'un composé à augmenter le transport inverse du cholestérol (RCT) est considérée comme un mécanisme par lequel les HDL protégent de l'athérosclérose.
Le RCT est un processus qui permet au cholestérol, en excès dans les tissus extra-hépatiques, d'être récupéré et exporté vers le foie où il est converti en acides biliaires avant d'être excrété dans la bile.
La présence de cellules spumeuses dérivées de macrophages est une des caractéristiques des premières étapes de la formation de la lésion d'athérosclérose.
L'efflux du cholestérol des macrophages est donc une phase critique pour la prévention de la formation des cellules spumeuses et, par conséquent, présente un effet protecteur vis-à-vis de l'athérosclérose. L'étape cruciale du RCT est le transfert du cholestérol en excès et des phospholipides des membranes cellulaires aux HDL naissantes. A ce titre, le transporteur ABCA1 (ATP binding cassette A1) joue un rôle clef dans ce processus et son expression est corrélée avec la réduction du développement de la plaque d'athérosclérose en stimulant l'efflux de cholestérol dans les macrophages.
D'autre part, il a récemment été démontré que ABCA1 est un gène cible du récepteur nucléaire LXRα, lui même gène cible des récepteurs PPARα et de PPARγ.

Les composés selon l'invention induisent l'expression de LXRα et d'ABCA1 et stimulent l'efflux du cholestérol dans 2 modèles *in vitro* de macrophages THP1 et primaires.

### 1/ mesure de l'expression d'ABCA1 et de LXRα:

### a/ Différentiation et traitement des macrophages humains THP-1 et primaires

Des monocytes THP-1 (ATCC, Rockville, Maryland) sont étalés dans des boîtes de culture à 6 puits en présence de PMA (Phorbol myristate acetate) et de sérum de veau foetal et incubées à 37°C pendant 48h, ce qui permet leur différenciation en macrophages.

En ce qui concerne les macrophages primaires, les cellules mononuclées sont isolées à partir de sang humain comme précédemment décrit (Chinetti et al. Nat. Medecine 7(1), 53-58, 2001) et sont étalées sur des plaques 6 puits et cultivées pendant 10 jours en présence de sérum humain pour permettre l'adhérence et la différenciation des monocytes primaires en macrophages.
Le traitement à l'aide des différents composés est réalisé pendant 48h dans un milieu sans sérum humain ou de veau foetal mais supplémenté avec du sérum Nutridoma HU (Boehringer) à 1%.

### b/ Mesures des ARNs messagers

Les ARNs totaux ont été extraits des macrophages traités à l'aide du kit RNeasy mini (QIAGEN, Hilden, Germany) suivant les instructions du fournisseur, dosés par spectrométrie et quantifiés par RT-PCR quantitative à l'aide du kit Light Cycler Fast DNA Master Green 1 (Hoffman-La Riche, Basel, Suisse) sur un appareil Light Cycler System (Hoffman-La Riche, Basel, Suisse). Des paires d'amorces spécifiques des gènes ABCA1 & LXRα ont été utilisées comme sondes.
Les résultats obtenus sont présentés dans la figure 12.

### 2/ mesure de l'efflux de cholestérol:

### a/ Différentiation et traitement des macrophages humains THP-1 et primaires

Les macrophages sont différenciés à partir de monocytes THP-1 ou primaires comme dans l'expérience précédente (1/ mesure de l'expression d'ABCA1 et de LXRα)

### b/ Chargement des macrophages en cholestérol et mesure de l'efflux

Les macrophages sont pré-traités pendant 24 h avec les composés, mais aussi chaque 24h pendant toute la durée de l'expérience. Ils sont chargés en cholestérol lors d'une incubation de 48h en présence de LDL acétylées (50 µg/ml contenant du cholestérol marqué au Tritium) dans un milieu RPMI 1640 supplémenté avec 1% de Nutidoma HU (Boehringer).
Après cette étape, les cellules sont lavées 2 fois avec du PBS et incubées pendant 24h dans du milieu RPMI sans Nutridoma avec ou sans apolipoprotéine A-I. A la fin de cette étape, le milieu est récupéré et les lipides intracellulaires sont extraits dans un mélange hexane / isopropanol, et séchés sous azote.
L'efflux est quantifié à l'aide d'un lecteur à scintillation Tri-Carb^{®} 2100 TR (Packard, Meriden, CT, USA) en divisant le nombre de coups comptés dans le milieu par le nombre de coups total comptés dans le milieu et dans les cellules.

### EXEMPLE 13 : Evaluation des effets sur le syndrome métabolique (syndrome X) et le diabète

Les composés selon l'invention testés sont les composés dont la préparation est décrite dans les exemples 2 à 5 ci-dessus.

La résistance à l'insuline est à la base du syndrome métabolique caractérisé par une intolérance au glucose, une hyperinsulinémie, une dislipidémie et l'hypertension. La combinaison de plusieurs facteurs de risque cardiovasculaire qui se traduit par un risque accru de maladies cardiovasculaires consécutives à l'athérosclérose est responsable de la majorité de la mortalité et de la morbidité liée au diabète de type II. Les traitements médicamenteux du syndrome métabolique ont donc pour principale cible la résistance à l'insuline.

Les composés selon l'invention réduisent les manifestations du syndrome métabolique (Syndrome X) telles que l'élévation des acides gras libres, l'hyperinsulinémie, l'hyperglycémie et la réponse insulinémique au glucose (test de tolérance au glucose) et le diabète dans deux modèles animaux qui présentent une résistance à l'insuline à l'origine du syndrome métabolique, les souris C57BL/6 maintenues sous régime riche en graisse et le rat Zucker obèse (fa/fa). Ces propriétés sont mises en évidence de la manière suivante :

### 1) Traitement des animaux

Des souris C57BL/6 (Charles River, L'Arbresle, France) mâles âgées de 6 semaines au début de l'expérience ont été aléatoirement rassemblées par groupes de 6 animaux sélectionnés de telle sorte que la distribution de leur poids corporel soit uniforme. Les souris ont reçu un régime pauvre en graisse (UAR AO4), un régime enrichi en graisse (huile de coco 29% poids/poids) ou le même régime enrichi complété avec les composés étudiés. Des rats Zucker mâles obèses (fa/fa) ou non obèses (fa /+) âgés de 5 semaines ou de 21 semaines (Charles River, L'Arbresle, France) rassemblés par groupes de 8 animaux sélectionnés de telle sorte que la distribution de taux plasmatiques de cholestérol et de triglycérides soient uniformes sont maintenus sous régime standard. Les animaux sont maintenus sous un cycle lumière/obscurité de 12 heures à une température constante de 20 ± 3°C. Les animaux ont un accès libre à l'eau et à la nourriture. La prise de nourriture et la prise de poids sont enregistrées. Les composés testés sont suspendus dans la carboxymethylcellulose et administrés par gavage intra-gastrique, à raison d'une fois par jour pendant 15 jours aux doses indiquées. A l'issue du traitement, certains animaux subissent un test-de tolérance au glucose comme décrit ci-dessous. A l'issue de l'expérience les autres animaux sont pesés et sacrifiés sous anesthésie après un jeûne de 5 heures. Le sang est collecté sur EDTA. Le plasma est préparé par centrifugation à 3000 tours/minutes pendant 20 minutes. Des échantillons de foie sont prélevés et conservés congelés dans l'azote liquide pour analyses ultérieures.

### 2) Dosage des acides gras libres et des lipides

Les taux d'acides gras libres sont variables chez les rats diabétiques. La concentration en acides gras libres dans le sérum ou le plasma, est mesurée par réaction enzymatique colorimétrique « NEFA/FFA » WAKO (Labo immuno systems, Neuss, Allemagne) sur du sérum ou du plasma.

Les concentrations plasmatiques des lipides (cholestérol total et triglycérides) sont mesurées par dosage colorimétrique (Bio-Mérieux, Marcy l'Etoile, France) selon les indications du fournisseur.
Les résultats obtenus sont présentés dans les figures 6 et 10.

### 3) Dosage de la glycémie

La mesure de la glycémie s'effectue par une réaction enzymatique colorimétrique (Sigma Aldrich, St Louis, MO).
Les résultats obtenus sont présentés dans la figure 7.

### 4) Dosage de l'insuline

Afin de mettre en évidence une hyperinsulinémie caractéristique des maladies métaboliques, les taux d'insuline sont dosés en utilisant le kit de dosage radioactif (Mercodia, Uppsala, Suède). L'insulinémie est déterminée sur des sérums ou des plasmas récoltés sur EDTA.
Les résultats obtenus sont présentés dans la figure 7.

### 5) Test de tolérance au glucose

Les animaux ont été anesthésiés après un jeûne de 8 h par injection intrapéritonéale de pentobarbital sodique (50 mg/kg). Pour initier le test de tolérance au glucose, une injection de glucose (1 g/kg) est réalisée dans la cavité intrapéritonéale avant de collecter des échantillons de sang au niveau de la veine caudale sur des tubes héparinés 0, 15, 30, 45, et 60 minutes après la charge en glucose. Les échantillons sont conservés sur glace, le plasma isolé et conservé à -20°C avant analyse.
Les résultats obtenus sont présentés dans la figure 8.

### EXEMPLE 14 : Evaluation des effets sur l'obésité

Les composés selon l'invention testés sont les composés dont la préparation est décrite dans les exemples 2 à 5 ci-dessus.

L'obésité est associée à une augmentation de la résistance à l'insuline, au diabète de type II et à une augmentation du risque cardiovasculaire et de cancer.
Elle joue donc un rôle central dans plusieurs pathologies typiques des sociétés occidentales et de ce fait représente un défi pharmacologique important.

Les composés selon l'invention réduisent la prise de poids dans deux modèles animaux qui présentent une obésité, les souris C57BU6 maintenues sous régime riche en graisse et le rat Zucker obèse (fa/fa). Ces propriétés sont mises en évidence de la manière suivante:

### 1) Traitement des animaux

Des souris C57BU6 (Iffa Credo, L'Arbresle, France) mâles âgées de 6 semaines au début de l'expérience ont été aléatoirement rassemblées par groupes de 6 animaux sélectionnés de telle sorte que la distribution de leur poids corporel soit uniforme. Les souris ont reçu un régime pauvre en graisse (UAR AO4), un régime enrichi en graisse (huile de coco 29% poids/poids) ou le même régime enrichi complété avec les composés étudiés. Des rats Zucker mâles obèses (fa/fa) âgés de 5 semaines (Iffa Credo, L'Arbresle, France) rassemblés par groupes de 8 animaux sélectionnés de telle sorte que la distribution de taux plasmatiques de cholestérol et de triglycérides soit uniforme, sont maintenus sous régime standard complété avec les composés étudiés pendant 15 jours. Les animaux sont maintenus sous un cycle lumière/obscurité de 12 heures à une température constante de 20 ± 3°C. Les animaux ont un accès libre à l'eau et à la nourriture. La prise de nourriture et la prise de poids sont enregistrées. A l'issue de l'expérience les animaux sont pesés et sacrifiés sous anesthésie. Le plasma est préparé par centrifugation à 3000 tours/minutes pendant 20 minutes.

Des échantillons de foie et de tissu adipeux sont prélevés, pesés et conservés congelés dans l'azote liquide pour analyses ultérieures.

### 2) Dosage de la leptine

La leptine, un marqueur de développement de l'obésité, est mesurée à l'aide du kit de dosage « Rat Leptin assay » de Linco Research (St Charles, MI, USA).

### EXEMPLE 15 : Evaluation des effets sur la croissance cellulaire

Les composés selon l'invention testés sont les composés dont la préparation est décrite dans les exemples 2 à 5 ci-dessus.

Les composés selon l'invention diminuent la croissance des cellules tumorales.

Cette activité peut être constatée en utilisant le protocole décrit par Hvattum et al, Biochem J. 294, 917-921, 1993.

### EXEMPLE 16 : Evaluation des effets des composés sur la resténose

Les composés selon l'invention testés sont les composés dont la préparation est décrite dans les exemples 2 à 5 ci-dessus.

La prolifération des cellules musculaires lisses est une des principales composantes de l'athérogenèse, de la resténose et de l'hypertension associées aux maladies cardiovasculaire. L'identification d'inhibiteurs de cette prolifération représente donc un défi pharmacologique intéressant.

Les composés selon l'invention diminuent la croissance des cellules musculaires lisses vasculaires *in vitro* et réduisent la resténose *in vivo* dans le modèle d'angioplastie coronarienne au ballonnet chez le rat. Ces propriétés sont mises en évidence de la manière suivante :

### 1) Mesures de la prolifération des cellules musculaires lisses.

Les cellules musculaires lisses d'artère coronaire ou d'aorte proviennent de Promocell (Heidelberg, Allemagne) et sont cultivées selon les indications du fournisseur dans un milieu de culture sélectionné pour les cellules musculaires lisses en présence de 10% de sérum de veau foetal. Les cellules cultivées à 50% de confluence sont rendues quiescentes par omission de sérum pendant 24 heures. Les cellules sont ensuite traitées pendant 3 à 6 jours en présence de mitogènes (10% sérum, 20 ng/ml bFGF ou 2 U /ml α-thrombine) et des composés selon l'invention. A l'issue de l'expérience, les cellules sont trypsinées et comptées à l'hémocytomètre.

### 2) Mesures de la resténose dans le modèle d'angioplastie coronarienne au ballonnet chez le rat.

Des rats Sprague-Dawley adultes de 200 à 300 g (Iffa Credo, L'Arbresle, France) sont maintenus sous un cycle lumière/obscurité de 12 heures à une température constante de 20 ± 3°C. Après une acclimatation d'une semaine, les rats sont pesés et rassemblés par groupes de 6 animaux sélectionnés de telle sorte que la distribution de leur poids corporel soit uniforme. L'artère coronaire interne gauche est blessée à l'aide d'un ballonnet comme décrit précédemment (Ruef et al., Arterioscl., Thromb. And Vasc. Biol. 20, 1745-1758, 2000). Les composés selon l'invention sont suspendus dans la carboxymethylcellulose et administrés par gavage intra-gastrique, à raison d'une fois par jour pendant 4, 10 et 21 jours à différentes doses. Le traitement débute un jour avant l'intervention au ballonnet. Les animaux ont un accès libre à l'eau et à la nourriture. Les animaux sont ensuite sacrifiés et les artères coronaires sont fixées et analysées comme décrit précédemment (Ruef et al., Arterioscl., Thromb. And Vasc. Biol. 20, 1745-1758, 2000).

### EXEMPLE 17 : Evaluation des effets des composés sur l'hypertension

Les composés selon l'invention testés sont les composés dont la préparation est décrite dans les exemples 2 à 5 ci-dessus.

L'hypertension artérielle est un facteur de risque important de maladies cardiovasculaires et représente un défi pharmacologique important.

Les composés selon l'invention diminuent la pression sanguine *in vivo* quand ils sont administrés à des rats spontanément hypertendus (rat SHR) utilisés comme modèle d'hypertension. Ces propriétés sont mises en évidence de la manière suivante :

### 1) Traitement des animaux.

Des rats SHR adultes de 200 à 300 g (Harlan France, Gannat, France) sont maintenus sous un cycle lumière/obscurité de 12 heures à une température constante de 20 ± 3°C. Après une acclimatation d'une semaine, les rats sont pesés et rassemblés par groupes de 6 animaux sélectionnés de telle sorte que la distribution de leur poids corporel soit uniforme. Les composés selon l'invention sont suspendus dans la carboxymethylcellulose et administrés par gavage intra-gastrique, à raison d'une fois par jour pendant 7 jours à différentes doses. Les animaux ont un accès libre à l'eau et à la nourriture.

### 2) Mesure de la pression sanguine.

La pression sanguine est mesurée selon le protocole décrit précédemment (Siragy et Carey, J. Clin. Invest., 100, 264-269, 1997)

### EXEMPLE 18 : Evaluation des propriétés antioxydantes sur des cultures de cellules

Les composés selon l'invention testés sont les composés dont la préparation est décrite dans les exemples 2 à 5 ci-dessus.

### a) Obtention et culture des kératinocytes humains normaux

Les cultures de kératinocytes humains normaux (KHN) sont réalisées à partir de prélèvements de peau. Le prélèvement est dans un premier temps rincé 4 fois dans du PBS (Phosphate Buffered Saline - Invitrogen, France). Il est ensuite décontaminé en le trempant dans deux bains successifs d'éthanol à 70% pendant 30 secondes. On découpe alors des bandelettes de 3 mm de largeur en prenant soin d'éliminer un maximum de tissu adipeux et de derme. Les bandelettes sont alors placées pendant 4h à 37°C dans une solution de trypsine à 0,25% (Invitrogen, France).

Après séparation de l'épiderme du derme, la préparation épidermique est filtrée et centrifugée pendant 5 minutes à 1000 tours/min.. Le culot est repris avec du milieu KHN-D (DMEM + 10% sérum de veau foetal (SVF) + hydrocortisone 0,4 µg/ml + EGF 10 ng/ml + toxine cholérique 10⁻⁹M, (Sigma, St Quentin, France)). Les cellules sont comptées puis ensemencées à 10x10⁶ cellules/75 cm².

Après 24h de culture, le milieu est changé, les cellules sont rincées avec du PBS et on utilise pour la suite de la culture du milieu de prolifération K-SFM (Invitrogen, France). Les cellules sont ensuite ensemencées à la densité voulue. Le milieu des cellules est changé toutes les 48h et elles sont cultivées à 37°C avec 5% CO₂. Le traitement des cellules avec ou sans les composés selon l'invention est effectué avant la confluence (70-80%), les composés sont ajoutés à des concentrations variant de 1 à 100µM directement dans le milieu de culture.

### b) Obtention et culture des fibroblastes humains

Les cultures de fibroblastes humains normaux sont réalisées à partir d'un prélèvement de peau. Le prélèvement est dans un premier temps rincé 4 fois dans du PBS (Phosphate Buffered Saline - Invitrogen, France). Il est ensuite décontaminé en le trempant dans deux bains successifs d'éthanol à 70% pendant 30 secondes. Des morceaux de derme d'une surface d'environ 5mm² sont déposés au fond d'une boite de Pétri. Une fois que les morceaux ont adhérés au support (environ 5min), ils sont recouverts de 4ml de DMEM 20 % de SVF. Le milieu est renouvelé tous les deux jours. Les cellules sortent de l'explant au bout d'une semaine et colonisent alors la boite de Pétri. Lorsque les cellules ont colonisé le support, elles sont trypsinées, réensemencées et cultivées dans du milieu de culture DMEM 10% SVF à 37°C et 5% CO₂ (Invitrogen, France). Le traitement des cellules est effectué à la confluence de celles-ci, les composés selon l'invention sont ajoutés à des concentrations variant de 1 à 100 µM directement dans le milieu de culture.

### c) Mesure des ARN messagers

Les ARNm ont été extraits des kératinocytes et des fibroblastes humains normaux en culture traités ou non avec les composés selon l'invention. L'extraction est réalisée à l'aide des réactifs du kit Absolutely RNA RT-PCR miniprep Kit (Stratagene, France) selon les indications du fournisseur. Les ARNm sont ensuite dosés par spectrométrie et quantifiés par RT-PCR quantitative à l'aide du kit Light Cycler Fast start DNA Master Sybr Green I kit (Roche) sur un appareil Light Cycler System (Roche, France). Des paires d'amorces spécifiques des gènes de la Super Oxyde Dismutase (SOD) et de la Glutathion Peroxydase (GPx), enzymes anti-oxydantes, ont été utilisées comme sondes. Des paires d'amorces spécifiques des gènes 36B4, β-actine et GAPDH ont été utilisées comme sondes témoins (Cf. tableau I).

### d) Mesure de l'activité de la glutathion peroxydase (GPx)

L'activité de la Glutathion peroxydase est déterminée à partir d'extraits protéiques de cellules (kératinocytes, fibroblastes) traitées ou non avec les composés selon l'invention à des concentrations variant de 1 à 100 µM. La mesure de l'activité de la GPx est également réalisée dans des conditions stressantes pour les cellules, 0,5 mM paraquat ou 0,6 mM H₂O₂ (inducteurs des espèces oxygénées réactives). La mesure de l'activité des extraits protéiques se fait à l'aide du kit Glutathione Peroxidase Cellular-Activity Assay Kit (Sigma) selon les indications du fournisseur. La mesure indirecte est basée sur l'oxydation du glutathion en glutathion oxydé catalysé par la glutathion peroxydase. Le retour à la forme non oxydée est catalysé par la glutathion réductase et le NADPH (β-nicotinamide Adenine Dinucléotide Phophate). La diminution de l'absorbance du NADPH est mesurée à 340 nm à l'aide d'un spectrofluorimètre Shimazu 1501 (Shimadzu Corporation, Kyoto, Japon) et reflète l'activité de la GPx, puisque la GPx est le facteur limitant de cette réaction.

### e) Mesure de la peroxydation lipidique

Les réactifs proviennent de chez Sigma (St Quentin, France) sauf lorsque mentionné autrement.

La peroxydation lipidique est mesurée par dosage du malondialdéhyde (MDA) par l'acide thiobarbiturique (TBA). Après les traitements, le surnageant des cellules est collecté (900 µl) et 90 µl d'hydroxytoluène butylé y sont ajoutés (Morliere P. et al. (1991). UVA-induced lipid peroxidation in cultured human fibroblasts. Biochim Biophys Acta 1084, 261-8.). Un ml d'une solution de TBA à 0,375% dans 0,25M HCl contenant 15% d'acide trichloroacétique est également ajouté au surnageant. Le mélange est chauffé à 80°C pendant 15 min, refroidi sur glace et la phase organique est extraite avec du butanol. L'analyse de la phase organique se fait par spectrofluorométrie (λexc =515 nm et λem=550 nm), en utilisant un spectrofluorimètre Shimazu 1501 (Shimadzu Corporation, Kyoto, Japon). Les TBARS sont exprimés en équivalents MDA en utilisant comme standard le tétra-éthoxypropane. Les résultats sont normalisés par rapport au contenu en protéines des cellules. L'induction de la peroxydation lipidique est obtenue en traitant les cellules avec du paraquat 0,5 mM (inducteur des espèces oxygénées réactives) ou avec du peroxyde d'hydrogène 0,6 mM pendant 4h. La protection anti-radicalaire des composés selon l'invention à des concentrations variant de 1 à 100 µM est évaluée par un pré-traitement de 24h, avant l'induction de la peroxydation lipidique.

### EXEMPLE 19: Evaluation des propriétés anti-inflammatoires sur des épidermes reconstruits

Les épidermes reconstruits sont fournis par la société SkinEthic (Nice, France). Les épidermes sont utilisés à j 17 (0,63 cm²) lorsque la couche cornée est présente et que l'ultra structure de l'épithélium se rapproche de celle de l'épiderme humain *in vivo.* Les épidermes reconstruits sont maintenus en culture selon les indications du fournisseur. Les doses de composés selon l'invention employées pour le traitement des épidermes reconstruits varient entre 2 et 10 mg/cm² pendant 24 et 72h.

Les composés selon l'invention testés sont les composés dont la préparation est décrite dans les exemples 2 à 5 ci-dessus.

### a) Mesure des propriétés anti-inflammatoires

Les épidermes reconstruits sont préincubés avec les composés selon l'invention à des concentrations variant de 2 à 10 mg/cm² pendant 24h, puis traités pendant 6h avec du SDS 0,4% ou 1µg de TPA (12-O-tétradécanoylphorbol-13-acétate). Le potentiel anti-inflammatoire des composés est évalué à l'aide de la technique ELISA. Les milieux de culture (sous-jacents) des épidermes contrôles ou traités sont collectés et congelés à -20°C. La quantification de l'interleukine 1-α (IL1-α) est réalisée à l'aide du kit de détection ELISA IL1-α Kit (R&D system, UK) selon les indications du fournisseur.

### b) Mesure des ARN messagers

Les ARNm ont été extraits des épidermes reconstruits traités avec ou sans les composés selon l'invention dans les mêmes conditions que précédemment. L'extraction est réalisée à l'aide des réactifs du kit Absolutely RNA RT-PCR miniprep Kit-(Stratagene) selon les indications du fournisseur, les-ARNm sont ensuite dosés par spectrométrie et quantifiés par RT-PCR quantitative à l'aide du kit Light Cycler Fast start DNA Master Sybr Green I kit (Roche) sur un appareil Light Cycler System (Roche). Des paires d'amorces spécifiques des gènes IL1 (interleukine 1) et IL6, ont été utilisées comme sondes. Des paires d'amorces spécifiques des gènes 36B4, β-actine et GAPDH ont été utilisées comme sondes témoins (Cf. tableau I).

### EXEMPLE 20 : Evaluation des propriétés anti-oxydantes sur des épidermes reconstruits

Les épidermes reconstruits sont fournis par la société SkinEthic (Nice, France).
Les épidermes sont utilisés à j 17 (0,63 cm²) lorsque la couche cornée est présente et que l'ultra structure de l'épithélium se rapproche de celle de l'épiderme humain *in vivo.* Les épidermes reconstruits sont maintenus en culture selon les indications du fournisseur. Les doses de composés selon l'invention employées pour le traitement des épidermes reconstruits varient entre 2 et 10 mg/cm² pendant 24 et 72h.

Les composés selon l'invention testés sont les composés dont la préparation est décrite dans les exemples 2 à 5 ci-dessus.

### a) Mesure des ARN messagers

Les ARNm ont été extraits des kératinocytes (provenant des épidermes reconstruits traités avec ou sans les composés selon l'invention). L'extraction est réalisée à l'aide des réactifs du kit Absolutely RNA RT-PCR miniprep Kit (Stratagene) selon les indications du fournisseur, les ARNm sont ensuite dosés par spectrométrie et quantifiés par RT-PCR quantitative à l'aide du kit Light Cycler Fast start DNA Master Sybr Green I kit (Roche) sur un appareil Light Cycler System (Roche). Des paires d'amorces spécifiques des gènes de la Super Oxyde Dismutase (SOD) et de la Glutathion Peroxydase (GPx), enzymes anti-oxydantes, ont été utilisées comme sondes. Des paires d'amorces spécifiques des gènes 36B4, β-actine et GAPDH ont été utilisées comme sondes témoins (Cf. tableau 1).

### b) Mesure de l'activité de la glutathion peroxydase (GPx)

L'activité de la Glutathion peroxydase est déterminée à partir d'extraits protéiques d'épidermes reconstruits traités ou non avec les composés selon l'invention (2 à 10 mg/cm²). La mesure de l'activité de la GPx est également réalisée dans des conditions stressantes pour les cellules, 0,5 mM paraquat (inducteur des espèces oxygénées réactives). La mesure de l'activité des extraits protéiques se fait à l'aide du kit Glutathione Peroxidase Cellular Activity Assay Kit (Sigma) selon les indications du fournisseur. La mesure indirecte est basée sur l'oxydation du glutathion en glutathion oxydé catalysée par la glutathion peroxydase. Le retour à la forme non oxydée est catalysé par la glutathion réductase et le NADPH (β-nicotinamide Adenine Dinucléotide Phophate). La diminution de l'absorbance du NADPH est mesurée à 340 nm à l'aide d'un spectrofluorimètre Shimazu 1501 (Shimadzu Corporation, Kyoto, Japon) et reflète l'activité de la GPx, puisque la GPx est le facteur limitant de cette réaction.

### EXEMPLE 21: Composition cosmétique : crème de jour pour le visage, anti-âge

| | |
|---|---|
| Glycéryl stéarate + PEG-100 stéarate | 6.00 % |
| Squalane | 3,00 % |
| Polyisobutène hydrogéné | 3,00 % |
| Tricaprylate/caprate de glycérol | 3,00 % |
| Glycérine | 2,00 % |
| Méthoxycinnamate d'octyl | 2,00 % |
| Cire d'abeille | 1,50 % |
| Octanoate de cétostéaryl | 1,50 % |
| Alcool cétylique | 1,00 % |
| Alcool stéarylique | 1,00 % |
| Diméthicone | 1,00 % |
| Gomme Xanthane | 0,20 % |
| Carbomer | 0,15 % |
| 1,2,3-tritétradécylthioacétylglycérol | 0,10 % |
| Neutralisant | qs. |
| Conservateurs | qs. |
| Parfum, Colorants | qs. |
| Eau | qsp 100,00 % |

### EXEMPLE 22 : Composition cosmétique : émulsion-gel pour le visage, antirides

| | |
|---|---|
| Glycérine | 5,00 % |
| Caprylic/capric/Succinic triglycérides | 3,00 % |
| Méthoxycinnamate d'octyl | 1,00 % |
| 1,3-dipalmitoyl-2-tétradécylthioacétylglycérol | 0,50 % |
| Acrylates/C10-30 alkyl acrylate crosspolymer | 0,50 % |
| Hydrolysat de protéine de blé | 0,50 % |
| Diméthicone copolyol | 0,50 % |
| Neutralisant | qs. |
| Conservateurs | qs. |
| Parfum, Colorants | qs. |
| Eau | qsp 100,00 % |

## Revendications

1. Composé de formule générale (1): 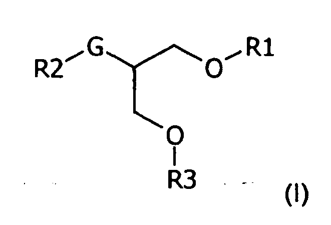 dans laquelle
- G représente un atome d'oxygène, un atome de soufre ou un groupe N-R4 dans lequel R4 est un atome d'hydrogène ou un groupe alkyle linéaire ou ramifié, saturé ou non, éventuellement substitué, comportant de 1 à 5 atomes de carbone,
- R1, R2 et R3, identiques ou différents, représentent (i) un atome d'hydrogène, (ii) un groupe CO-R dans lequel R est un groupe alkyle linéaire ou ramifié, saturé ou non, éventuellement substitué, dont la chaîne principale comporte de 1 à 25 atomes de carbone, ou (iii) un groupe de formule CO-(CH₂)₂ₙ₊₁-X-R' dans lequel X est un atome de soufre, un atome de sélénium, un groupe SO ou un groupe SO₂, n est un nombre entier compris entre 0 et 11, préférentiellement égal à 0 ou 1, et R' est un groupe alkyle linéaire ou ramifié, saturé ou non, éventuellement substitué, dont la chaîne principale comporte de 13 à 23 atomes de carbone et éventuellement un ou plusieurs hétérogroupes choisis parmi un atome d'oxygène, un atome de soufre, un atome de sélénium, un groupe SO et un groupe SO₂, l'un au moins des groupes R1, R2 et R3 étant un groupe de formule CO-(CH₂)₂ₙ₊₁-X-R' tel que défini ci-dessus.

2. Composé selon la revendication 1, **caractérisé en ce que** le ou les groupes R, identiques ou différents, représentent un groupe alkyle linéaire ou ramifié, saturé ou insaturé, substitué ou non, dont la chaîne principale comporte de 1 à 20 atomes de carbone de préférence de 7 à 17 atomes de carbone.

3. Composé selon la revendication 1 ou 2, **caractérisé en ce que** le ou les groupes R', identiques ou différents, représentent un groupe alkyle linéaire ou ramifié, saturé ou insaturé, substitué ou non, dont la chaîne principale comporte de 13 à 20 atomes de carbone, plus préférentiellement de 14 à 17 atomes de carbone.

4. Composé selon la revendication 1 ou 2, **caractérisé en ce que** le ou les groupes R , identiques ou différents, sont choisis parmi C₇H₁₅, C₁₀H₂₁, C₁₁H₂₃, C₁₃H₂₇, C₁₄H₂₉, C₁₆H₃₃, C₁₇H₃₅, C₁₅H₃₁, C_{20:5}(5, 8, 11, 14, 17), C_{22:6}(4, 7, 10, 13, 16, 19), C₁₄H₂₇, C₁₄H₂₅, C₁₅H₂₉, C₁₇H₂₉, C₁₇H₃₁, C₁₇H₃₃, C₁₉H₂₉, C₁₉H₃₁, C₂₁H₃₁, C₂₁H₃₅, C₂₁H₃₇, C₂₁H₃₉, C₂₃H₄₅, (CH₂)ₙ-CH(CH₃)C₂H₅, (CH=C(CH₃)(CH₂)₂)_{n"}-CH=C(CH₃)₂ et (CH₂)₂ₓ₋₁-C(CH₃)₂-(CH₂)_{n'"}-CH₃, x étant un nombre entier égal à ou compris entre 1 et 11, n' étant un nombre entier égal à ou compris entre 1 et 22, n" étant un nombre entier égal à ou compris entre 1 et 5, n"' étant un nombre entier égal à ou compris entre 0 et 22, et (2x+n"') étant inférieur ou égal à 22.

5. Composé selon la revendication 1 ou 3, **caractérisé en ce que** le ou les groupes R', identiques ou différents, sont choisis parmi C₁₃H₂₇, C₁₄H₂₉, C₁₆H₃₃, C₁₇H₃₅, C₁₅H₃₁, C_{20:5}(5, 8, 11, 14, 17), C_{22:6}(4, 7, 10, 13, 16, 19), C₁₄H₂₇, C₁₄H₂₅, C₁₅H₂₉, C₁₇H₂₉, C₁₇H₃₁, C₁₇H₃₃, C₁₉H₂₉, C₁₉H₃₁, C₂₁H₃₁, C₂₁H₃₅, C₂₁H₃₇, C₂₁H₃₉, C₂₃H₄₅, (CH₂)ₙ,-CH(CH₃)C₂H₅, (CH=C(CH₃)(CH₂)₂)_{n"}-CH=C(CH₃)₂ et (CH₂)₂ₓ₊₁-C(CH₃)₂-(CH₂)_{n"'}-CH₃, X étant un nombre entier égal à ou compris entre 1 et 11, n' étant un nombre entier égal à ou compris entre 1 et 22, n" étant un nombre entier égal à ou compris entre 1 et 5, n"' étant un nombre entier égal à ou compris entre 0 et 22, et (2x+n"') étant inférieur ou égal à 20.

6. Composé selon la revendication 1, **caractérisé en ce que** le ou les groupes R, identiques ou différents, représentent un groupe alkyle inférieur comportant de 1 à 6 atomes de carbone.

7. Composé selon l'une quelconque des revendications précédentes, **caractérisé en ce que** le ou les groupes R', identiques ou différents, sont des groupes alkyle saturés et linéaires comportant de 13 à 17 atomes de carbone, de préférence de 14 à 16, plus préférentiellement 14.

8. Composé selon l'une quelconque des revendications précédentes, **caractérisé en ce que** les groupes alkyle sont substitués par un ou plusieurs substituants, identiques ou différents choisis parmi un atome d'halogène (iode, chlore, fluor, brome) et un groupe OH, =O, NO₂, NH₂, CN, CH₂-O, CH₂OCH₃, CF₃ et COOZ dans lequel Z est un atome d'hydrogène ou un groupe alkyle comportant de 1 à 6 atomes de carbone.

9. Composé selon l'une quelconque des revendications précédentes, **caractérisé en ce que** X est un atome de soufre ou de sélénium, de préférence un atome de soufre.

10. Composé selon l'une quelconque des revendications précédentes, **caractérisé en ce que** le groupe G représente un atome d'oxygène ou un groupe N-R4 et, lorsque G est N-R4, R4 représente préférentiellement un atome d'hydrogène ou un groupe méthyle.

11. Composé selon l'une des revendications précédentes, **caractérisé en ce que** dans le groupe CO-(CH₂)₂ₙ₊₁-X-R', n est différent de 1 et est en particulier égal à 0.

12. Composé de formule (I) selon l'une quelconque des revendications précédentes, dans laquelle au moins un des groupes R1, R2 et R3 représente un groupe CO-(CH₂)₂ₙ₊₁-X-R' dans lequel X représente un atome de sélénium ou de préférence de soufre et/ou R' est un groupe alkyle saturé et linéaire comprenant de 13 à 17 atomes de carbone, de préférence de 14 à 16, encore plus préférentiellement 14 atomes de carbone.

13. Composé selon l'une quelconque des revendications précédentes, **caractérisé en ce que** R2 est un groupe de formule CO-(CH₂)₂ₙ₊₁-X-R', de préférence dans laquelle X représente un atome de sélénium ou de préférence de soufre et/ou R' est un groupe alkyle saturé et linéaire comprenant de 13 à 17 atomes de carbone, plus préférentiellement dans laquelle n est égal à 0, en particulier un groupe de formule CO-CH₂-S-C₁₄H₂₉.

14. Composé selon la revendication 13, **caractérisé en ce que** R1 et R3, identiques ou différents, représentent un atome d'hydrogène ou un groupe CO-R.

15. Composé selon la revendication 14, **caractérisé en ce que** R1 et R3, identiques ou différents, représentent un groupe CO-R.

16. Composé selon l'une des revendications 1 à 12, **caractérisé en ce que** deux des groupes R1, R2 et R3 sont des groupes CO-(CH₂)₂ₙ₊₁-X-R', identiques ou différents, de préférence dans lesquels X représente un atome de sélénium ou de préférence de soufre et/ou R' est un groupe alkyle saturé et linéaire comprenant de 13 à 17 atomes de carbone, plus préférentiellement dans lesquels n est égal à 0, en particulier des groupes CO-CH₂-S-C₁₄H₂₉.

17. Composé selon l'une des revendications 1 à 12, **caractérisé en ce que** R1, R2 et R3, identiques ou différents, de préférence identiques, sont des groupes CO-(CH₂)₂ₙ₋₁-X-R', de préférence dans lesquels X représente un atome de sélénium ou de préférence de soufre et/ou R' est un groupe alkyle saturé et linéaire comprenant de 13 à 17 atomes de carbone, plus préférentiellement dans lesquels n est égal à 0, et en particulier des groupes CO-CH₂-S-C₁₄H₂₉.

18. Composé selon l'une quelconque des revendications 1 à 12, **caractérisé en ce que** R1 est un groupe de formule CO-(CH₂)₂ₙ₊₁-X-R', de préférence dans laquelle X représente un atome de sélénium ou de préférence de soufre et/ou R' est un groupe alkyle saturé et linéaire comprenant de 13 à 17 atomes de carbone, plus préférentiellement dans laquelle n est égal à 0 et en particulier un groupe CO-CH₂-S-C₁₄H₂₉.

19. Composé selon la revendication 18, **caractérisé en ce que** l'un et/ou les deux groupes R2 et R3 représentent un atome d'hydrogène.

20. Composé selon la revendication 18, **caractérisé en ce que** l'un et/ou les deux groupes R2 et R3 représentent un groupe CO-R, identique ou non.

21. Composé selon l'une quelconque des revendications 1 à 16 et 18 à 20, **caractérisé en ce que** l'un des substituants R1, R2 ou R3 est un groupe COCH₃.

22. Composé selon la revendication 1, **caractérisé en ce que** G représente un atome d'oxygène et **en ce que** les groupes R1, R2 et R3, identiques, représentent des groupes CO-CH₂-S-C₁₄H₂₉.

23. Composition pharmaceutique **caractérisée en ce qu'**elle comprend, dans un véhicule acceptable sur le plan pharmaceutique, au moins un composé de formule générale (I) telle que décrite dans la revendication 1, dans laquelle
- G représente un atome d'oxygène, un atome de soufre ou un groupe N-R4 dans lequel R4 est un atome d'hydrogène ou un groupe alkyle linéaire ou ramifié, saturé ou non, éventuellement substitué, comportant de 1 à 5 atomes de carbone,
- R1, R2 et R3, identiques ou différents, représentent (i) un atome d'hydrogène, (ii) un groupe CO-R dans lequel R est un groupe alkyle linéaire ou ramifié, saturé ou non, éventuellement substitué, dont la chaîne principale comporte de 1 à 25 atomes de carbone, ou (iii) un groupe de formule CO-(CH₂)₂ₙ₊₁-X-R' dans lequel X est un atome de soufre, un atome de sélénium, un groupe SO ou un groupe SO₂, n est un nombre entier compris entre 0 et 11, préférentiellement égal à 0 ou 1 et encore plus préférentiellement à 0, et R' est un groupe alkyle linéaire ou ramifié, saturé ou non, éventuellement substitué, dont la chaîne principale comporte de 2 à 23 atomes de carbone et éventuellement un ou plusieurs hétérogroupes, de préférence 0, 1 ou 2, plus préférentiellement 0 ou 1, choisis parmi un atome d'oxygène, un atome de soufre, un atome de sélénium, un groupe SO ou un groupe SO₂, l'un au moins des groupes R1, R2 et R3 étant un groupe de formule CO-(CH₂)₂ₙ₊₁-X-R' tel que défini ci-dessus.

24. Composition cosmétique **caractérisée en ce qu'**elle comprend, dans un véhicule acceptable sur le plan cosmétique au moins un composé de formule générale (I) telle que décrite dans la revendication 1, dans laquelle
- G représente un atome d'oxygène, un atome de soufre ou un groupe N-R4 dans lequel R4 est un atome d'hydrogène ou un groupe alkyle linéaire ou ramifié, saturé ou non, éventuellement substitué, comportant de 1 à 5 atomes de carbone,
- R1, R2 et R3, identiques ou différents, représentent (i) un atome d'hydrogène, (ii) un groupe CO-R dans lequel R est un groupe alkyle linéaire ou ramifié, saturé ou non, éventuellement substitué, dont la chaîne principale comporte de 1 à 25 atomes de carbone, ou (iii) un groupe de formule CO-(CH₂)₂ₙ₊₁-X-R' dans lequel X est un atome de soufre, un atome de sélénium, un groupe SO ou un groupe SO₂, n est un nombre entier compris entre 0 et 11, préférentiellement égal à 0 ou 1 et encore plus préférentiellement à 0, et R' est un groupe alkyle linéaire ou ramifié, saturé ou non, éventuellement substitué, dont la chaîne principale comporte de 2 à 23 atomes de carbone et éventuellement un ou plusieurs hétérogroupes, de préférence 0, 1 ou 2, plus préférentiellement 0 ou 1, choisis parmi un atome d'oxygène, un atome de soufre, un atome de sélénium, un groupe SO ou un groupe SO₂, l'un au moins des groupes R1, R2 et R3 étant un groupe de formule CO-(CH₂)₂ₙ₊₁-X-R' tel que défini ci-dessus.

25. Composition nutritionnelle **caractérisée en ce qu'**elle comprend dans un véhicule acceptable sur le plan nutritionnel, au moins un composé de formule générale (I) telle que décrite dans la revendication 1, dans laquelle
- G représente un atome d'oxygène, un atome de soufre ou un groupe N-R4 dans lequel R4 est un atome d'hydrogène ou un groupe alkyle linéaire ou ramifié, saturé ou non, éventuellement substitué, comportant de 1 à 5 atomes de carbone,
- R1, R2 et R3, identiques ou différents, représentent (i) un atome d'hydrogène, (ii) un groupe CO-R dans lequel R est un groupe alkyle linéaire ou ramifié, saturé ou non éventuellement substitué, dont la chaîne principale comporte de 1 à 25 atomes de carbone, ou (iii) un groupe de formule CO-(CH₂)₂ₙ₊₁-X-R' dans lequel X est un atome de soufre, un atome de sélénium, un groupe SO ou un groupe SO₂, n est un nombre entier compris entre 0 et 11, préférentiellement égal à 0 ou 1 et encore plus préférentiellement à 0, et R' est un groupe alkyle linéaire ou ramifié, saturé ou non, éventuellement substitué, dont la chaîne principale comporte de 2 à 23 atomes de carbone et éventuellement un ou plusieurs hétérogroupes, de préférence 0, 1 ou 2, plus préférentiellement 0 ou 1, choisis parmi un atome d'oxygène, un atome de soufre, un atome de sélénium, un groupe SO ou un groupe SO₂, l'un au moins des groupes R1, R2 et R3 étant un groupe de formule CO-(CH₂)₂ₙ₊₁-X-R' tel que défini ci-dessus.

26. Composition selon l'une quelconque des revendications 23 à 25, **caractérisée en ce que** le composé est de formule (I) dans laquelle R' est un groupe alkyle linéaire ou ramifié, saturé ou non, éventuellement substitué, dont la chaîne principale comporte de 9 à 23 atomes de carbone et éventuellement un ou plusieurs hétérogroupes.

27. Composition selon l'une des revendications 23 à 26, **caractérisée en ce que** le composé de formule (I) est tel que défini à l'une des revendications 1 à 22.

28. Composition selon la revendication 27, **caractérisée en ce que** le composé de formule (I) est tel que défini dans la revendication 22.

29. Utilisation d'un composé de formule générale (I) telle que définie à la revendication 1 pour la préparation d'une composition pharmaceutique pour la prévention et/ou le traitement de dislipidémies, de maladies cardiovasculaires, du syndrome X, de la resténose, du diabète, de l'obésité, de l'hypertension, de cancers ou de maladies dermatologiques, le composé étant de formule (I) dans laquelle :
- G représente un atome d'oxygène, un atome de soufre ou un groupe N-R4 dans lequel R4 est un atome d'hydrogène ou un groupe alkyle linéaire ou ramifié, saturé ou non, éventuellement substitué, comportant de 1 à 5 atomes de carbone,
- R1, R2 et R3, identiques ou différents, représentent (i) un atome d'hydrogène, (ii) un groupe CO-R dans lequel R est un groupe alkyle linéaire ou ramifié, saturé ou non, éventuellement substitué, dont la chaîne principale comporte de 1 à 25 atomes de carbone, ou (iii) un groupe de formule CO-(CH₂)₂ₙ₊₁-X-R' dans lequel X est un atome de soufre, un atome de sélénium, un groupe SO ou un groupe SO₂, n est un nombre entier compris entre 0 et 11, préférentiellement égal à 0 ou 1 et encore plus préférentiellement à 0, et R' est un groupe alkyle linéaire ou ramifié, saturé ou non, éventuellement substitué, dont la chaîne principale comporte de 2 à 23 atomes de carbone et éventuellement un ou plusieurs hétérogroupes, de préférence 0, 1 ou 2, plus préférentiellement 0 ou 1, choisis parmi un atome d'oxygène, un atome de soufre, un atome de sélénium, un groupe SO ou un groupe SO₂, l'un au moins des groupes R1, R2 et R3 étant un groupe de formule CO-(CH₂)₂ₙ₊₁-X-R' tel que défini ci-dessus.

30. Utilisation d'un composé de formule générale (I) telle que définie à la revendication 1 pour la préparation d'une composition cosmétique pour la prévention et/ou le traitement du vieillissement cutané et de ses effets, de la protection de la peau, de l'apparition ou du développement des rides, le composé étant de formule générale (I) dans laquelle :
- G représente un atome d'oxygène, un atome de soufre ou un groupe N-R4 dans lequel R4 est un atome d'hydrogène ou un groupe alkyle linéaire ou ramifié, saturé ou non, éventuellement substitué, comportant de 1 à 5 atomes de carbone,
- R1, R2 et R3, identiques ou différents, représentent (i) un atome d'hydrogène, (ii) un groupe CO-R dans lequel R est un groupe alkyle linéaire ou ramifié, saturé ou non, éventuellement substitué, dont la chaîne principale comporte de 1 à 25 atomes de carbone, ou (iii) un groupe de formule CO-(CH₂)₂ₙ₊₁-X-R' dans lequel X est un atome de soufre, un atome de sélénium, un groupe SO ou un groupe SO₂, n est un nombre entier compris entre 0 et 11, préférentiellement égal à 0 ou 1 et encore plus préférentiellement à 0, et R' est un groupe alkyle linéaire ou ramifié, saturé ou non, éventuellement substitué, dont la chaîne principale comporte de 2 à 23 atomes de carbone et éventuellement un ou plusieurs hétérogroupes, de préférence 0, 1 ou 2, plus préférentiellement 0 ou 1, choisis parmi un atome d'oxygène, un atome de soufre, un atome de sélénium, un groupe SO ou un groupe SO₂, l'un au moins des groupes R1, R2 et R3 étant un groupe de formule CO-(CH₂)₂ₙ₊₁-X-R' tel que défini ci-dessus.

31. Utilisation d'un composé de formule générale (I) telle que définie dans la revendication_1, pour la préparation d'une composition pharmaceutique destinée à diminuer les taux de triglycérides et/ou de cholestérol circulants ou à inhiber la modification oxydative des LDL, le composé étant de formule (I) dans laquelle :
- G représente un atome d'oxygène, un atome de soufre ou un groupe N-R4 dans lequel R4 est un atome d'hydrogène ou un groupe alkyle linéaire ou ramifié, saturé ou non, éventuellement substitué, comportant de 1 à 5 atomes de carbone,
- R1, R2 et R3, identiques ou différents, représentent (i) un atome d'hydrogène, (ii) un groupe CO-R dans lequel R est un groupe alkyle linéaire ou ramifié, saturé ou non, éventuellement substitué, dont la chaîne principale comporte de 1 à 25 atomes de carbone, ou (iii) un groupe de formule CO-(CH₂)₂ₙ₊₁-X-R' dans lequel X est un atome de soufre, un atome de sélénium, un groupe SO ou un groupe SO₂, n est un nombre entier compris entre 0 et 11, préférentiellement égal à 0 ou 1 et encore plus préférentiellement à 0, et R' est un groupe alkyle linéaire ou ramifié, saturé ou non, éventuellement substitué, dont la chaîne principale comporte de 2 à 23 atomes de carbone et éventuellement un ou plusieurs hétérogroupes, de préférence 0, 1 ou 2, plus préférentiellement 0 ou 1, choisis parmi un atome d'oxygène, un atome de soufre, un atome de sélénium, un groupe SO ou un groupe SO₂, l'un au moins des groupes R1, R2 et R3 étant un groupe de formule CO-(CH₂)₂ₙ₊₁-X-R' tel que défini ci-dessus.

32. Utilisation d'un composé de formule générale (I) telle que définie à la revendication 1, pour la préparation d'une composition pharmaceutique destinée à induire l'expression d'enzymes impliquées dans la β-oxydation mitochondriale et peroxysomale et/ou à augmenter les capacités d'oxydation des acides gras hépatiques et/ou à induire la croissance des mitochondries dans les fibres musculaires de type I et II et/ou à activer PPARα et PPARγ, le composé étant de formule générale (I) dans laquelle :
- G représente un atome d'oxygène, un atome de soufre ou un groupe N-R4 dans lequel R4 est un atome d'hydrogène ou un groupe alkyle linéaire ou ramifié, saturé ou non, éventuellement substitué, comportant de 1 à 5 atomes de carbone,
- R1, R2 et R3, identiques ou différents, représentent (i) un atome d'hydrogène, (ii) un groupe CO-R dans lequel R est un groupe alkyle linéaire ou ramifié, saturé ou non, éventuellement substitué, dont la chaîne principale comporte de 1 à 25 atomes de carbone, ou (iii) un groupe de formule CO-(CH₂)₂ₙ₊₁-X-R' dans lequel X est un atome de soufre, un atome de sélénium, un groupe SO ou un groupe SO₂, n est un nombre entier compris entre 0 et 11, préférentiellement égal à 0 ou 1 et encore plus préférentiellement à 0, et R' est un groupe alkyle linéaire ou ramifié, saturé ou non, éventuellement substitué, dont la chaîne principale comporte de 2 à 23 atomes de carbone et éventuellement un ou plusieurs hétérogroupes, de préférence 0, 1 ou 2, plus préférentiellement 0 ou 1, choisis parmi un atome d'oxygène, un atome de soufre, un atome de sélénium, un groupe SO ou un groupe SO₂, l'un au moins des groupes R1, R2 et R3 étant un groupe de formule CO-(CH₂)₂ₙ₊₁-X-R' tel que défini ci-dessus.

33. Utilisation d'un composé de formule générale (I) telle que définie dans la revendication_1, pour la préparation d'une composition pharmaceutique destinée à diminuer la croissance des cellules tumorales, le composé étant de formule générale (I) dans laquelle :
- G représente un atome d'oxygène, un atome de soufre ou un groupe N-R4 dans lequel R4 est un atome d'hydrogène ou un groupe alkyle linéaire ou ramifié, saturé ou non, éventuellement substitué, comportant de 1 à 5 atomes de carbone,
- R1, R2 et R3, identiques ou différents, représentent (i) un atome d'hydrogène, (ii) un groupe CO-R dans lequel R est un groupe alkyle linéaire ou ramifié, saturé ou non, éventuellement substitué, dont la chaîne principale comporte de 1 à 25 atomes de carbone, ou (iii) un groupe de formule CO-(CH₂)₂ₙ₊₁-X-R' dans lequel X est un atome de soufre, un atome de sélénium, un groupe SO ou un groupe SO₂, n est un nombre entier compris entre 0 et 11, préférentiellement égal à 0 ou 1 et encore plus préférentiellement à 0, et R' est un groupe alkyle linéaire ou ramifié, saturé ou non, éventuellement substitué, dont la chaîne principale comporte de 2 à 23 atomes de carbone et éventuellement un ou plusieurs hétérogroupes, de préférence 0, 1 ou 2, plus préférentiellement 0 ou 1, choisis parmi un atome d'oxygène, un atome de soufre, un atome de sélénium, un groupe SO ou un groupe SO₂, l'un au moins des groupes R1, R2 et R3 étant un groupe de formule CO-(CH₂)₂ₙ₊₁-X-R' tel que défini ci-dessus.

34. Utilisation selon l'une des revendications 29 à 33, **caractérisée en ce que** le composé est de formule (I) dans laquelle R' est un groupe alkyle linéaire ou ramifié, saturé ou non, éventuellement substitué, dont la chaîne principale comporte de 9 à 23 atomes de carbone et éventuellement un ou plusieurs hétérogroupes.

35. Utilisation selon l'une des revendications 29 à 34, **caractérisée en ce que** le composé de formule (I) est tel que défini à l'une des revendications 1 à 22.

36. selon la revendication 35, **caractérisée en ce que** le composé de formule (I) est tel que défini dans la revendication 22.

## Claims

1. Compound represented by general formula (I) : 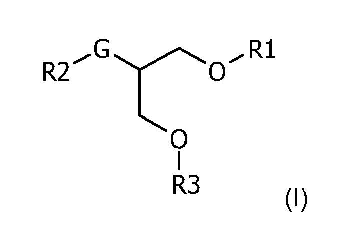 wherein
- G represents an oxygen atom, a sulfur atom or an N-R4 group in which R4 is a hydrogen atom or a linear or branched alkyl group, saturated or not, possibly substituted, containing from 1 to 5 carbon atoms,
- R1, R2 and R3, which are the same or different, represent (i) a hydrogen atom, (ii) a CO-R in which R a linear or branched alkyl group, saturated or not, possibly substituted, the main chain of which contains from 1 to 25 carbon atoms, or (iii) a group having the formula CO-(CH₂)₂ₙ₊₁-X-R' in which X is a sulfur atom, a selenium atom, an SO group or an SO₂ group, n is a whole number comprised between 0 and 11, preferably equal to 0 or 1, and R' a linear or branched alkyl group, saturated or not, possibly substituted, the main chain of which contains from 13 to 23 carbon atoms and possibly one or more heterogroups selected in the group consisting of an oxygen atom, a sulfur atom, a selenium atom, an SO group and an SO₂ group, at least one of the groups R1, R2 and R3 being a group having the formula CO-(CH₂)₂ₙ₊₁-X-R' such as defined hereinabove.

2. Compound according to claim 1, wherein the R group or groups, which are the same or different, represent a linear or branched alkyl group, saturated or unsaturated, substituted or not, the main chain of which contains from 1 to 20 carbon atoms, preferably from 7 to 17 carbon atoms.

3. Compound according to claim 1 or 2, wherein the R' group or groups, which are the same or different, represent a linear or branched alkyl group, saturated or unsaturated, substituted or not, the main chain of which contains from 13 to 20 carbon atoms, more preferably from 14 to 17 carbon atoms.

4. Compound according to claim 1 or 2, wherein the R group or groups, which are the same or different, are selected in the group consisting of C₇H₁₅, C₁₀H₂₁, C₁₁H₂₃, C₁₃H₂₇, C₁₄H₂₉, C₁₆H₃₃, C₁₇H₃₅, C₁₅H₃₁, C_{20:5}(5, 8, 11, 14, 17), C_{22:6}(4, 7, 10, 13, 16, 19), C₁₄H₂₇, C₁₄H₂₅, C₁₅H₂₉, C₁₇H₂₉, C₁₇H₃₁, C₁₇H₃₃, C₁₉H₂₉, C₁₉H₃₁, C₂₁H₃₁, C₂₁H₃₅, C₂₁H₃₇, C₂₁H₃₉, C₂₃H₄₅, (CH₂)_{n'}-CH(CH₃)C₂H₅, (CH=C(CH₃)(CH₂)₂)_{n"}-CH=C(CH₃)₂ and (CH₂)₂ₓ₊₁-C(CH₃)₂-(CH₂)_{n'"}-CH₃, x being a whole number equal to or comprised between 1 and 11, n' being a whole number equal to or comprised between 1 and 22, n" being a whole number equal to or comprised between 1 and 5, n'" being a whole number equal to or comprised between 0 and 22, and (2x+n"') being less than or equal to 22.

5. Compound according to claim 1 or 3, wherein the R' group or groups, which are the same or different, are selected in the group consisting of C₁₃H₂₇, C₁₄H₂₉, C₁₆H₃₃, C₁₇H₃₅, C₁₅H₃₁, C_{20:5}(5, 8, 11, 14, 17), C_{22:6}(4, 7, 10, 13, 16, 19), C₁₄H₂₇, C₁₄H₂₅, C₁₅H₂₉, C₁₇H₂₉, C₁₇H₃₁, C₁₇H₃₃, C₁₉H₂₉, C₁₉H₃₁, C₂₁H₃₁, C₂₁H₃₅, C₂₁H₃₇, C₂₁H₃₉, C₂₃H₄₅, (CH₂)_{n'}-CH(CH₃)C₂H₅ (CH=C(CH₃)(CH₂)₂)_{n"}-CH=C(CH₃)₂ and (CH₂)₂ₓ₊₁-C(CH₃)₂-(CH₂)_{n"'}-CH₃, x being a whole number equal to or comprised between 1 and 11, n' being a whole number equal to or comprised between 1 and 22, n" being a whole number equal to or comprised between 1 and 5, n'" being a whole number equal to or comprised between 0 and 22, and (2x+n"') being less than or equal to 20.

6. Compound according to claim 1, wherein the R group or groups, which are the same or different, represent a lower alkyl group containing from 1 to 6 carbon atoms.

7. Compound according to any one of the preceding claims, wherein the R' group or groups, which are the same or different, are saturated and linear alkyl groups containing from 13 to 17 carbon atoms, preferably from 14 to 16, more preferably 14.

8. Compound according to any one of the preceding claims, wherein the alkyl groups are substituted by one or more substituents, which are the same or different, selected in the group consisting of a halogen atom (iodine, chlorine, fluorine, bromine) and a OH, =O, NO₂, NH₂, CN, CH₂-O, CH₂OCH₃, CF₃ and COOZ group in which Z is a hydrogen atom or an alkyl group containing from 1 to 6 carbon atoms.

9. Compound according to any one of the preceding claims, wherein X is a sulfur or selenium atom, preferably a sulfur atom.

10. Compound according to any one of the preceding claims, wherein the group G represents an oxygen atom or an N-R4 group and, when G is N-R4, R4 preferably represents a hydrogen atom or a methyl group.

11. Compound according to any one of the preceding claims, wherein in the group CO-(CH₂)₂ₙ₊₁-X-R', n is different from 1 and in particular is equal to 0.

12. Compound represented by formula (I) according to any one of the preceding claims, wherein at least one of the groups R1, R2 and R3 represents a CO-(CH₂)₂ₙ₊₁-X-R' group in which X represents a selenium atom or preferably a sulfur atom and/or R' is a saturated and linear alkyl group containing from 13 to 17 carbon atoms, preferably from 14 to 16, even more preferably 14 carbon atoms.

13. Compound according to any one of the preceding claims, wherein R2 is a group corresponding to the formula CO-(CH₂)₂ₙ₊₁-X-R', preferably in which X represents a selenium atom or preferably a sulfur atom and/or R' is a saturated and linear alkyl group containing from 13 to 17 carbon atoms, more preferably in which n is equal to 0, in particular a group corresponding to the formula CO-CH₂-S-C₁₄H₂₉.

14. Compound according to claim 13, wherein R1 and R3, which are the same or different, represent a hydrogen atom or a CO-R group.

15. Compound according to claim 14, wherein R1 and R3, which are the same or different, represent a CO-R group.

16. Compound according to any one of claims 1 to 12, wherein two of the group R1, R2 and R3 are CO-(CH₂)₂ₙ₊₁-X-R' groups, which are the same or different, preferably in which X represents a selenium atom or preferably a sulfur atom and/or R' is a saturated and linear alkyl group containing from 13 to 17 carbon atoms, more preferably in which n is equal to 0, in particular CO-CH₂-S-C₁₄H₂₉ groups.

17. Compound according to any one of claims 1 to 12, wherein R1, R2 and R3, which are the same or different, preferably the same, are CO-(CH₂)₂ₙ₊₁-X-R' groups, preferably in which X represents a selenium atom or preferably a sulfur atom and/or R' is a saturated and linear alkyl group containing from 13 to 17 carbon atoms, more preferably in which n is equal to 0, in particular CO-CH₂-S-C₁₄H₂₉ groups.

18. Compound according to any one of claims 1 to 12, wherein R1 is a group corresponding to the formula CO-(CH₂)₂ₙ₊₁-X-R', preferably in which X represents a selenium atom or preferably a sulfur atom and/or R' is a saturated and linear alkyl group containing from 13 to 17 carbon atoms, more preferably in which n is equal to 0 and in particular a CO-CH₂-S-C₁₄H₂₉ group.

19. Compound according to claim 18, wherein one and/or both groups R2 and R3 represent a hydrogen atom.

20. Compound according to claim 18, wherein one and/or both groups R2 and R3 represent a CO-R group, which is the same or not.

21. Compound according to any one of claims 1 to 16 and 18 to 20, wherein one of the groups R1, R2 or R3 is a COCH₃ group.

22. Compound according to claim 1, wherein G represents an oxygen atom and R1, R2 and R3, which are the same, represent CO-CH₂-S-C₁₄H₂₉ groups.

23. Pharmaceutical composition, wherein it comprises, in a pharmaceutically acceptable vehicle, at least one compound represented by general formula (I) such as defined in claim 1, wherein
- G represents an oxygen atom, a sulfur atom or an N-R4 group in which R4 is a hydrogen atom or a linear or branched alkyl group, saturated or not, possibly substituted, containing from 1 to 5 carbon atoms,
- R1, R2 and R3, which are the same or different, represent (i) a hydrogen atom, (ii) a CO-R group in which R is a linear or branched alkyl group, saturated or not, possibly substituted, the main chain of which contains from 1 to 25 carbon atoms, or (iii) a group corresponding to the formula CO-(CH₂)₂ₙ₊₁-X-R' in which X is a sulfur atom, a selenium atom, an SO group or an SO₂ group, n is a whole number comprised between 0 and 11, preferably equal to 0 or 1 and even more preferably to 0, and R' is a linear or branched alkyl group, saturated or not, possibly substituted, the main chain of which contains from 2 to 23 carbon atoms and possibly one or more heterogroups, preferably 0, 1 or 2, more preferably 0 or 1, selected in the group consisting of an oxygen atom, a sulfur atom, a selenium atom, an SO group or an SO₂ group, at least one of the groups R1, R2 and R3 being a group corresponding to the formula CO-(CH₂)₂ₙ₊₁-X-R' such as defined hereinabove.

24. Cosmetic composition wherein it comprises, in a cosmetically acceptable vehicle, at least one compound represented by general formula (I) such as defined in claim 1, wherein
- G represents an oxygen atom, a sulfur atom or an N-R4 group in which R4 is a hydrogen atom or a linear or branched alkyl group, saturated or not, possibly substituted, containing from 1 to 5 carbon atoms,
- R1, R2 and R3, which are the same or different, represent (i) a hydrogen atom, (ii) a CO-R group in which R is a linear or branched alkyl group, saturated or not, possibly substituted, the main chain of which contains from 1 to 25 carbon atoms, or (iii) a group corresponding to the formula CO-(CH₂)₂ₙ₊₁-X-R' in which X is a sulfur atom, a selenium atom, an SO group or an SO₂ group, n is a whole number comprised between 0 and 11, preferably equal to 0 or 1 and even more preferably to 0, and R' is a linear or branched alkyl group, saturated or not, possibly substituted, the main chain of which contains from 2 to 23 carbon atoms and possibly one or more heterogroups, preferably 0, 1 or 2, more preferably 0 or 1, selected in the group consisting of an oxygen atom, a sulfur atom, a selenium atom, an SO group or an SO₂ group, at least one of the groups R1, R2 and R3 being a group corresponding to the formula CO-(CH₂)₂ₙ₊₁-X-R' such as defined hereinabove.

25. Nutritional composition wherein it comprises, in a nutritionally acceptable vehicle, at least one compound represented by general formula (I) such as defined in claim 1, wherein
- G represents an oxygen atom, a sulfur atom or an N-R4 group in which R4 is a hydrogen atom or a linear or branched alkyl group, saturated or not, possibly substituted, containing from 1 to 5 carbon atoms,
- R1, R2 and R3, which are the same or different, represent (i) a hydrogen atom, (ii) a CO-R group in which R is a linear or branched alkyl group, saturated or not, possibly substituted, the main chain of which contains from 1 to 25 carbon atoms, or (iii) a group corresponding to the formula CO-(CH₂)₂ₙ₊₁-X-R' in which X is a sulfur atom, a selenium atom, an SO group or an SO₂ group, n is a whole number comprised between 0 and 11, preferably equal to 0 or 1 and even more preferably to 0, and R' is a linear or branched alkyl group, saturated or not, possibly substituted, the main chain of which contains from 2 to 23 carbon atoms and possibly one or more heterogroups, preferably 0, 1 or 2, more preferably 0 or 1, selected in the group consisting of an oxygen atom, a sulfur atom, a selenium atom, an SO group or an SO₂ group, at least one of the groups R1, R2 and R3 being a group corresponding to the formula CO-(CH₂)₂ₙ₊₁-X-R' such as defined hereinabove.

26. Composition according to any one of claims 23 to 25, wherein the compound is represented by formula (I) in which R' is a linear or branched alkyl group, saturated or not, possibly substituted, the main chain of which contains from 9 to 23 carbon atoms and possibly one or more heterogroups.

27. Composition according to any one of claims 23 to 26, wherein the compound represented by formula (I) is such as defined in any one of claims 1 to 22.

28. Composition according to claim 27, wherein the compound represented by formula (I) is such as defined in claim 22.

29. Use of a compound represented by general formula (I) such as defined in claim 1 for preparing a pharmaceutical composition for preventing and/or treating dyslipidemiae, cardiovascular diseases, syndrome X, restenosis, diabetes, obesity, hypertension, cancers or dermatological diseases, the compound being represented by formula (I) in which :
- G represents an oxygen atom, a sulfur atom or an N-R4 group in which R4 is a hydrogen atom or a linear or branched alkyl group, saturated or not, possibly substituted, containing from 1 to 5 carbon atoms,
- R1, R2 and R3, which are the same or different, represent (i) a hydrogen atom, (ii) a CO-R group in which R is a linear or branched alkyl group, saturated or not, possibly substituted, the main chain of which contains from 1 to 25 carbon atoms, or (iii) a group corresponding to the formula CO-(CH₂)₂ₙ₊₁-X-R' in which X is a sulfur atom, a selenium atom, an SO group or an SO₂ group, n is a whole number comprised between 0 and 11, preferably equal to 0 or 1 and even more preferably to 0, and R' is a linear or branched alkyl group, saturated or not, possibly substituted, the main chain of which contains from 2 to 23 carbon atoms and possibly one or more heterogroups, preferably 0, 1 or 2, more preferably 0 or 1, selected in the group consisting of an oxygen atom, a sulfur atom, a selenium atom, an SO group or an SO₂ group, at least one of the groups R1, R2 and R3 being a group corresponding to the formula CO-(CH₂)₂ₙ₊₁-X-R' such as defined hereinabove.

30. Use of a compound represented by general formula (I) such as defined in claim 1 for preparing a cosmetic composition for preventing and/or treating skin aging and the effects thereof, protecting the skin against the appearance or development of wrinkles, the compound being represented by formula (I) in which :
- G represents an oxygen atom, a sulfur atom or an N-R4 group in which R4 is a hydrogen atom or a linear or branched alkyl group, saturated or not, possibly substituted, containing from 1 to 5 carbon atoms,
- R1, R2 and R3, which are the same or different, represent (i) a hydrogen atom, (ii) a CO-R group in which R is a linear or branched alkyl group, saturated or not, possibly substituted, the main chain of which contains from 1 to 25 carbon atoms, or (iii) a group corresponding to the formula CO-(CH₂)₂ₙ₊₁-X-R' in which X is a sulfur atom, a selenium atom, an SO group or an SO₂ group, n is a whole number comprised between 0 and 11, preferably equal to 0 or 1 and even more preferably to 0, and R' is a linear or branched alkyl group, saturated or not, possibly substituted, the main chain of which contains from 2 to 23 carbon atoms and possibly one or more heterogroups, preferably 0, 1 or 2, more preferably 0 or 1, selected in the group consisting of an oxygen atom, a sulfur atom, a selenium atom, an SO group or an SO₂ group, at least one of the groups R1, R2 and R3 being a group corresponding to the formula CO-(CH₂)₂ₙ₊₁-X-R' such as defined hereinabove.

31. Use of a compound represented by general formula (I) such as defined in claim 1 for preparing a pharmaceutical composition for lowering circulating triglyceride and/or cholesterol levels or inhibiting oxidative modification of LDL, the compound being represented by formula (I) in which :
- G represents an oxygen atom, a sulfur atom or an N-R4 group in which R4 is a hydrogen atom or a linear or branched alkyl group, saturated or not, possibly substituted, containing from 1 to 5 carbon atoms,
- R1, R2 and R3, which are the same or different, represent (i) a hydrogen atom, (ii) a CO-R group in which R is a linear or branched alkyl group, saturated or not, possibly substituted, the main chain of which contains from 1 to 25 carbon atoms, or (iii) a group corresponding to the formula CO-(CH₂)₂ₙ₊₁-X-R' in which X is a sulfur atom, a selenium atom, an SO group or an SO₂ group, n is a whole number comprised between 0 and 11, preferably equal to 0 or 1 and even more preferably to 0, and R' is a linear or branched alkyl group, saturated or not, possibly substituted, the main chain of which contains from 2 to 23 carbon atoms and possibly one or more heterogroups, preferably 0, 1 or 2, more preferably 0 or 1, selected in the group consisting of an oxygen atom, a sulfur atom, a selenium atom, an SO group or an SO₂ group, at least one of the groups R1, R2 and R3 being a group corresponding to the formula CO-(CH₂)₂ₙ₊₁-X-R' such as defined hereinabove.

32. Use of a compound represented by general formula (I) such as defined in claim 1 for preparing a pharmaceutical composition for inducing the expression of enzymes involved in mitochondrial and peroxisomal β-oxidation and/or increasing the oxidation capacity of hepatic fatty acids and/or inducing the growth of mitochondria in type I and II muscle fiber and/or activating PPARα et PPARγ, the compound being represented by formula (I) in which :
- G represents an oxygen atom, a sulfur atom or an N-R4 group in which R4 is a hydrogen atom or a linear or branched alkyl group, saturated or not, possibly substituted, containing from 1 to 5 carbon atoms,
- R1, R2 and R3, which are the same or different, represent (i) a hydrogen atom, (ii) a CO-R group in which R is a linear or branched alkyl group, saturated or not, possibly substituted, the main chain of which contains from 1 to 25 carbon atoms, or (iii) a group corresponding to the formula CO-(CH₂)₂ₙ₊₁-X-R' in which X is a sulfur atom, a selenium atom, an SO group or an SO₂ group, n is a whole number comprised between 0 and 11, preferably equal to 0 or 1 and even more preferably to 0, and R' is a linear or branched alkyl group, saturated or not, possibly substituted, the main chain of which contains from 2 to 23 carbon atoms and possibly one or more heterogroups, preferably 0, 1 or 2, more preferably 0 or 1, selected in the group consisting of an oxygen atom, a sulfur atom, a selenium atom, an SO group or an SO₂ group, at least one of the groups R1, R2 and R3 being a group corresponding to the formula CO-(CH₂)₂ₙ₊₁-X-R' such as defined hereinabove.

33. Use of a compound represented by general formula (I) such as defined in claim 1 for preparing a pharmaceutical composition for decreasing the growth of tumor cells, the compound being represented by formula (I) in which :
- G represents an oxygen atom, a sulfur atom or an N-R4 group in which R4 is a hydrogen atom or a linear or branched alkyl group, saturated or not, possibly substituted, containing from 1 to 5 carbon atoms,
- R1, R2 and R3, which are the same or different, represent (i) a hydrogen atom, (ii) a CO-R group in which R is a linear or branched alkyl group, saturated or not, possibly substituted, the main chain of which contains from 1 to 25 carbon atoms, or (iii) a group corresponding to the formula CO-(CH₂)₂ₙ₊₁-X-R' in which X is a sulfur atom, a selenium atom, an SO group or an SO₂ group, n is a whole number comprised between 0 and 11, preferably equal to 0 or 1 and even more preferably to 0, and R' is a linear or branched alkyl group, saturated or not, possibly substituted, the main chain of which contains from 2 to 23 carbon atoms and possibly one or more heterogroups, preferably 0, 1 or 2, more preferably 0 or 1, selected in the group consisting of an oxygen atom, a sulfur atom, a selenium atom, an SO group or an SO₂ group, at least one of the groups R1, R2 and R3 being a group corresponding to the formula CO-(CH₂)₂ₙ₊₁-X-R' such as defined hereinabove.

34. Use according to any one of claims 29 to 33, wherein the compound is represented by formula (I) in which R' is a linear or branched alkyl group, saturated or not, possibly substituted, the main chain of which contains from 9 to 23 carbon atoms and possibly one or more heterogroups.

35. Use according to any one of claims 29 to 34, wherein the compound represented by formula (I) is such as defined in any one of claims 1 to 22.

36. Use according to claim 35, wherein the compound represented by formula (I) is such as defined in claim 22.

## Patentansprüche

1. Verbindung der allgemeinen Formel (I): 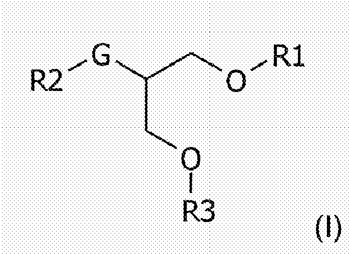 wobei
- G ein Sauerstoffatom, ein Schwefelatom oder einen Rest N-R4 darstellt, wobei R4 ein Wasserstoffatom oder ein geradkettiger oder verzweigter, gesättigter oder ungesättigter, gegebenenfalls substituierter Alkylrest, enthaltend 1 bis 5 Kohlenstoffatome, ist,
- R1, R2 und R3, welche gleich oder verschieden sind, (i) ein Wasserstoffatom, (ii) einen Rest CO-R, wobei R ein geradkettiger oder verzweigter, gesättigter oder ungesättigter, gegebenenfalls substituierter Alkylrest ist, dessen Hauptkette 1 bis 25 Kohlenstoffatome enthält oder (iii) einen Rest der Formel CO-(CH₂)₂ₙ₊₁-X-R', wobei X ein Schwefelatom, ein Selenatom, SO oder SO₂ ist, n eine ganze Zahl von 0 bis 11, vorzugsweise gleich 0 oder 1 ist und R' ein geradkettiger oder verzweigter, gesättigter oder ungesättigter, gegebenenfalls substituierter Alkylrest ist, dessen Hauptkette 13 bis 23 Kohlenstoffatome und gegebenenfalls einen oder mehrere Heteroeinheiten, ausgewählt aus einem Sauerstoffatom, einem Schwefelatom, einem Selenatom, SO und SO₂, enthält, darstellen, wobei mindestens einer der Reste R1, R2 und R3 ein Rest der Formel CO-(CH₂)₂ₙ₊₁-X-R' wie oben definiert ist.

2. Verbindung gemäß Anspruch 1, **dadurch gekennzeichnet, dass** der oder die Reste R, welche gleich oder verschieden sind, einen geradkettigen oder verzweigten, gesättigten oder ungesättigten, substituierten oder nichtsubstituierten Alkylrest darstellen, dessen Hauptkette 1 bis 20 Kohlenstoffatome, vorzugsweise 7 bis 17 Kohlenstoffatome, enthält.

3. Verbindung gemäß Anspruch 1 oder 2, **dadurch gekennzeichnet, dass** der oder die Reste R', welche gleich oder verschieden sind, einen geradkettigen oder verzweigten, gesättigten oder ungesättigten, substituierten oder nichtsubstituierten Alkylrest darstellen, dessen Hauptkette 13 bis 20 Kohlenstoffatome, besonders bevorzugt 14 bis 17 Kohlenstoffatome, enthält.

4. Verbindung gemäß Anspruch 1 oder 2, **dadurch gekennzeichnet, dass** der oder die Reste R, welche gleich oder verschieden sind, ausgewählt sind aus C₇H₁₅, C₁₀H₂₁, C₁₁H₂₃, C₁₃H₂₇, C₁₄H₂₉, C₁₆H₃₃, C₁₇H₃₅, C₁₅H₃₁, C_{20:5}(5, 8, 11, 14, 17), C_{22:6}(4, 7, 10, 13, 16, 19), C₁₄H₂₇, C₁₄H₂₅, C₁₅H₂₉, C₁₇H₂₉, C₁₇H₃₁ C₁₇H₃₃, C₁₉H₂₉, C₁₉H₃₁ C₂₁H₃₁ C₂₁H₃₅, C₂₁H₃₇, C₂₁H₃₉, C₂₃H₄₅, (CH₂)_{n'}-CH(CH₃)C₂H₅, (CH=C(CH₃)(CH₂)₂)_{n"}-CH=C(CH₃)₂ und (CH₂)₂ₓ₊₁-C(CH₃)₂-(CH₂)_{n'''}-CH₃, x eine ganze Zahl von 1 bis 11 ist, n' eine ganze Zahl von 1 bis 22 ist, n" eine ganze Zahl von 1 bis 5 ist, n"' eine ganze Zahl von 0 bis 22 ist und (2x+n''') kleiner oder gleich 22 ist.

5. Verbindung gemäß Anspruch 1 oder 3, **dadurch gekennzeichnet, dass** der oder die Reste R', welche gleich oder verschieden sind, ausgewählt sind aus C₁₃H₂₇, C₁₄H₂₉, C₁₆H₃₃, C₁₇H₃₅, C₁₅H₃₁, C_{20:5}(5, 8, 11, 14, 17), C_{22:6}(4, 7, 10, 13, 16, 19), C₁₄H₂₇, C₁₄H₂₅, C₁₅H₂₉, C₁₇H₂₉, C₁₇H₃₁ C₁₇H₃₃, C₁₉H₂₉, C₁₉H₃₁ C₂₁H₃₁ C₂₁H₃₅, C₂₁H₃₇, C₂₁H₃₉, C₂₃H₄₅, (CH₂)ₙ,-CH(CH₃)C₂H₅, (CH=C(CH₃)(CH₂)₂)_{n"}-CH=C(CH₃)₂ und (CH₂)₂ₓ₊₁-C(CH₃)₂-(CH₂)_{n"'}-CH₃, x eine ganze Zahl von 1 bis 11 ist, n' eine ganze Zahl von 1 bis 22 ist, n" eine ganze Zahl von 1 bis 5 ist, n"' eine ganze Zahl von 0 bis 22 ist und (2x+n"') kleiner oder gleich 20 ist.

6. Verbindung gemäß Anspruch 1, **dadurch gekennzeichnet, dass** der oder die Reste R, welche gleich oder verschieden sind, einen Niederalkylrest, enthaltend 1 bis 6 Kohlenstoffatome, darstellen.

7. Verbindung gemäß einem der vorhergehenden Ansprüche, **dadurch gekennzeichnet, dass** der oder die Reste R', welche gleich oder verschieden sind, gesättigte und geradkettige Alkylreste, enthaltend 13 bis 17 Kohlenstoffatome, vorzugsweise 14 bis 16, besonders bevorzugt 14 Kohlenstoffatome, darstellen.

8. Verbindung gemäß einem der vorhergehenden Ansprüche, **dadurch gekennzeichnet, dass** die Alkylreste mit einem oder mehreren Substituenten substituiert sind, welche gleich oder verschieden sind, ausgewählt aus einem Halogenatom (Iod, Chlor, Fluor, Brom) und OH, =O, NO₂ NH₂, CN, CH₂-O, CH₂OCH₃, CF₃ und COOZ, wobei Z ein Wasserstoffatom oder ein Alkylrest mit 1 bis 6 Kohlenstoffatomen ist.

9. Verbindung gemäß einem der vorhergehenden Ansprüche, **dadurch gekennzeichnet, dass** X ein Schwefel- oder Selenatom, vorzugsweise ein Schwefelatom, ist.

10. Verbindung gemäß einem der vorhergehenden Ansprüche, **dadurch gekennzeichnet, dass** der Rest G ein Sauerstoffatom oder einen Rest N-R4 darstellt und, wenn G gleich N-R4 ist, R4 vorzugsweise ein Wasserstoffatom oder eine Methylgruppe darstellt.

11. Verbindung gemäß einem der vorhergehenden Ansprüche, **dadurch gekennzeichnet, dass** in dem Rest CO-(CH₂)₂ₙ₊₁-X-R' n von 1 verschieden ist und insbesondere gleich 0 ist.

12. Verbindung der Formel (I) gemäß einem der vorhergehenden Ansprüche, wobei mindestens einer der Reste R1, R2 und R3 CO-(CH₂)₂ₙ₊₁-X-R' darstellt, wobei X ein Selenatom oder vorzugsweise ein Schwefelatom ist und/oder R' ein gesättigter und geradkettiger Alkylrest, enthaltend 13 bis 17 Kohlenstoffatome, vorzugsweise 14 bis 16, besonders bevorzugt 14 Kohlenstoffatome, ist.

13. Verbindung gemäß einem der vorhergehenden Ansprüche, **dadurch gekennzeichnet, dass** R2 ein Rest der Formel CO-(CH₂)₂ₙ₊₁-X-R' ist, vorzugsweise in welcher X ein Selenatom oder vorzugsweise ein Schwefelatom darstellt und/oder R' ein gesättigter und geradkettiger Alkylrest, enthaltend 13 bis 17 Kohlenstoffatome, ist, besonders bevorzugt in welcher n gleich 0 ist, insbesondere ein Rest der Formel CO-CH₂-S-C₁₄H₂₉.

14. Verbindung gemäß Anspruch 13, **dadurch gekennzeichnet, dass** R1 und R3, welche gleich oder verschieden sind, ein Wasserstoffatom oder einen Rest CO-R darstellen.

15. Verbindung gemäß Anspruch 14, **dadurch gekennzeichnet, dass** R1 und R3, welche gleich oder verschieden sind, einen Rest CO-R darstellen.

16. Verbindung gemäß einem der Ansprüche 1 bis 12, **dadurch gekennzeichnet, dass** zwei der Reste R1, R2 und R3 Reste CO-(CH₂)₂ₙ₊₁-X-R' darstellen, welche gleich oder verschieden sind, vorzugsweise in welchen X ein Selenatom oder vorzugsweise ein Schwefelatom darstellt und/oder R' ein gesättigter und geradkettiger Alkylrest, enthaltend 13 bis 17 Kohlenstoffatome, ist, besonders bevorzugt in welchen n gleich 0 ist, insbesondere Reste CO-CH₂-S-C₁₄H₂₉.

17. Verbindung gemäß einem der Ansprüche 1 bis 12, **dadurch gekennzeichnet, dass** R1, R2 und R3, welche gleich oder verschieden, vorzugsweise gleich, sind, Reste CO-(CH₂)₂ₙ₊₁-X-R' darstellen, vorzugsweise in welchen X ein Selenatom oder vorzugsweise ein Schwefelatom darstellt und/oder R' ein gesättigter und geradkettiger Alkylrest, enthaltend 13 bis 17 Kohlenstoffatome, ist, besonders bevorzugt in welchen n gleich 0 ist, insbesondere Reste CO-CH₂-S-C₁₄H₂₉.

18. Verbindung gemäß einem der Ansprüche 1 bis 12, **dadurch gekennzeichnet, dass** R1 ein Rest der Formel CO-(CH₂)₂ₙ₊₁-X-R' ist, vorzugsweise in welchem X ein Selenatom oder vorzugsweise ein Schwefelatom darstellt und/oder R' ein gesättigter und geradkettiger Alkylrest, enthaltend 13 bis 17 Kohlenstoffatome, ist, besonders bevorzugt in welchem n gleich 0 ist und insbesondere ein Rest CO-CH₂-S-C₁₄H₂₉.

19. Verbindung gemäß Anspruch 18, **dadurch gekennzeichnet, dass** ein und/oder beide Rest(e) R2 und R3 ein Wasserstoffatom darstellen.

20. Verbindung gemäß Anspruch 18, **dadurch gekennzeichnet, dass** ein und/oder beide Rest(e) R2 und R3 einen identischen oder nicht identischen Rest CO-R darstellen.

21. Verbindung gemäß einem der Ansprüche 1 bis 16 und 18 bis 20, **dadurch gekennzeichnet, dass** einer der Reste R1, R2 oder R3 ein Rest COCH₃ ist.

22. Verbindung gemäß Anspruch 1, **dadurch gekennzeichnet, dass** G ein Sauerstoffatom darstellt und R1, R2 und R3, welche gleich sind, Reste CO-CH₂-S-C₁₄H₂₉ darstellen.

23. Arzneimittel, **dadurch gekennzeichnet, dass** es mindestens eine Verbindung der allgemeinen Formel (I) wie in Anspruch 1 definiert in einem pharmazeutisch verträglichen Träger umfasst, wobei
- G ein Sauerstoffatom, ein Schwefelatom oder einen Rest N-R4 darstellt, wobei R4 ein Wasserstoffatom oder ein geradkettiger oder verzweigter, gesättigter oder ungesättigter, gegebenenfalls substituierter Alkylrest, enthaltend 1 bis 5 Kohlenstoffatome, ist,
- R1, R2 und R3, welche gleich oder verschieden sind, (i) ein Wasserstoffatom, (ii) einen Rest CO-R, wobei R ein geradkettiger oder verzweigter, gesättigter oder ungesättigter, gegebenenfalls substituierter Alkylrest ist, dessen Hauptkette 1 bis 25 Kohlenstoffatome enthält, oder (iii) einen Rest der Formel CO-(CH₂)₂ₙ₊₁-X-R', wobei X ein Schwefelatom, ein Selenatom, SO oder SO₂ ist, n eine ganze Zahl von 0 bis 11, vorzugsweise gleich 0 oder 1, besonders bevorzugt gleich 0 ist, und R' ein geradkettiger oder verzweigter, gesättigter oder ungesättigter, gegebenenfalls substituierter Alkylrest ist, dessen Hauptkette 2 bis 23 Kohlenstoffatome und gegebenenfalls einen oder mehrere Heteroeinheiten, vorzugsweise 0, 1 oder 2, besonders bevorzugt 0 oder 1, ausgewählt aus einem Sauerstoffatom, einem Schwefelatom, einem Selenatom, SO oder SO₂ enthält, darstellen, wobei mindestens einer der Reste R1, R2 und R3 ein Rest der Formel CO-(CH₂)₂ₙ₊₁-X-R' wie oben definiert ist.

24. Kosmetische Zusammensetzung, **dadurch gekennzeichnet, dass** sie mindestens eine Verbindung der allgemeinen Formel (I) wie in Anspruch 1 definiert in einem kosmetisch verträglichen Träger umfasst, wobei
- G ein Sauerstoffatom, ein Schwefelatom oder einen Rest N-R4 darstellt, wobei R4 ein Wasserstoffatom oder ein geradkettiger oder verzweigter, gesättigter oder ungesättigter, gegebenenfalls substituierter Alkylrest, enthaltend 1 bis 5 Kohlenstoffatome, ist,
- R1, R2 und R3, welche gleich oder verschieden sind, (i) ein Wasserstoffatom, (ii) einen Rest CO-R, wobei R ein geradkettiger oder verzweigter, gesättigter oder ungesättigter, gegebenenfalls substituierter Alkylrest ist, dessen Hauptkette 1 bis 25 Kohlenstoffatome enthält, oder (iii) einen Rest der Formel CO-(CH₂)₂ₙ₊₁-X-R', wobei X ein Schwefelatom, ein Selenatom, SO oder SO₂ ist, n eine ganze Zahl von 0 bis 11, vorzugsweise gleich 0 oder 1, besonders bevorzugt gleich 0 ist, und R' ein geradkettiger oder verzweigter, gesättigter oder ungesättigter, gegebenenfalls substituierter Alkylrest ist, dessen Hauptkette 2 bis 23 Kohlenstoffatome und gegebenenfalls einen oder mehrere Heteroeinheiten, vorzugsweise 0, 1 oder 2, besonders bevorzugt 0 oder 1, ausgewählt aus einem Sauerstoffatom, einem Schwefelatom, einem Selenatom, SO oder SO₂ enthält, darstellen, wobei mindestens einer der Reste R1, R2 und R3 ein Rest der Formel CO-(CH₂)₂ₙ₊₁-X-R' wie oben definiert ist.

25. Nahrungsmittelzusammensetzung, **dadurch gekennzeichnet, dass** sie mindestens eine Verbindung der allgemeinen Formel (I) wie in Anspruch 1 definiert in einem für Nahrungsmittel verträglichen Träger umfasst, wobei
- G ein Sauerstoffatom, ein Schwefelatom oder einen Rest N-R4 darstellt, wobei R4 ein Wasserstoffatom oder ein geradkettiger oder verzweigter, gesättigter oder ungesättigter, gegebenenfalls substituierter Alkylrest, enthaltend 1 bis 5 Kohlenstoffatome, ist,
- R1, R2 und R3, welche gleich oder verschieden sind, (i) ein Wasserstoffatom, (ii) einen Rest CO-R, wobei R ein geradkettiger oder verzweigter, gesättigter oder ungesättigter, gegebenenfalls substituierter Alkylrest ist, dessen Hauptkette 1 bis 25 Kohlenstoffatome enthält, oder (iii) einen Rest der Formel CO-(CH₂)₂ₙ₊₁-X-R', wobei X ein Schwefelatom, ein Selenatom, SO oder SO₂ ist, n eine ganze Zahl von 0 bis 11, vorzugsweise gleich 0 oder 1, besonders bevorzugt gleich 0 ist, und R' ein geradkettiger oder verzweigter, gesättigter oder ungesättigter, gegebenenfalls substituierter Alkylrest ist, dessen Hauptkette 2 bis 23 Kohlenstoffatome und gegebenenfalls einen oder mehrere Heteroeinheiten, vorzugsweise 0, 1 oder 2, besonders bevorzugt 0 oder 1, ausgewählt aus einem Sauerstoffatom, einem Schwefelatom, einem Selenatom, SO oder SO₂ enthält, darstellen, wobei mindestens einer der Reste R1, R2 und R3 ein Rest der Formel CO-(CH₂)₂ₙ₊₁-X-R' wie oben definiert ist.

26. Zusammensetzung gemäß einem der Ansprüche 23 bis 25, **dadurch gekennzeichnet, dass** die Verbindung durch die Formel (I) dargestellt ist, in welcher R' ein geradkettiger oder verzweigter, gesättigter oder ungesättigter, gegebenenfalls substituierter Alkylrest ist, dessen Hauptkette 9 bis 23 Kohlenstoffatome und gegebenenfalls einen oder mehrere Heteroeinheiten enthält.

27. Zusammensetzung gemäß einem der Ansprüche 23 bis 26, **dadurch gekennzeichnet, dass** die Verbindung der Formel (I) eine wie in einem der Ansprüche 1 bis 22 definierte Verbindung ist.

28. Zusammensetzung gemäß Anspruch 27, **dadurch gekennzeichnet, dass** die Verbindung der Formel (I) eine wie in Anspruch 22 definierte Verbindung ist.

29. Verwendung einer Verbindung der allgemeinen Formel (I) wie in Anspruch 1 definiert zur Herstellung eines Arzneimittels zur Vorbeugung und/oder Behandlung von Dyslipidämie, kardiovaskulären Erkrankungen, Syndrom X, Restenose, Diabetes, Fettsucht, Bluthochdruck, Krebs oder Hauterkrankungen, wobei in der durch Formel (I) dargestellten Verbindung:
- G ein Sauerstoffatom, ein Schwefelatom oder einen Rest N-R4 darstellt, wobei R4 ein Wasserstoffatom oder ein geradkettiger oder verzweigter, gesättigter oder ungesättigter, gegebenenfalls substituierter Alkylrest, enthaltend 1 bis 5 Kohlenstoffatome, ist,
- R1, R2 und R3, welche gleich oder verschieden sind, (i) ein Wasserstoffatom, (ii) einen Rest CO-R, wobei R ein geradkettiger oder verzweigter, gesättigter oder ungesättigter, gegebenenfalls substituierter Alkylrest ist, dessen Hauptkette 1 bis 25 Kohlenstoffatome enthält, oder (iii) einen Rest der Formel CO-(CH₂)₂ₙ₊₁-X-R', wobei X ein Schwefelatom, ein Selenatom, SO oder SO₂ ist, n eine ganze Zahl von 0 bis 11, vorzugsweise gleich 0 oder 1, besonders bevorzugt gleich 0 ist, und R' ein geradkettiger oder verzweigter, gesättigter oder ungesättigter, gegebenenfalls substituierter Alkylrest ist, dessen Hauptkette 2 bis 23 Kohlenstoffatome und gegebenenfalls einen oder mehrere Heteroeinheiten, vorzugsweise 0, 1 oder 2, besonders bevorzugt 0 oder 1, ausgewählt aus einem Sauerstoffatom, einem Schwefelatom, einem Selenatom, SO oder SO₂ enthält, darstellen, wobei mindestens einer der Reste R1, R2 und R3 ein Rest der Formel CO-(CH₂)₂ₙ₊₁-X-R' wie oben definiert ist.

30. Verwendung einer Verbindung der allgemeinen Formel (I) wie in Anspruch 1 definiert zur Herstellung einer kosmetischein Zusammensetzung zur Vorbeugung und/oder Behandlung von Hautalterung und deren Auswirkungen, zum Schutz der Haut gegen Auftreten und Bildung von Falten, wobei in der durch Formel (I) dargestellten Verbindung:
- G ein Sauerstoffatom, ein Schwefelatom oder einen Rest N-R4 darstellt, wobei R4 ein Wasserstoffatom oder ein geradkettiger oder verzweigter, gesättigter oder ungesättigter, gegebenenfalls substituierter Alkylrest, enthaltend 1 bis 5 Kohlenstoffatome, ist,
- R1, R2 und R3, welche gleich oder verschieden sind, (i) ein Wasserstoffatom, (ii) einen Rest CO-R, wobei R ein geradkettiger oder verzweigter, gesättigter oder ungesättigter, gegebenenfalls substituierter Alkylrest ist, dessen Hauptkette 1 bis 25 Kohlenstoffatome enthält, oder (iii) einen Rest der Formel CO-(CH₂)₂ₙ₊₁-X-R', wobei X ein Schwefelatom, ein Selenatom, SO oder SO₂ ist, n eine ganze Zahl von 0 bis 11, vorzugsweise gleich 0 oder 1 und besonders bevorzugt gleich 0 ist, und R' ein geradkettiger oder verzweigter, gesättigter oder ungesättigter, gegebenenfalls substituierter Alkylrest ist, dessen Hauptkette 2 bis 23 Kohlenstoffatome und gegebenenfalls einen oder mehrere Heteroeinheiten, vorzugsweise 0, 1 oder 2, besonders bevorzugt 0 oder 1, ausgewählt aus einem Sauerstoffatom, einem Schwefelatom, einem Selenatom, SO oder SO₂ enthält, darstellen, wobei mindestens einer der Reste R1, R2 und R3 ein Rest der Formel CO-(CH₂)₂ₙ₊₁-X-R' wie oben definiert ist.

31. Verwendung einer Verbindung der allgemeinen Formel (I) wie in Anspruch 1 definiert zur Herstellung eines Arzneimittels zur Senkung der Mengen an zirkulierenden Triglyceriden und/oder an zirkulierendem Cholesterin oder zur Hemmung von oxidativer Veränderung des LDL, wobei in der durch Formel (I) dargestellten Verbindung:
- G ein Sauerstoffatom, ein Schwefelatom oder einen Rest N-R4 darstellt, wobei R4 ein Wasserstoffatom oder ein geradkettiger oder verzweigter, gesättigter oder ungesättigter, gegebenenfalls substituierter Alkylrest, enthaltend 1 bis 5 Kohlenstoffatome, ist,
- R1, R2 und R3, welche gleich oder verschieden sind, (i) ein Wasserstoffatom, (ii) einen Rest CO-R, wobei R ein geradkettiger oder verzweigter, gesättigter oder ungesättigter, gegebenenfalls substituierter Alkylrest ist, dessen Hauptkette 1 bis 25 Kohlenstoffatome enthält, oder (iii) einen Rest der Formel CO-(CH₂)₂ₙ₊₁-X-R', wobei X ein Schwefelatom, ein Selenatom, SO oder SO₂ ist, n eine ganze Zahl von 0 bis 11, vorzugsweise gleich 0 oder 1 und besonders bevorzugt gleich 0 ist, und R' ein geradkettiger oder verzweigter, gesättigter oder ungesättigter, gegebenenfalls substituierter Alkylrest ist, dessen Hauptkette 2 bis 23 Kohlenstoffatome und gegebenenfalls einen oder mehrere Heteroeinheiten, vorzugsweise 0, 1 oder 2, besonders bevorzugt 0 oder 1, ausgewählt aus einem Sauerstoffatom, einem Schwefelatom, einem Selenatom, SO oder SO₂ enthält, darstellen, wobei mindestens einer der Reste R1, R2 und R3 ein Rest der Formel CO-(CH₂)₂ₙ₊₁-X-R' wie oben definiert ist.

32. Verwendung einer Verbindung der allgemeinen Formel (I) wie in Anspruch 1 definiert zur Herstellung eines Arzneimittels zur Induzierung der Exprimierung von Enzymen, welche mit mitochondrialer und peroxisomaler β-Oxidation in Verbindung stehen und/oder zur Erhöhung der Oxidationskapazität von hepatischen Fettsäuren und/oder zur Anregung des Wachstums von Mitochondrien in Typ I und II Muskelfasern und/oder zur Aktivierung von PPARα und PPARγ, wobei in der durch Formel (I) dargestellten Verbindung:
- G ein Sauerstoffatom, ein Schwefelatom oder einen Rest N-R4 darstellt, wobei R4 ein Wasserstoffatom oder ein geradkettiger oder verzweigter, gesättigter oder ungesättigter, gegebenenfalls substituierter Alkylrest, enthaltend 1 bis 5 Kohlenstoffatome, ist,
- R1, R2 und R3, welche gleich oder verschieden sind, (i) ein Wasserstoffatom, (ii) einen Rest CO-R, wobei R ein geradkettiger oder verzweigter, gesättigter oder ungesättigter, gegebenenfalls substituierter Alkylrest ist, dessen Hauptkette 1 bis 25 Kohlenstoffatome enthält, oder (iii) einen Rest der Formel CO-(CH₂)₂ₙ₊₁-X-R', wobei X ein Schwefelatom, ein Selenatom, SO oder SO₂ ist, n eine ganze Zahl von 0 bis 11, vorzugsweise gleich 0 oder 1 und besonders bevorzugt gleich 0 ist, und R' ein geradkettiger oder verzweigter, gesättigter oder ungesättigter, gegebenenfalls substituierter Alkylrest ist, dessen Hauptkette 2 bis 23 Kohlenstoffatome und gegebenenfalls einen oder mehrere Heteroeinheiten, vorzugsweise 0, 1 oder 2, besonders bevorzugt 0 oder 1, ausgewählt aus einem Sauerstoffatom, einem Schwefelatom, einem Selenatom, SO oder SO₂ enthält, darstellen, wobei mindestens einer der Reste R1, R2 und R3 ein Rest der Formel CO-(CH₂)₂ₙ₊₁-X-R' wie oben definiert ist.

33. Verwendung einer Verbindung der allgemeinen Formel (I) wie in Anspruch 1 definiert zur Herstellung eines Arzneimittels zur Verminderung des Wachstums von Tumorzellen, wobei in der durch Formel (I) dargestellten Verbindung:
- G ein Sauerstoffatom, ein Schwefelatom oder einen Rest N-R4 darstellt, wobei R4 ein Wasserstoffatom oder ein geradkettiger oder verzweigter, gesättigter oder ungesättigter, gegebenenfalls substituierter Alkylrest, enthaltend 1 bis 5 Kohlenstoffatome, ist,
- R1, R2 und R3, welche gleich oder verschieden sind, (i) ein Wasserstoffatom, (ii) einen Rest CO-R, wobei R ein geradkettiger oder verzweigter, gesättigter oder ungesättigter, gegebenenfalls substituierter Alkylrest ist, dessen Hauptkette 1 bis 25 Kohlenstoffatome enthält, oder (iii) einen Rest der Formel CO-(CH₂)₂ₙ₊ᵢ-X-R', wobei X ein Schwefelatom, ein Selenatom, SO oder SO₂ ist, n eine ganze Zahl von 0 bis 11, vorzugsweise gleich 0 oder 1 und besonders bevorzugt gleich 0 ist, und R' ein geradkettiger oder verzweigter, gesättigter oder ungesättigter, gegebenenfalls substituierter Alkylrest ist, dessen Hauptkette 2 bis 23 Kohlenstoffatome und gegebenenfalls einen oder mehrere Heteroeinheiten, vorzugsweise 0, 1 oder 2, besonders bevorzugt 0 oder 1, ausgewählt aus einem Sauerstoffatom, einem Schwefelatom, einem Selenatom, SO oder SO₂ enthält, darstellen, wobei mindestens einer der Reste R1, R2 und R3 ein Rest der Formel CO-(CH₂)₂ₙ₊₁-X-R' wie oben definiert ist.

34. Verwendung gemäß einem der Ansprüche 29 bis 33, **dadurch gekennzeichnet, dass** die Verbindung durch die Formel (I) dargestellt ist, in welcher R' ein geradkettiger oder verzweigter, gesättigter oder ungesättigter, gegebenenfalls substituierter Alkylrest ist, dessen Hauptkette 9 bis 23 Kohlenstoffatome und gegebenenfalls einen oder mehrere Heteroeinheiten enthält.

35. Verwendung gemäß einem der Ansprüche 29 bis 34, **dadurch gekennzeichnet, dass** die Verbindung der Formel (I) wie in einem der Ansprüche 1 bis 22 definiert ist.

36. Verwendung gemäß Anspruch 35, **dadurch gekennzeichnet, dass** die Verbindung der Formel (I) wie in Anspruch 22 definiert ist.
